# EUROPEAN PATENT APPLICATION

(11) **EP 4 389 749 A1**
(43) Date of publication of application: **26.06.2024**
(21) Application number: 22215234.0
(22) Date of filing: 20.12.2022
(51) Int. Cl.: C07D 473/06, A61P 35/00, A61P 37/04, A61K 31/522

(54) **SUBSTITUTED 3,7-DIHYDRO-1H-PURINE-2,6-DIONES AND USE THEREOF**

(71) Applicant: invIOs GmbH, 1030 Wien (AT)
(72) Inventor: The designation of the inventor has not yet been filed
(74) Representative: Arth, Hans-Lothar

(57) **Abstract**

The present invention relates to 3,7,8-trisubstituted 3,7-dihydro-1/-/-purine-2,6-dione compounds also named as 3,7,8-trisubstituted xanthine compounds of general formula (I) or pharmaceutically acceptable salts thereof.

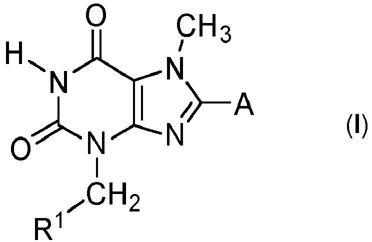

The invention further relates to pharmaceutical compositions comprising such compounds as well as the use of the compounds or pharmaceutical compositions as medicaments, especially in methods for the treatment or prophylaxis of a neoplastic and/or infectious disease and in vitro methods.

These compounds activate immune cells, in particular T-cells such as CD4+ and CD8+ cells.

## Description

### Field of the Invention

The present invention relates to 3,7,8-trisubstituted 3,7-dihydro-1H-purine-2,6-dione compounds also named as 3,7,8-trisubstituted xanthine compounds of general formula (I) or pharmaceutically acceptable salts thereof.

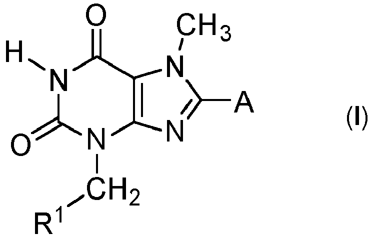

The invention further relates to pharmaceutical compositions comprising such compounds as well as their use as medicaments, especially in methods for the treatment or prophylaxis of a neoplastic and/or infectious disease and in vitro methods. These compounds activate immune cells, including NK cells or T-cells such as CD4+ and CD8+ cells.

### Background of the Invention

Malignant neoplasia (cancer) and infectious diseases are two of the main causes of death all over the world. In an increasing number of cases, it is known that there is often interdependency between neoplastic and infectious diseases, such as, e.g., between cervix neoplasia and herpes simplex virus infections. Although a large variety of compounds for treating and preventing these diseases has been found, it is well-known that such compounds bear significant drawbacks such as provoking severe side effects. Therefore, there is still an unmet need for new compounds for treating and preventing neoplastic and/or infectious diseases.

In order to overcome these drawbacks, therapeutic and prophylactic approaches based on modulating the immune response of the patient gain increasing importance in today's medicine. The immunological activity of the patient is hereby often supported by a medicinal treatment. In practice, in this context, immunologic treatments against neoplastic and infectious diseases are of particular interest. In this regard, it is well-known that in a body several types of immune cells such as, e.g., natural killer (NK) cells, T cells, B cells, dendritic cells, monocytes and macrophages, are often involved in the inactivation and removal of pathogens. With respect to neoplasia for instance, it is known that each matured neoplasm (tumor) bears specific antigens and/or neo-antigens (e.g., Sensi and Anichini, 2006, Clin Cancer Res. 12:5023-5032). This may in general trigger the adaptive immune system (e.g., T cells and/or B cells) as well as the innate immune system (e.g., natural killer (NK) cells). The immune system in a healthy body is mostly effective enough to prevent or cure said body from neoplastic and infectious diseases.

CD4+T cells along with CD8+T cells make up the majority of T-lymphocytes. CD4+T cells after being activated and differentiated into distinct effector subtypes play a major role in mediating immune response through the secretion of specific cytokines. The CD4+T cells carry out multiple functions, ranging from activation of the cells of the innate immune system, B-lymphocytes, cytotoxic T cells, as well as nonimmune cells, and also play critical role in the suppression of immune reaction.

Cytotoxic CD8⁺ T cells also play a key role in the elimination of intracellular infections and malignant cells and can provide long-term protective immunity. In the response to infection, CD8⁺ T cell metabolism is coupled to transcriptional, translational and epigenetic changes that are driven by extracellular metabolites and immunological signals. These programs facilitate the adaptation of CD8⁺ T cells to the diverse and dynamic metabolic environments encountered in the circulation and in the tissues.

In some cases, the immune system however fails to eliminate such neoplastic or infectious disease and such disease becomes chronic. In these cases, in particular when the patient suffers from malignant neoplasia (cancer), the immune system is often downregulated. Whereas in a healthy body (i.e., in a non-suppressed immune environment) a suitable expression of major histocompatibility complex I (MHC I) presenting antigens to immune cells, e.g. cytotoxic CD8 T cells is found, the expression of MHC I is down-regulated in tumor cells. This can be countered by NK cells, specifically recognizing and destroying cells with decreased MHC-I surface expression. However, during the maturation of neoplasms (in particular tumor progression), due to a multitude of immunosuppressive mechanisms leading to immune tolerance, maturating neoplasms can increasingly escape the immune system, i.e., the neoplastic antigen is not recognized as non-self, and the immune system is not activated. This mechanism is a general principle of maturating neoplasm and is neither restricted to specific neoplasms nor dependent on specific neoplastic antigens. Notably, it has been found that in most cancer patients, tumor-associated T cells and NK cells exist, but do not produce sufficient amounts of a number of cytokines (such as e.g., IL-2 and IFN-γ) or exert cytotoxic activity towards the tumor since suppression by various mechanisms hinders efficient anti-tumor immune responses (De Paola et al., 2003, British Journal of Cancer 88:320-326; Ahmadzadeh et al., 2009, Blood 114:1537-1544, in particular pages 1541-1542, section "PD-1+ TILs display an impaired effector function"). This is evidently also one of the reasons why tumor vaccination often fails.

Likewise, numerous infections are known to down-regulate the patient's immune system, in particular viral infections such as, e.g., human immunodeficiency virus (HIV) infections or herpes simplex virus (HSV) infections. Also in this context, the production of cytokines by T cells and other anti-viral immune cells is disordered.

There is still an unmet need for such compounds enabling to increase immunogenic activity and thereby enable the treatment and/or prophylaxis of neoplastic and/or infectious diseases.

It is the objective of the present invention to provide compounds and pharmaceutic compositions which activate immune cells, in particular T-cells. These compounds can be used as pharmaceutically active agents, especially for prophylaxis and/or treatment of neoplastic and infectious disease.

The present invention provides novel 3,7,8-trisubstituted xanthine compounds of general formula (I), activating immune cells.

Thus, the objective of the present invention is solved by the teachings of the independent claims. Further advantageous features, aspects and details of the invention are evident from the dependent claims, the description, the figures, and the examples of the present application.

### Description of the invention

Thus, the present invention is directed to a compound of the formula (I): 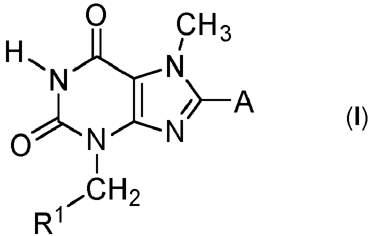 wherein
**A** represents 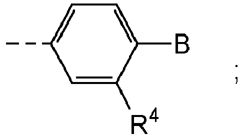
**B** represents -O-**R³**, -O-CH₂-**R³**, -O-CH₂-CH₂-**R³**, -O-CH₂-CH₂-CH₂-**R³**;
**R¹** represents 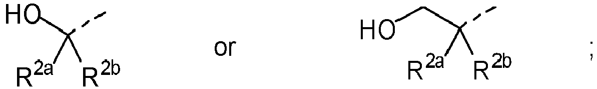
**R^{2a}** and **R^{2b}** represents independently of each other -H, or C₁-₃ alkyl, and the C₁-₃ alkyl is optionally substituted with 1 to 6 fluoro atoms or -OH;
**R³** represents 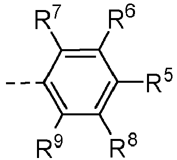
**R⁴** represents -H, halogen, C₁-₄ alkyl, -O-C₁-₄ alkyl, C₃-₄ cycloalkyl, -O-C₃-₄ cycloalkyl, and the C₁-₄ alkyl, -O-C₁-₄ alkyl, -O-C₃-₄ cycloalkyl, and C₃-₄ cycloalkyl may optionally be substituted with 1 to 6 fluoro atoms;
**R⁵**, **R⁶**, **R⁷**, **R⁸**, **R⁹**, **R¹⁰**, **R¹¹**, **R¹²** and **R¹³** represent independently of each other -H, -cyclo-C₃H₅, -cyclo-C₄H₇, -cyclo-C₅H₉, -cyclo-C₆H₁₁, -cyclo-C₇H₁₃, -cycloC₃H₅O, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂, -OCF₃, -CH₂-OCF₃, -C₂H₄-OCF₃, -C₃H₆-OCF₃, -CH₂-OCHF₂, -C₂H₄-OCHF₂, -C₃H₆-OCHF₂, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, -C≡C-C≡C-C₂H₅, 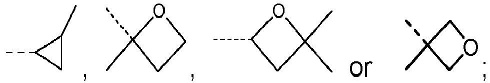 or
**R⁵** and **R⁶** or **R⁶** and **R⁷** may form together with the two carbon atoms of the phenyl ring to which they are attached a 4 to 8-membered ring system, which is optionally substituted with one or more substituents selected from **R¹⁰**, **R¹¹**, **R¹²,** and **R¹³**;
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

These compounds are suitable for enhancing the cytokine level secreted by stimulated immune cells and thereby increase the local activity of immune cells in proximity to said stimulated immune cells. These findings make that compounds of the invention useful for the treatment and/or prophylaxis of neoplastic and/or infectious diseases.

The term "pharmaceutically acceptable salt" refers to a salt of a compound that does not cause significant irritation to an organism to which it is administered and does not abrogate the biological activity and properties of the compound. The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, D-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form of the compounds of formula (I) with a sufficient amount of the desired acid to produce a salt in the conventional manner well known to those skilled in the art.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

As used herein, the term "4 to 8-membered ring system" refers to 4 to 8-membered aromatic ring, or 4 to 8-membered heteroaromatic ring, 4 to 8-membered carbohyclyl, or 4 to 8-membered heterocyclyl group. **R⁵** and **R⁶** or **R⁶** and **R⁷** form said 4 to 8-membered ring system and in said 4 to 8-membered ring system, two carbon atoms of the phenyl ring, at which **R⁵** and **R⁶** or **R⁶** and **R⁷** are substituted, are inculded.

Preferably, 4 to 8-membered aromatic ring refers to phenyl and naphthyl, wherein these phenyl and naphthyl residues can be substituted with 1 to 4 substituents selected from R¹⁰ to R¹³. However it is clear to a skilled person that the term "can be substituted" refers to the replacement of a hydrogen atom by one of the substituents **R¹⁰** to **R¹³**.

Preferably, 4- to 8-membered heteroaromatic ring" includes at least one heteroatom such as O, S, SO, SO₂, N, NO, and one double bond, wherein these monounsaturated 4-membered heterocyclic residues can be substituted with 1 to 4 substituents selected from **R¹⁰** to **R¹³**. It is clear to a skilled person that the term "can be substituted" refers to the replacement of a hydrogen atom by one of the substituents **R¹⁰** to **R¹³**.

Preferably, 4- to 8-membered heterocyclyl group" includes at least one heteroatom such as O, S, SO, SO₂, and N, and optionally one carbonyl (CO) bond, one or more double bond, wherein this 4 to 8-membered heterocyclyl group can be substituted with 1 to 4 substituents selected from **R¹⁰** to **R¹³**. it is clear to a skilled person that the term "can be substituted" refers to the replacement of a hydrogen atom by one of the substituents **R¹⁰** to **R¹³**.

Preferably, 4- to 8-membered carbocyclyl group" can include optionally one or more double bond and can be substituted with 1 to 4 substituents selected from R¹⁰ to R¹³. it is clear to a skilled person that the term "can be substituted" refers to the replacement of a hydrogen atom by one of the substituents **R¹⁰** to **R¹³**.

Preferably, in the formula (I), **R⁵** and **R⁶**, or **R⁶** and **R⁷** may form the following 4- to 6-membered ring systems, wherein the 4- to 6-membered ring system can be optionally substituted with 1 to 4 substituents selected from **R¹⁰** to **R¹³**:

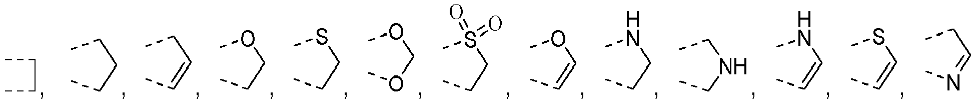

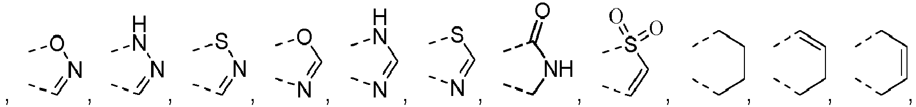

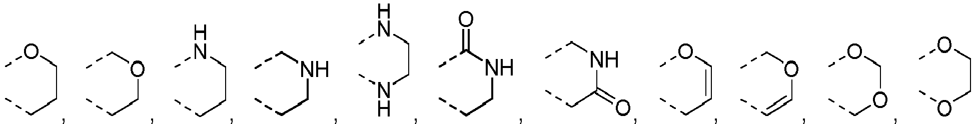

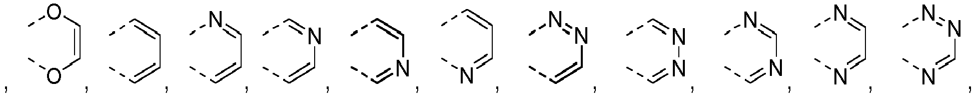

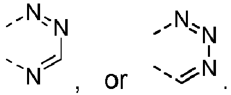

Thus, in the formula (**I**), **R³** represents preferably the following bicyclic ring, in which **R⁵** and **R⁶**, or **R⁶** and **R⁷** form the 4- to 6-membered ring system fused to a phenyl ring, wherein the 4- to 6-membered ring system can be optionally substituted with 1 to 4 substituents selected from **R¹⁰** to **R¹³**.

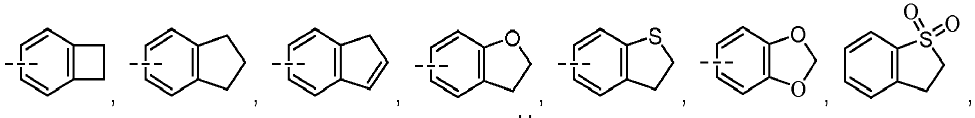

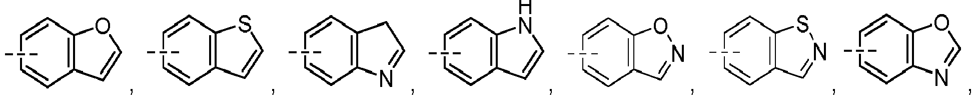

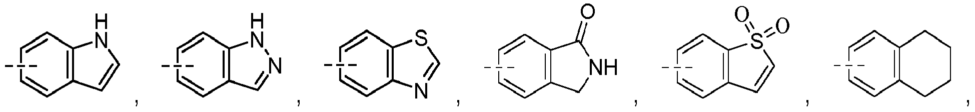

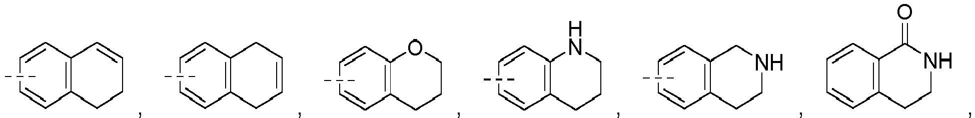

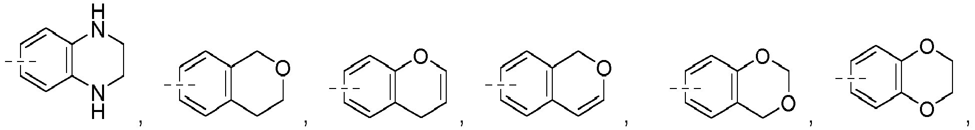

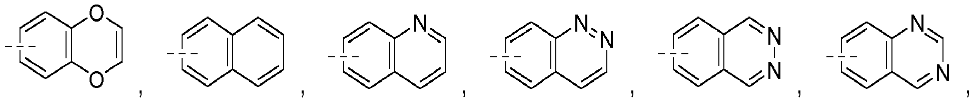

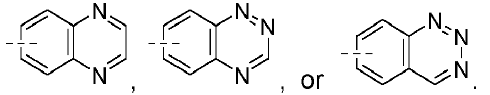

More preferably, in the formula (**I**), **R⁵** and **R⁶**, or **R⁶** and **R⁷** may form the following 4- to 6-membered ring system, wherein the 4- to 6-membered ring system can be optionally substituted with 1 to 4 substituents selected from R¹⁰ to R¹³:

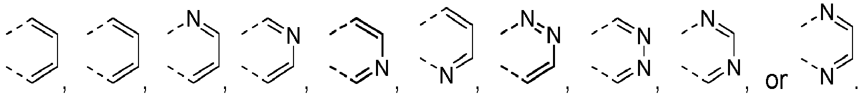

Thus, in the formula (**I**), **R³** represents preferably the following bicyclic ring, in which **R⁵** and **R⁶**, or **R⁶** and **R⁷** form the 4- to 6-membered ring system fused to a phenyl ring, wherein the 4- to 6-membered ring system can be optionally substituted with 1 to 4 substituents selected from **R¹⁰** to **R¹³**:

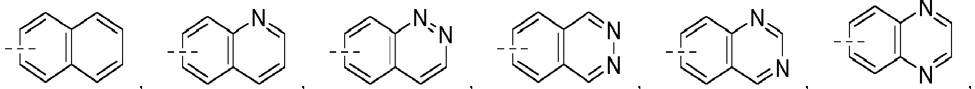

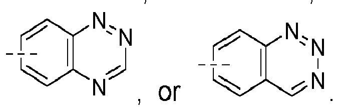

Still more preferably, in the formula (I), **R⁵** and **R⁶**, or **R⁶** and **R⁷** may form the following 6-membered ring system, wherein the 6-membered ring system can be optionally substituted with 1 to 4 substituents selected from **R¹⁰** to **R¹³**:

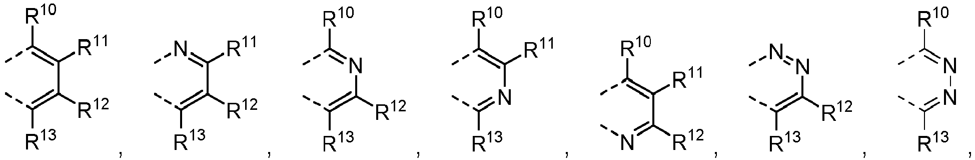

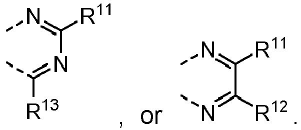

More preferably, in the formula (**I**), **R³** represents

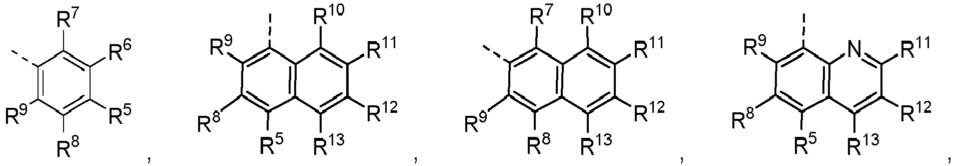

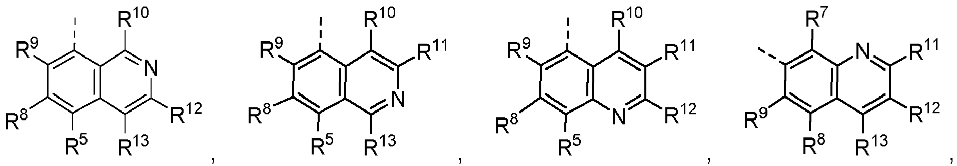

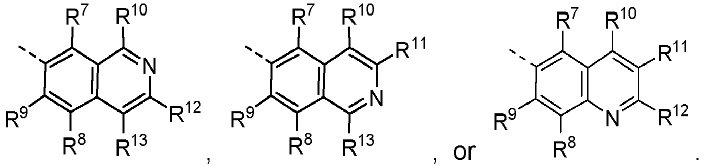

Still more preferably, in the formula (**I**), **R³** represents,

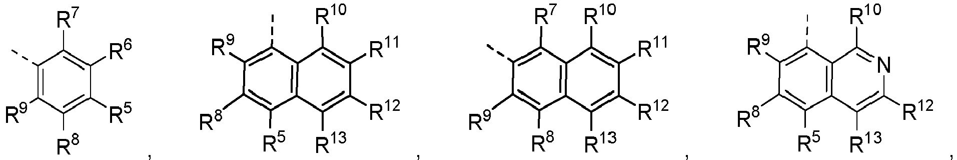

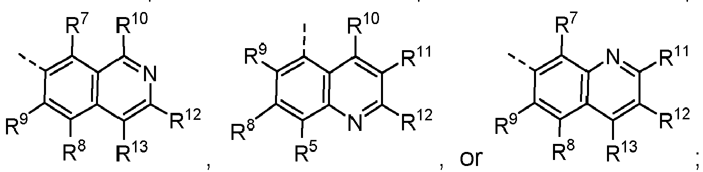

Still more preferably, in the formula (**I**), **R³** represents,

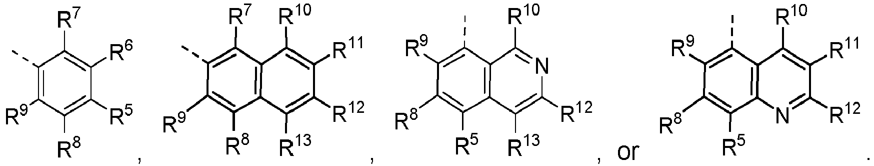

Thus, more preferably are the compound of the formula (**I**), 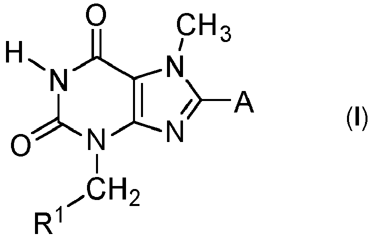 wherein
**A** represents 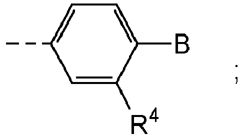
**B** represents -O-**R³**, -O-CH₂-**R³**, -O-CH₂-CH₂-**R³**, -O-CH₂-CH₂-CH₂-**R³**; preferably, **B** represents -O-**R³**, or -O-CH₂-**R³**;
**R¹** represents 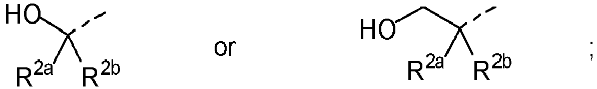
**R³** represents 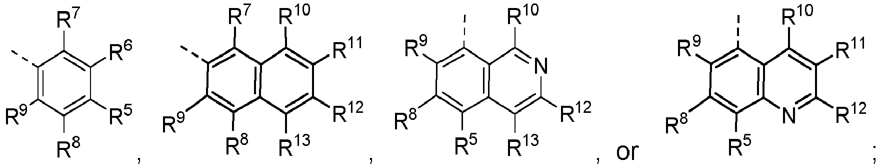
and **R^{2a}**, **R^{2b}**, **R⁴**, **R⁷** to **R¹³** have the same meanings as defined above.

Even more preferably, in the formula (**I**), **R³** represents,

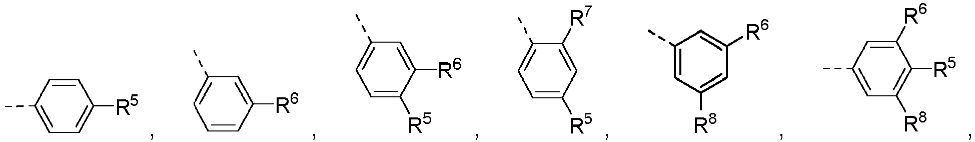

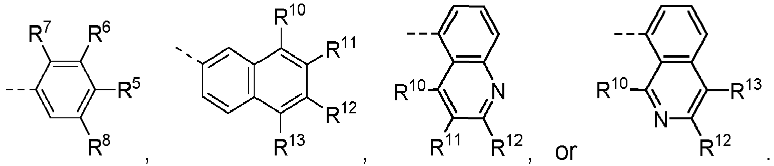

In some embodiments, the present invention refers to the compound of the formula (I), 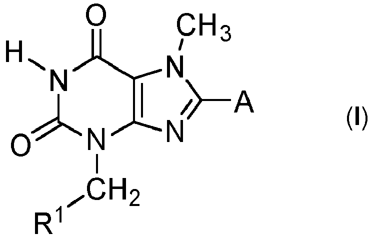 wherein
**A** represents 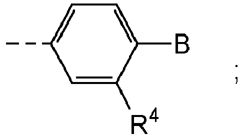
**R¹** represents 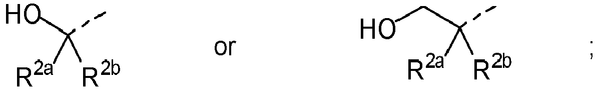
**R^{2a}** represents -H or -CH₃; and
**R^{2b}** represents -H, -CH₃, -CH₂F, -CHF₂, -CF₃, -CH₂CH₃, -CH₂OH, or -CH₂OCH₃;
**B** represents -O-**R³** or -O-CH₂-**R³**;
**R³** represents 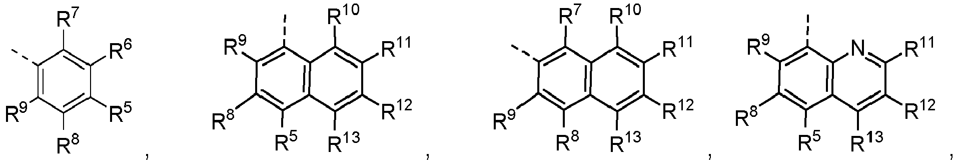 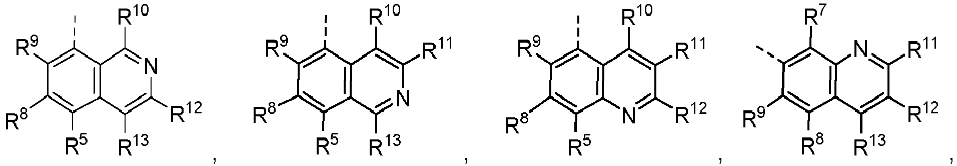 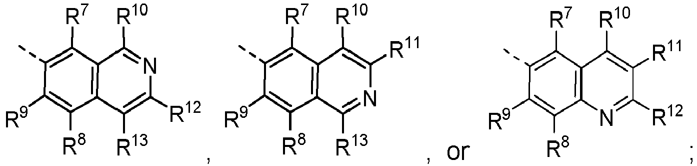
**R⁴** represents -H, -F, -Br, -Cl, -I, -CH₃, -CH₂F, -CHF₂, -CF₃, -OCH₃, -OCHF₂, or -OCF₃;
and **R⁵**, **R⁶**, **R⁷**, **R⁸**, **R⁹**, **R¹⁰**, **R¹¹**, **R¹²** and **R¹³** have the same meanings as defined above.

Preferably, the present invention refers to the compound of the formula (**I**), 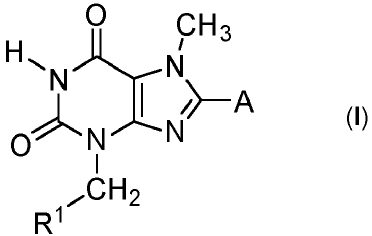 wherein
**A** represents 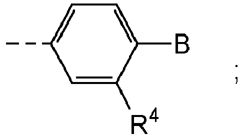
**R¹** represents 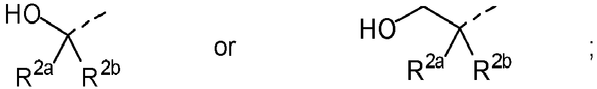
**R^{2a}** represents -H or -CH₃; and
**R^{2b}** represents -H, -CH₃, -CH₂F, -CHF₂, -CF₃, -CH₂CH₃, -CH₂OH, or -CH₂OCH₃;
**B** represents -O-**R³** or -O-CH₂-**R³**;
**R³** represents 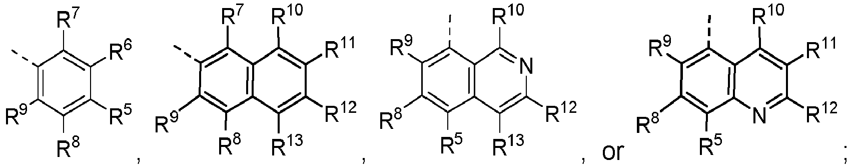
preferably, **R³** represents 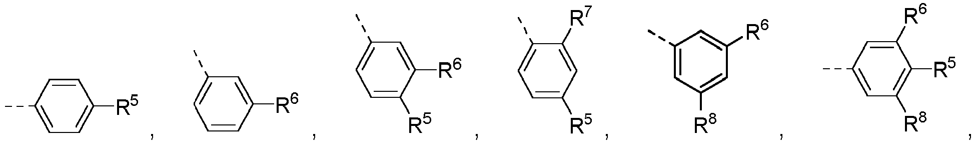 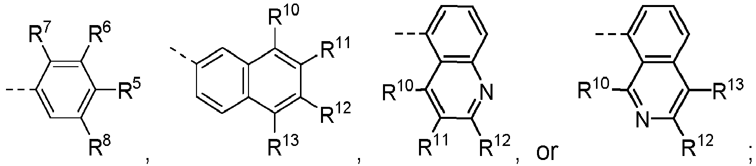
**R⁴** represents -H, -F, -Br, -Cl, -I, -CH₃, -CH₂F, -CHF₂, -CF₃, -OCH₃, -OCHF₂, or -OCF₃;
and **R⁵**, **R⁶**, **R⁷**, **R⁸**, **R⁹, R¹⁰**, R**¹¹**, **R¹²** and **R¹³** have the same meanings as defined above.

Preferably, in the compound of the formula (I) as defined above,
**R¹** represents

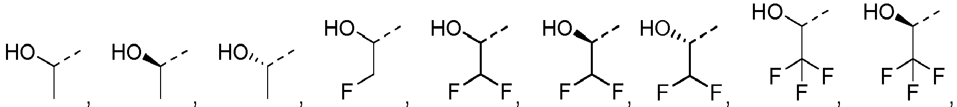

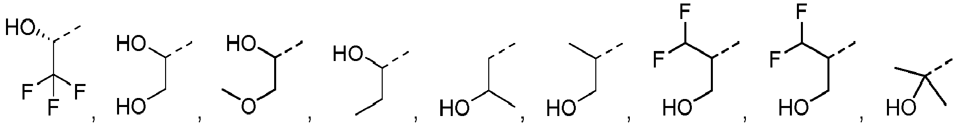

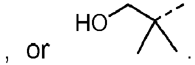

In some embodiments, the present invention relates to the compound of formula (**Ia**) or (**Ib**):

| | |
|---|---|
| 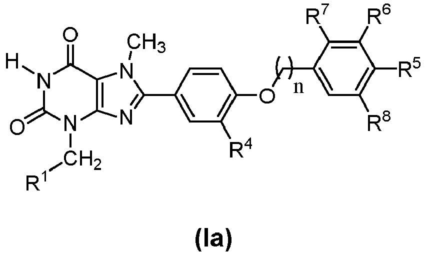 | 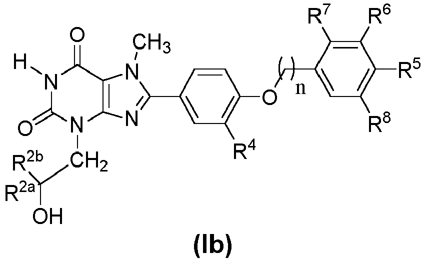 |

wherein
n is 0, 1, 2, or 3; preferably n is 0, 1, or 2, more preferably n is 0 or 1;
**R¹, R^{2a}**, **R^{2b}**, **R⁴**, **R⁵**, **R⁶**, **R⁷**, **R⁸**, and **R⁹** have the same meanings as defined above.

Preferred are the compounds of the formula (**Ia**), 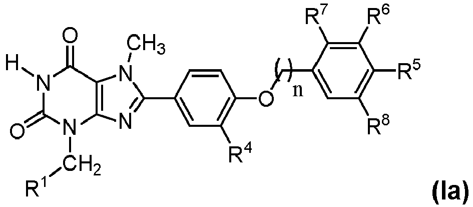 wherein
**n** is 0, 1, or 2, more preferably **n** is 0 or 1;
**R¹** represents 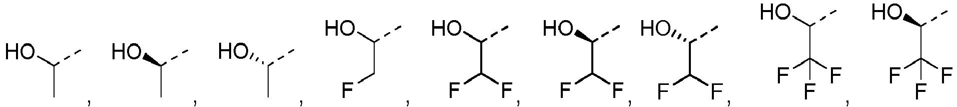 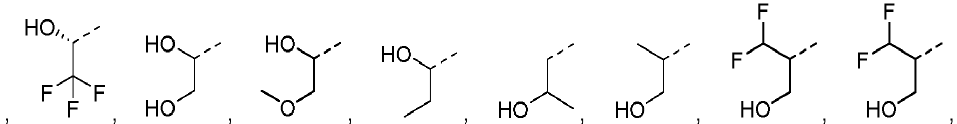 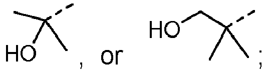
**R⁴** represents -H, -F, -Br, -Cl, -I, -CH₃, -CH₂F, -CHF₂, -CF₃, -OCH₃, -OCHF₂, or -OCF₃;
and **R¹** to **R⁹** have the same meanings as defined above.

Preferred are the compounds of the formula (**Ib**), 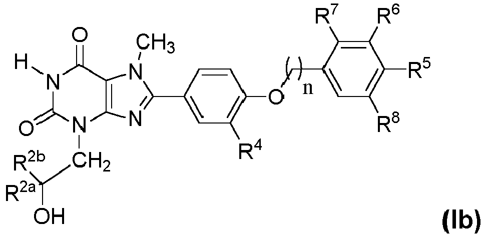 wherein
**n** is 0, 1, or 2, more preferably **n** is 0 or 1;
**R^{2a}** represents -H or -CH₃; and
**R^{2b}** represents -H, -CH₃, -CH₂F, -CHF₂, -CF₃, -CH₂CH₃, -CH₂OH, or -CH₂OCH₃;
**R⁴** represents -H, -F, -Br, -Cl, -I, -CH₃, -CH₂F, -CHF₂, -CF₃, -OCH₃, -OCHF₂, or -OCF₃;
and **R¹** to **R⁹** have the same meanings as defined above.

More preferably, in the formula (**Ia**) or (**Ib**),
**R¹** represents 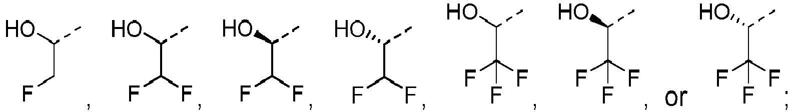
more preferably **R¹** represents

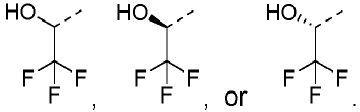

In some embodiments, the present invention relates to the compound of any one of the following formulae (**II-1**) to (**II-5**), (**III-1**) to (**III-6**), (**IV-1**) to (**IV-6**), (**V-1**) to (**V-3**): 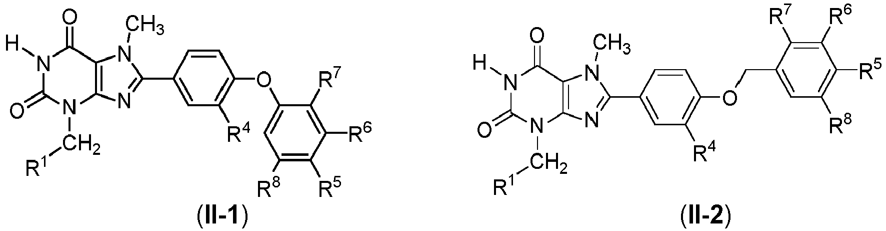 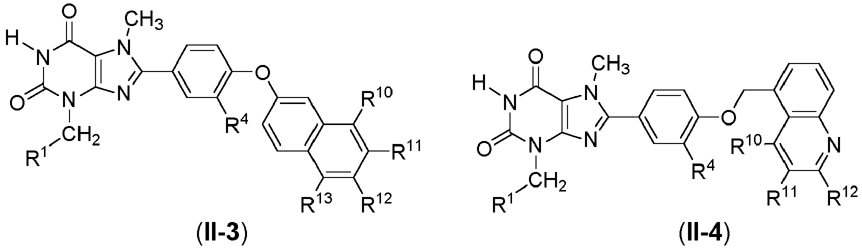 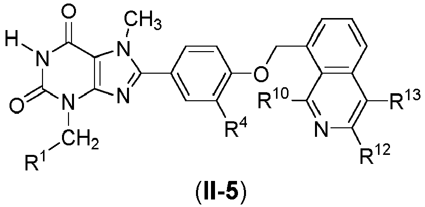 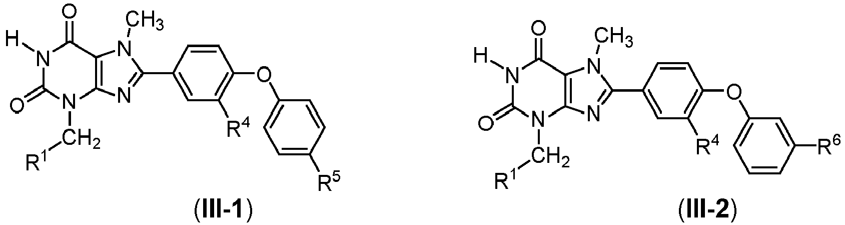 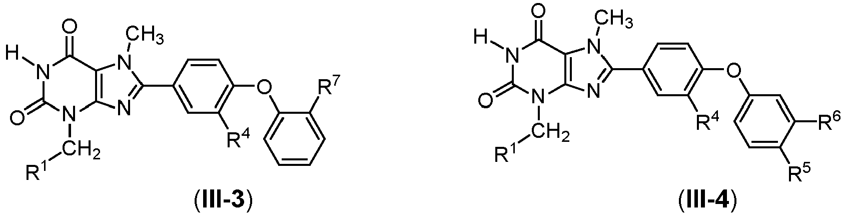 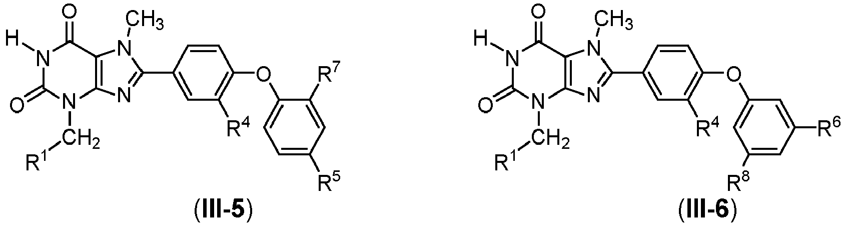 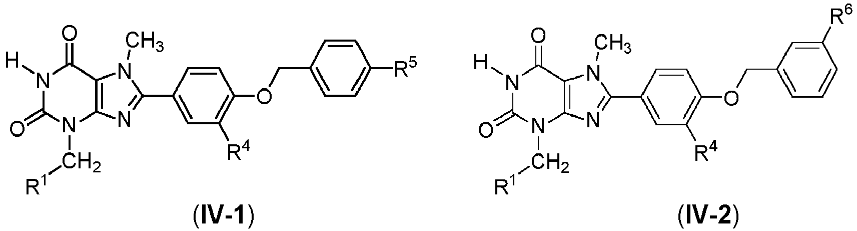 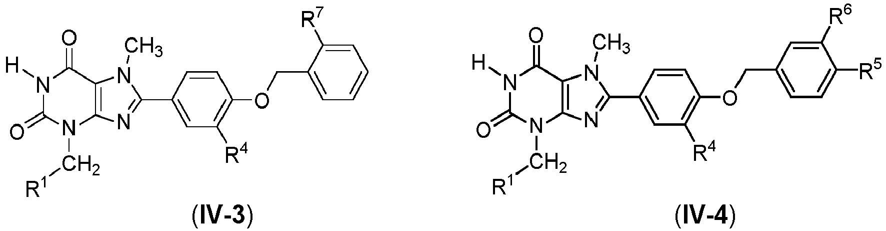 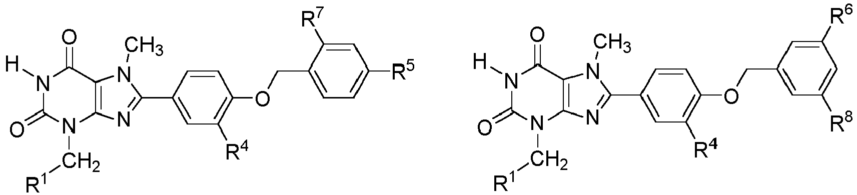 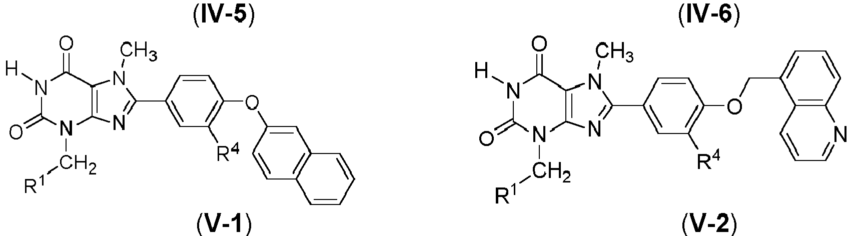 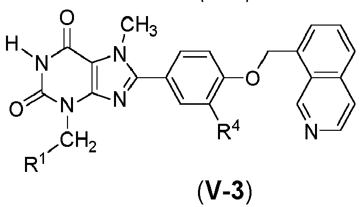 wherein **R¹**, **R⁴**, **R⁵**, **R⁶**, **R⁷**, **R⁸**, **R¹⁰**, **R¹¹**, **R¹²**, and **R¹³** have the same meanings as defined above.

Preferably, in the compound of any one of the following formulae (**II-1**) to (**II-5**), (**III-1**) to (**III-6**), (**IV-1**) to (**IV-6**), (**V-1**) to (**V-3**),
**R¹** represents 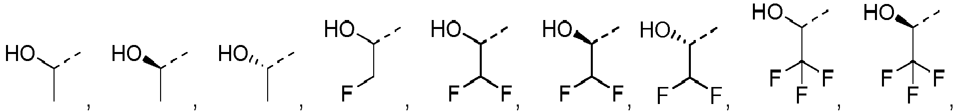 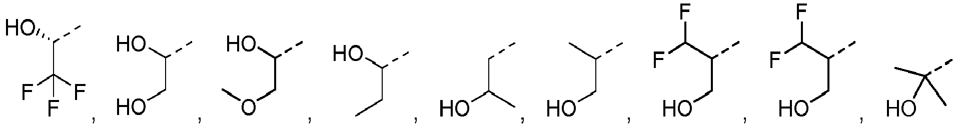 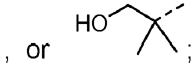
preferably **R¹** represents 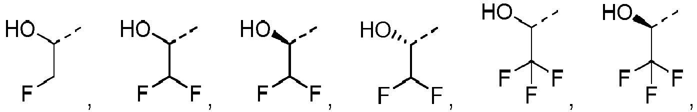 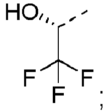 more preferably **R¹** represents

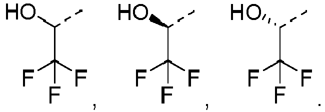

Preferably, in the compound of any one of the following formulae (**II-1**) to (**II-5**), (**III-1**) to (**III-6**), (**IV-1**) to (**IV-6**), (**V-1**) to (**V-3**),
**R⁴** represents -H, -F, -Cl, -Br, -I, -CH₃, -CH(CH₃)₂, -CH₂F, -CHF₂, -CHCl₂, -CHBr₂, -CF₃, -CCl₃, -CBr₃, -CH₂CF₃, -CF₂CF₃, -cyclo-C₃H₅, -OCH₃, -OCH₂F, -OCHF₂, , -OCF₃, , -OCH₂CH₂F, -OCH₂CF₃, -OCF₂CF₃, or -O-cyclo-C₃H₅;
preferably, **R⁴** represents -H, -F, -Cl, -Br, -CH₃, -CH₂F, -CHF₂, -CF₃, -CH₂CF₃, -CF₂CF₃, -OCH₃, -OCH₂F, -OCHF₂, -OCF₃, -OCH₂CH₂F, -OCH₂CF₃, or -OCF₂CF₃ ;
more preferably, **R⁴** represents -H, -F, -Cl, -CH₃, -CH₂F, -CHF₂, -CF₃, -OCH₃, -OCHF₂, or -OCF₃.

In a preferred embodiment, the present invention is directed to the compound of the formula (**I**), 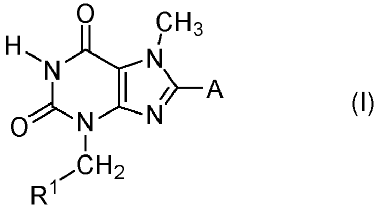 wherein
**A** represents 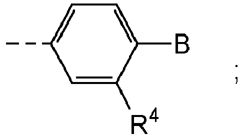
**B** represents -O-**R³** or -O-CH₂-**R³**;
**R¹** represents 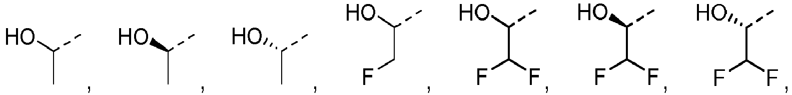 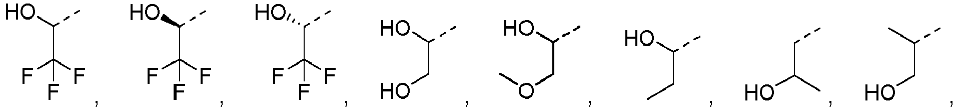 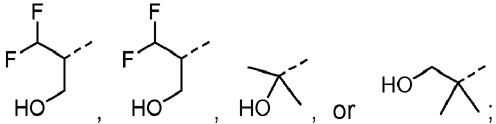
preferably **R¹** represents 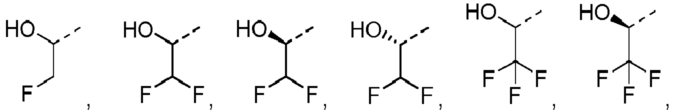 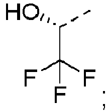 more preferably **R¹** represents 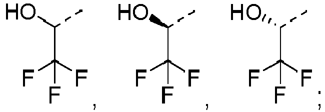
**R³** represents 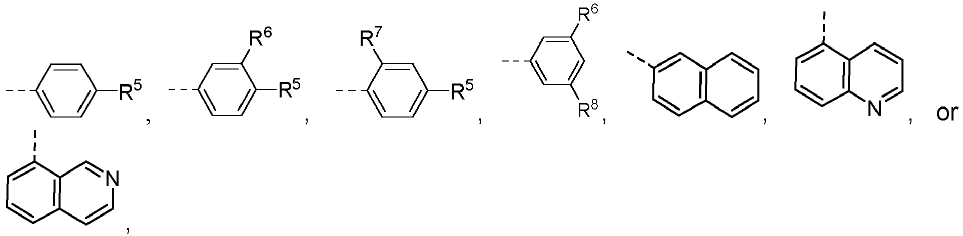
**R⁴** represents -H, -F, -Cl, -CH₃, -CF₃, -OCH₃, -OCHF₂, or -OCF₃; and
**R⁵**, **R⁶**, **R⁷**, and **R⁸** represent independently of each other -H, -F, -Cl, -CN, -CH₃, -CHF₂, -CF₃, -OCHF₂, -OCF₃, or -SO₂CH₃;
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

More preferred are the compounds of any one of the following formulae (**III-1**) to (**III-2**), (**III-4**) to (**III-6**), (**IV-1**) to (**IV-2**), and (**IV-4**) to (**IV-6**): 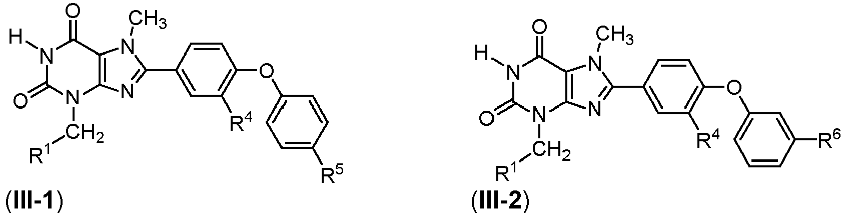 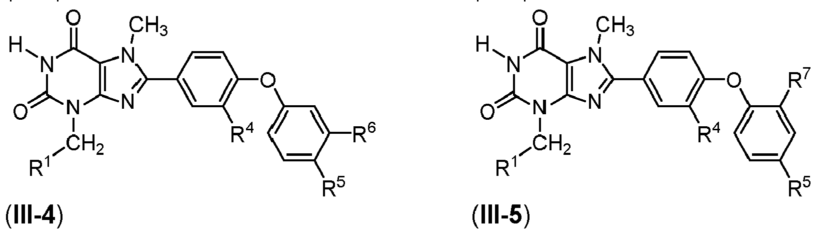 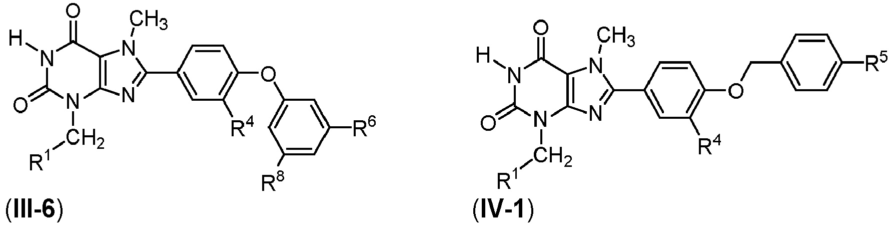 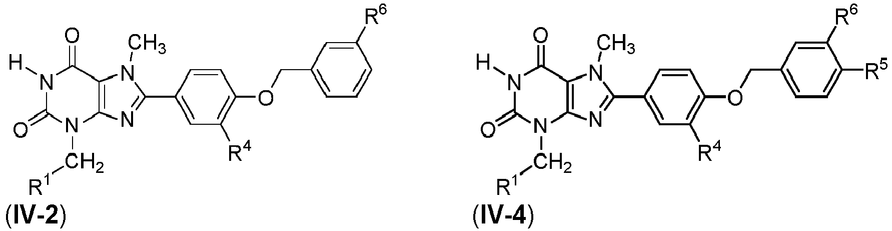 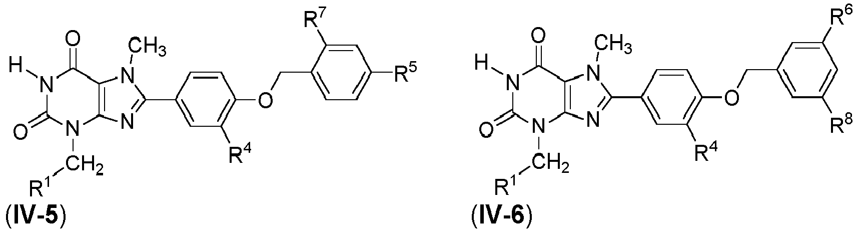 wherein
**R¹** represents 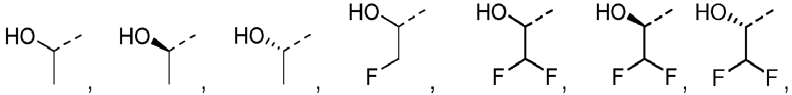 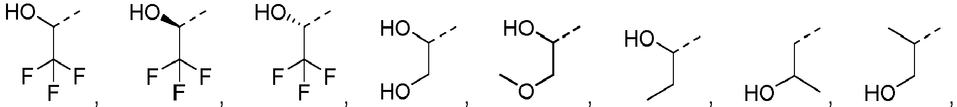 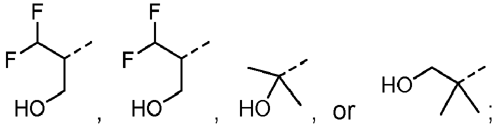
preferably **R¹** represents 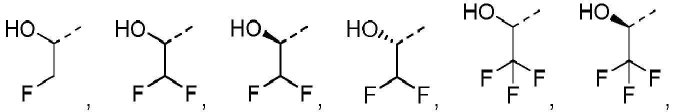 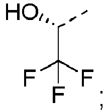 more preferably **R¹** represents 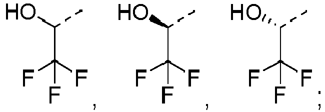
**R⁴** represents -F, -Cl, -Br, -CH₃, -CH₂F, -CHF₂, -CF₃, -CH₂CF₃, -CF₂CF₃, -OCH₃, -OCH₂F, -OCHF₂, -OCF₃, -OCH₂CH₂F, -OCH₂CF₃, or -OCF₂CF₃ ;
preferably, **R⁴** represents -H, -F, -Cl, -CH₃, -CF₃, -OCH₃, -OCHF₂, or -OCF₃; and
**R⁵**, **R⁶**, **R⁷**, and **R⁸** represent independently of each other -H, -F, -Cl, -CN, -CH₃, -CHF₂, -CF₃, -OCHF₂, -OCF₃, or -SO₂CH₃;
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

In some embodiment, the present invention relates to the compound of any one of the following formulae (**I**), (**II-1**) to (**II-5**), (**III-1**) to (**III-6**), (**IV-1**) to (**IV-6**), (**V-1**) to (**V-3**), wherein **R³** represents

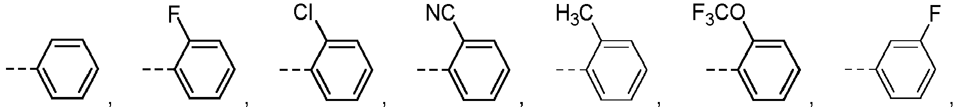

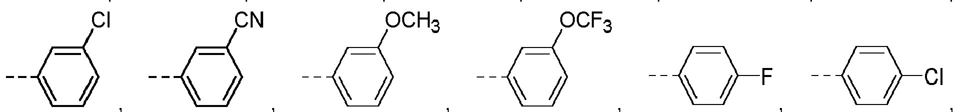

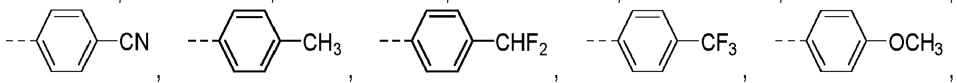

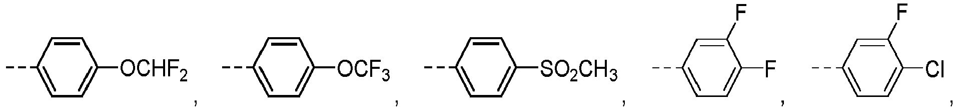

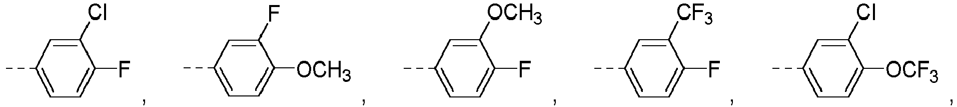

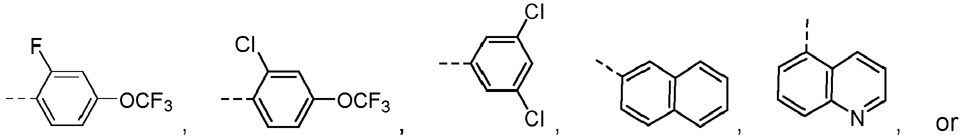

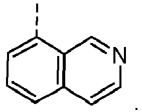

Preferably, **R³** represents

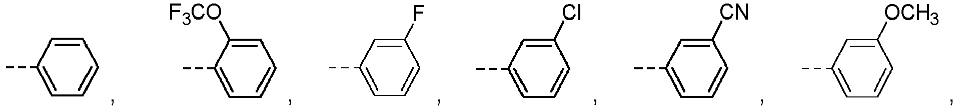

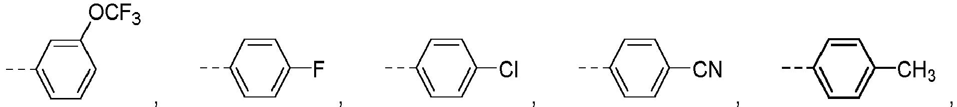

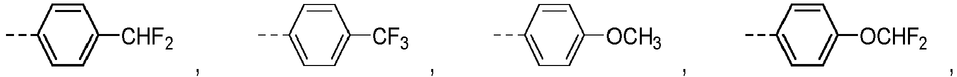

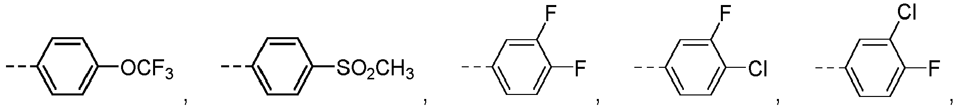

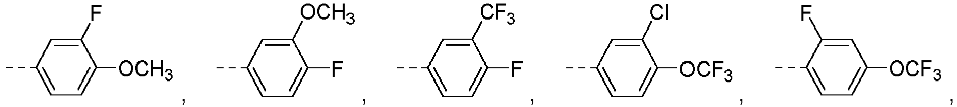

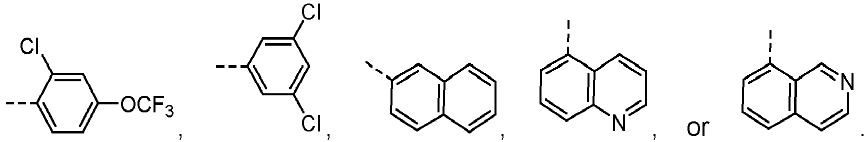

In a preferred embodiment, the present invention is directed to the compound of the formula (**I**), 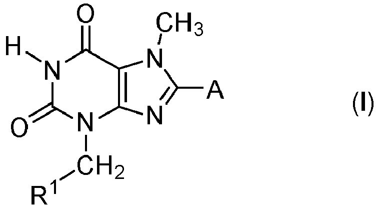 wherein
**A** represents 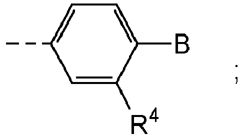
**B** represents -O-**R³** or -O-CH₂-**R³**;
**R¹** represents 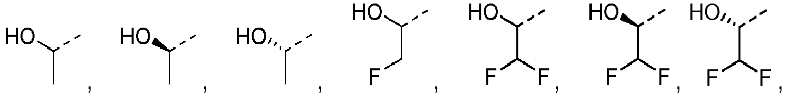 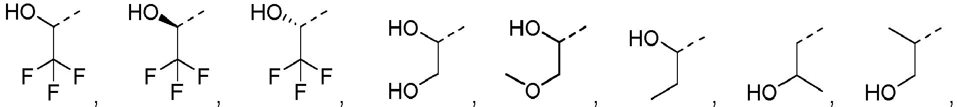 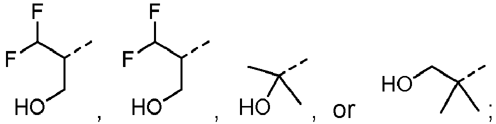
preferably **R¹** represents 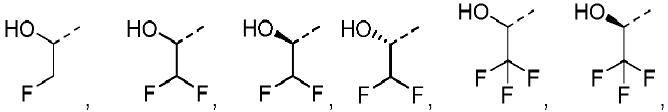 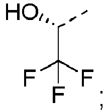 more preferably **R¹** represents 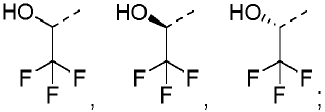
**R³** represents 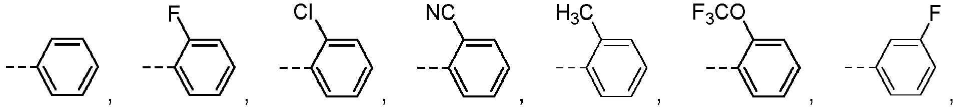 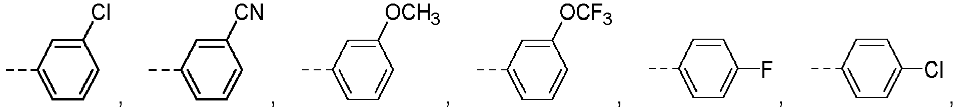 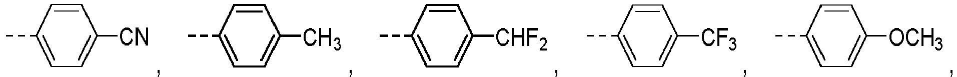 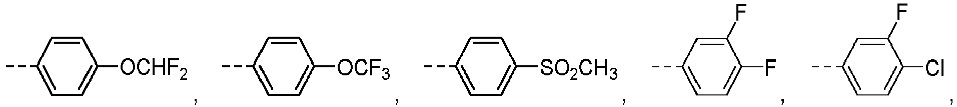 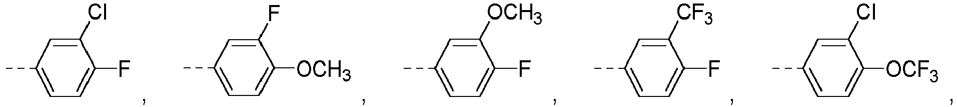 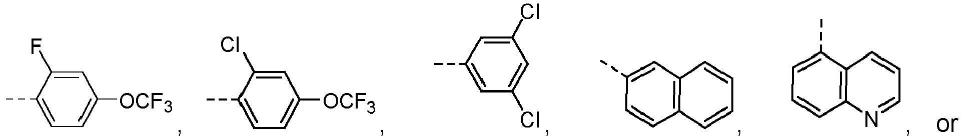 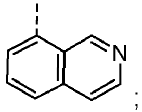
Preferably, **R³** represents 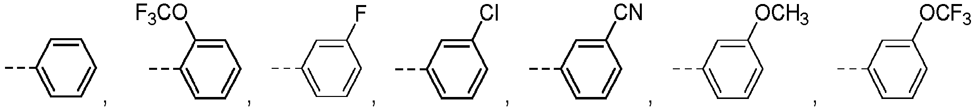 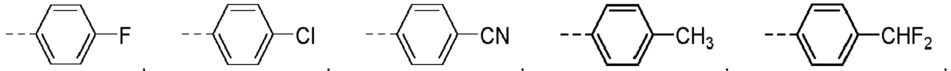 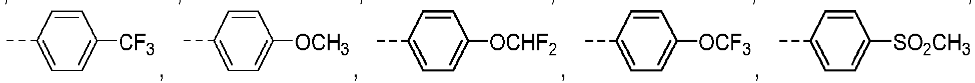 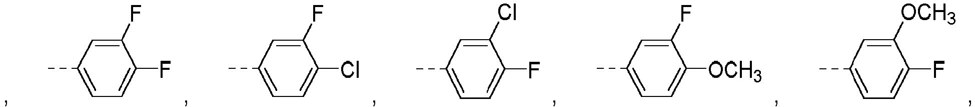 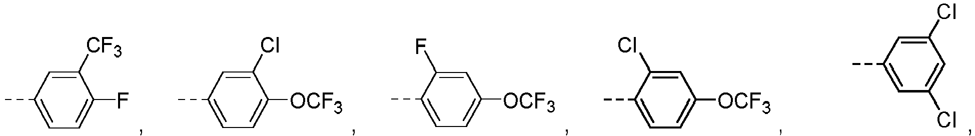 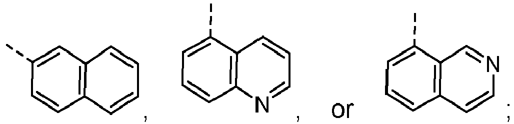
**R⁴** represents -H, -F, -Cl, -CH₃, -CF₃, -OCH₃, -OCHF₂, or -OCF₃; or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

Especially preferred compounds according to the present invention include compounds presented by **Table 1.**

**Table 1**

| **Cpd No** | **Structure** | **IUPAC-Name** |
|---|---|---|
| **16** | 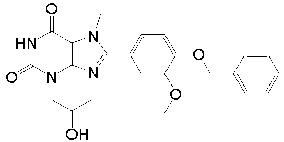 | 8-(4-(benzyloxy)-3-methoxyphenyl)-3-(2-hydroxypropyl)-7-methyl-3,7-dihydro-1H-purine-2,6-dione |
| **18** | 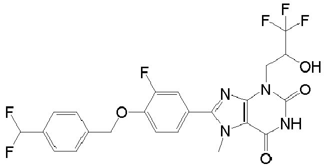 | 8-(4-(benzyloxy)-3-(trifluoromethoxy)phenyl)-3-(3,3-difluoro-2-(hydroxymethyl)propyl)-7-methyl-3,7-dihydro-1H-purine-2,6-dione |
| **19** | 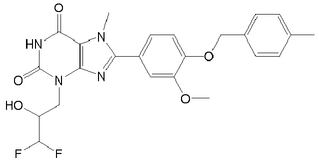 | 3-(3,3-difluoro-2-hydroxypropyl)-8-(3-methoxy-4-((4-methylbenzyl)oxy)phenyl)-7-methyl-3,7-dihydro-1H-purine-2,6-dione |
| **44** | 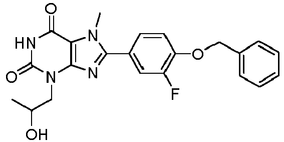 | 8-(4-(benzyloxy)-3-fluorophenyl)-3-(2-hydroxypropyl)-7-methyl-3,7-dihydro-1H-purine-2,6-dione |
| **52** | 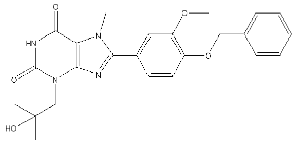 | 8-(4-(benzyloxy)-3-methoxyphenyl)-3-(2-hydroxy-2-methylpropyl)-7-methyl-3,7-dihydro-1H-purine-2,6-dione |
| **53** | 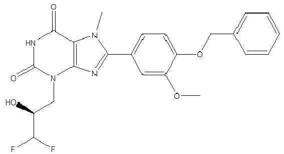 | (S)-8-(4-(benzyloxy)-3-methoxyphenyl)-3-(3,3-difluoro-2-hydroxypropyl)-7-methyl-3,7-dihydro-1H-purine-2,6-dione |
| **54** | 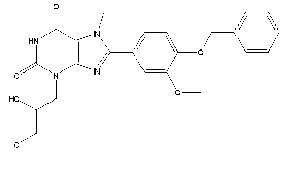 | 8-(4-(benzyloxy)-3-methoxyphenyl)-3-(2-hydroxy-3-methoxypropyl)-7-methyl-3,7-dihydro-1H-purine-2,6-dione |
| **55** | 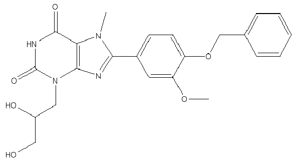 | 8-(4-(benzyloxy)-3-methoxyphenyl)-3-(2,3-dihydroxypropyl)-7-methyl-3,7-dihydro-1H-purine-2,6-dione |
| **56** | 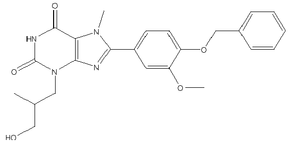 | 8-(4-(benzyloxy)-3-methoxyphenyl)-3-(3-hydroxy-2-methylpropyl)-7-methyl-3,7-dihydro-1H-purine-2,6-dione |
| **57** | 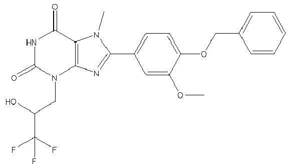 | 8-(4-(benzyloxy)-3-methoxyphenyl)-7-methyl-3-(3,3,3-trifluoro-2-hydroxypropyl)-3,7-dihydro-1H-purine-2,6-dione |
| **58** | 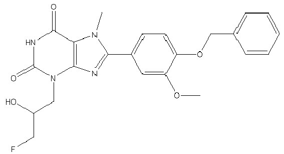 | 8-(4-(benzyloxy)-3-methoxyphenyl)-3-(3-fluoro-2-hydroxypropyl)-7-methyl-3,7-dihydro-1H-purine-2,6-dione |
| **59** | 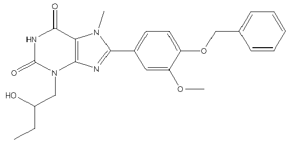 | 8-(4-(benzyloxy)-3-methoxyphenyl)-3-(2-hydroxybutyl)-7-methyl-3,7-dihydro-1H-purine-2,6-dione |
| **60** | 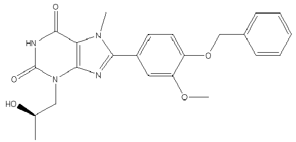 | (R)-8-(4-(benzyloxy)-3-methoxyphenyl)-3-(2-hydroxypropyl)-7-methyl-3,7-dihydro-1H-purine-2,6-dione |
| **61** | 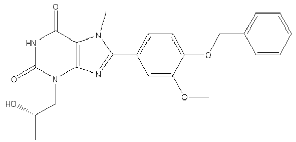 | (S)-8-(4-(benzyloxy)-3-methoxyphenyl)-3-(2-hydroxypropyl)-7-methyl-3,7-dihydro-1H-purine-2,6-dione |
| **62** | 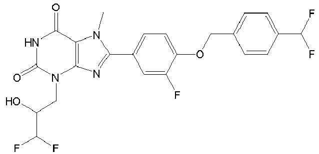 | 3-(3,3-difluoro-2-hydroxypropyl)-8-(4-((4-(difluoromethyl)benzyl)oxy)-3-fluorophenyl)-7-methyl-3,7-dihydro-1H-purine-2,6-dione |
| **63** | 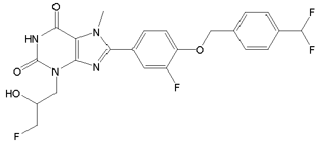 | 8-(4-((4-(difluoromethyl)benzyl)oxy)-3-fluorophenyl)-3-(3-fluoro-2-hydroxypropyl)-7-methyl-3,7-dihydro-1H-purine-2,6-dione |
| **64** | 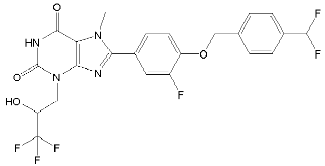 | 8-(4-((4-(difluoromethyl)benzyl)oxy)-3-fluorophenyl)-7-methyl-3-(3,3,3-trifluoro-2-hydroxypropyl)-3,7-dihydro-1H-purine-2,6-dione |
| **65** | 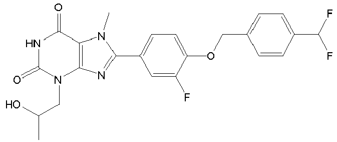 | 8-(4-((4-(difluoromethyl)benzyl)oxy)-3-fluorophenyl)-3-(2-hydroxypropyl)-7-methyl-3,7-dihydro-1H-purine-2,6-dione |
| **66** | 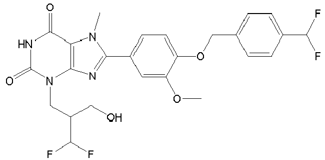 | 3-(3,3-difluoro-2-(hydroxymethyl)propyl)-8-(4-((4-(difluoromethyl)benzyl)oxy)-3-methoxyphenyl)-7-methyl-3,7-dihydro-1H-purine-2,6-dione |
| **67** | 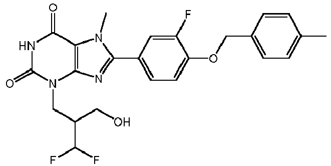 | 3-(3,3-difluoro-2-(hydroxymethyl)propyl)-8-(3-fluoro-4-((4-methylbenzyl)oxy)phenyl)-7-methyl-3,7-dihydro-1H-purine-2,6-dione |
| **68** | 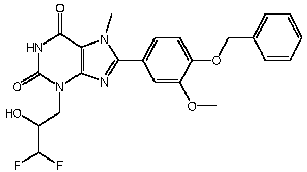 | 8-(4-(benzyloxy)-3-methoxyphenyl)-3-(3,3-difluoro-2-hydroxypropyl)-7-methyl-3,7-dihydro-1H-purine-2,6-dione |
| **72** | 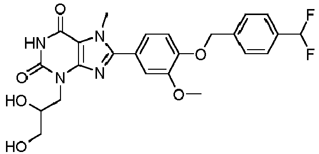 | 8-(4-((4-(difluoromethyl)benzyl)oxy)-3-methoxyphenyl)-3-(2,3-dihydroxypropyl)-7-methyl-3,7-dihydro-1H-purine-2,6-dione |
| **73** | 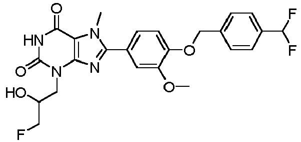 | 8-(4-((4-(difluoromethyl)benzyl)oxy)-3-methoxyphenyl)-3-(3-fluoro-2-hydroxypropyl)-7-methyl-3,7-dihydro-1H-purine-2,6-dione |
| **74** | 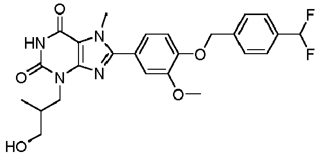 | 8-(4-((4-(difluoromethyl)benzyl)oxy)-3-methoxyphenyl)-3-(3-hydroxy-2-methylpropyl)-7-methyl-3,7-dihydro-1H-purine-2,6-dione |
| **75** | 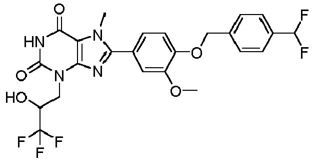 | 8-(4-((4-(difluoromethyl)benzyl)oxy)-3-methoxyphenyl)-7-methyl-3-(3,3,3-trifluoro-2-hydroxypropyl)-3,7-dihydro-1H-purine-2,6-dione |
| **76** | 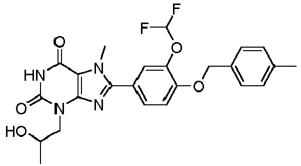 | 8-(3-(difluoromethoxy)-4-((4-methylbenzyl)oxy)phenyl)-3-(2-hydroxypropyl)-7-methyl-3,7-dihydro-1H-purine-2,6-dione |
| **77** | 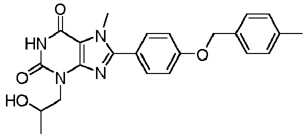 | 3-(2-hydroxypropyl)-7-methyl-8-(4-((4-methylbenzyl)oxy)phenyl)-3,7-dihydro-1H-purine-2,6-dione |
| **78** | 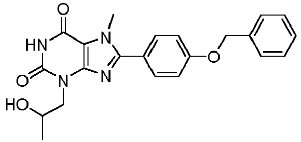 | 8-(4-(benzyloxy)phenyl)-3-(2-hydroxypropyl)-7-methyl-3,7-dihydro-1H-purine-2,6-dione |
| **79** | 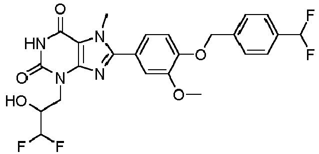 | 3-(3,3-difluoro-2-hydroxypropyl)-8-(4-((4-(difluoromethyl)benzyl)oxy)-3-methoxyphenyl)-7-methyl-3,7-dihydro-1H-purine-2,6-dione |
| **80** | 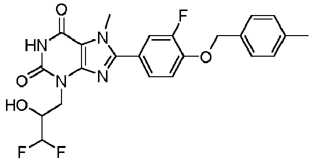 | 3-(3,3-difluoro-2-hydroxypropyl)-8-(3-fluoro-4-((4-methylbenzyl)oxy)phenyl)-7-methyl-3,7-dihydro-1H-purine-2,6-dione |
| **81** | 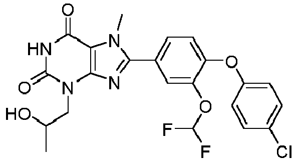 | 8-(4-(4-chlorophenoxy)-3-(difluoromethoxy) phenyl)-3-(2-hydroxypropyl)-7-methyl-3,7-dihydro-1H-purine-2,6-dione |
| **82** | 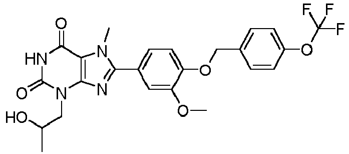 | 3-(2-hydroxypropyl)-8-(3-methoxy-4-((4-(trifluoromethoxy)benzyl)oxy)phenyl)-7-methyl-3,7-dihydro-1H-purine-2,6-dione |
| **83** | 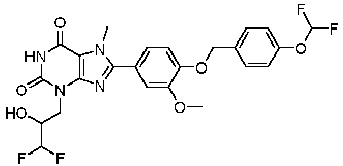 | 3-(3,3-difluoro-2-hydroxypropyl)-8-(4-((4-(difluoromethoxy)benzyl)oxy)-3-methoxyphenyl)-7-methyl-3,7-dihydro-1H-purine-2,6-dione |
| **84** | 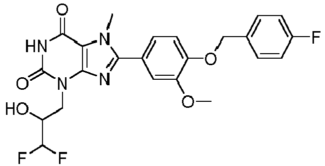 | 3-(3,3-difluoro-2-hydroxypropyl)-8-(4-((4-fluorobenzyl)oxy)-3-methoxyphenyl)-7-methyl-3,7-dihydro-1H-purine-2,6-dione |
| **85** | 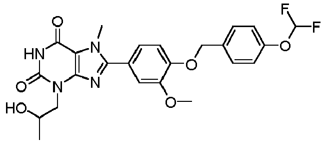 | 8-(4-((4-(difluoromethoxy)benzyl)oxy)-3-methoxyphenyl)-3-(2-hydroxypropyl)-7-methyl-3,7-dihydro-1H-purine-2,6-dione |
| **86** | 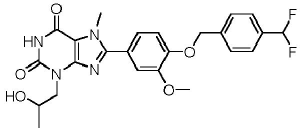 | 8-(4-((4-(difluoromethyl)benzyl)oxy)-3-methoxyphenyl)-3-(2-hydroxypropyl)-7-methyl-3,7-dihydro-1H-purine-2,6-dione |
| **87** | 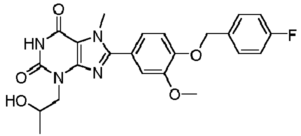 | 8-(4-((4-fluorobenzyl)oxy)-3-methoxyphenyl)-3-(2-hydroxypropyl)-7-methyl-3,7-dihydro-1H-purine-2,6-dione |
| **88** | 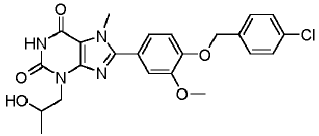 | 8-(4-((4-chlorobenzyl)oxy)-3-methoxyphenyl)-3-(2-hydroxypropyl)-7-methyl-3,7-dihydro-1H-purine-2,6-dione |
| **89** | 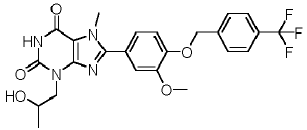 | 3-(2-hydroxypropyl)-8-(3-methoxy-4-((4-(trifluoromethyl)benzyl)oxy)phenyl)-7-methyl-3,7-dihydro-1H-purine-2,6-dione |
| **90** | 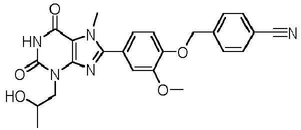 | 4-((4-(3-(2-hydroxypropyl)-7-methyl-2,6-dioxo-2,3,6,7-tetrahydro-1H-purin-8-yl)-2-methoxyphenoxy)methyl)benzonitrile |
| **91** | 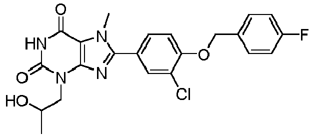 | 8-(3-chloro-4-((4-fluorobenzyl)oxy)phenyl)-3-(2-hydroxypropyl)-7-methyl-3,7-dihydro-1H-purine-2,6-dione |
| **92** | 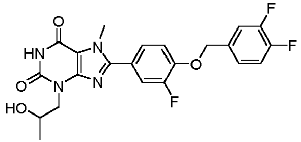 | 8-(4-((3,4-difluorobenzyl)oxy)-3-fluorophenyl)-3-(2-hydroxypropyl)-7-methyl-3,7-dihydro-1H-purine-2,6-dione |
| **93** | 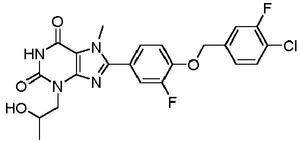 | 8-(4-((4-chloro-3-fluorobenzyl)oxy)-3-fluorophenyl)-3-(2-hydroxypropyl)-7-methyl-3,7-dihydro-1H-purine-2,6-dione |
| **94** | 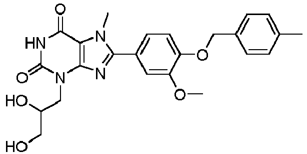 | 3-(2,3-dihydroxypropyl)-8-(3-methoxy-4-((4-methylbenzyl)oxy)phenyl)-7-methyl-3,7-dihydro-1H-purine-2,6-dione |
| **95** | 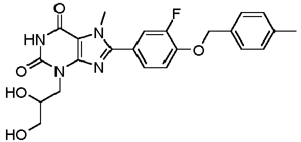 | 3-(2,3-dihydroxypropyl)-8-(3-fluoro-4-((4-methylbenzyl)oxy)phenyl)-7-methyl-3,7-dihydro-1H-purine-2,6-dione |
| **96** | 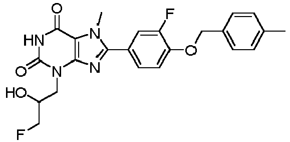 | 3-(3-fluoro-2-hydroxypropyl)-8-(3-fluoro-4-((4-methylbenzyl)oxy)phenyl)-7-methyl-3,7-dihydro-1H-purine-2,6-dione |
| **97** | 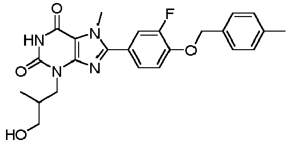 | 8-(3-fluoro-4-((4-methylbenzyl)oxy)phenyl)-3-(3-hydroxy-2-methylpropyl)-7-methyl-3,7-dihydro-1H-purine-2,6-dione |
| **98** | 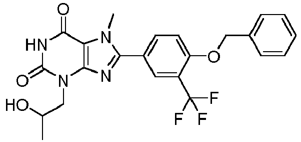 | 8-(4-(benzyloxy)-3-(trifluoromethyl)phenyl)-3-(2-hydroxypropyl)-7-methyl-3,7-dihydro-1H-purine-2,6-dione |
| **99** | 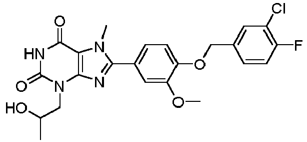 | 8-(4-((3-chloro-4-fluorobenzyl)oxy)-3-methoxyphenyl)-3-(2-hydroxypropyl)-7-methyl-3,7-dihydro-1H-purine-2,6-dione |
| **100** | 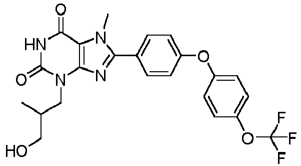 | 3-(3-hydroxy-2-methylpropyl)-7-methyl-8-(4-(4-(trifluoromethoxy) phenoxy) phenyl)-3,7-dihydro-1H-purine-2,6-dione |
| **101** | 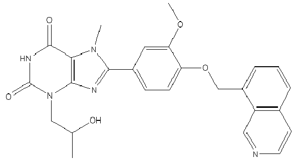 | 3-(2-hydroxypropyl)-8-(4-(isoquinolin-8-ylmethoxy)-3-methoxyphenyl)-7-methyl-3,7-dihydro-1H-purine-2,6-dione |
| **102** | 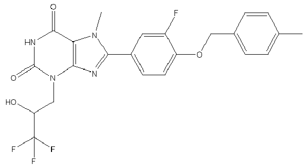 | 8-(3-fluoro-4-((4-methylbenzyl)oxy)phenyl)-7-methyl-3-(3,3,3-trifluoro-2-hydroxypropyl)-3,7-dihydro-1H-purine-2,6-dione |
| **103** | 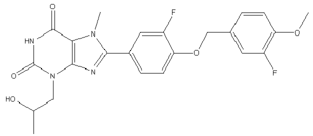 | 8-(3-fluoro-4-((3-fluoro-4-methoxybenzyl)oxy)phenyl)-3-(2-hydroxypropyl)-7-methyl-3,7-dihydro-1H-purine-2,6-dione |
| **113** | 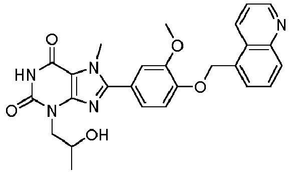 | 3-(2-hydroxypropyl)-8-(3-methoxy-4-(quinolin-5-ylmethoxy)phenyl)-7-methyl-3,7-dihydro-1H-purine-2,6-dione |
| **114** | 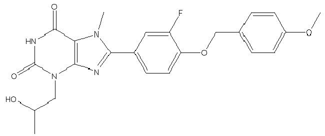 | 8-(3-fluoro-4-((4-methoxybenzyl)oxy)phenyl)-3-(2-hydroxypropyl)-7-methyl-3,7-dihydro-1H-purine-2,6-dione |
| **115** | 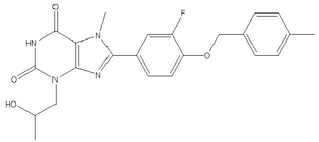 | 8-(3-fluoro-4-((4-methylbenzyl)oxy)phenyl)-3-(2-hydroxypropyl)-7-methyl-3,7-dihydro-1H-purine-2,6-dione |
| **116** | 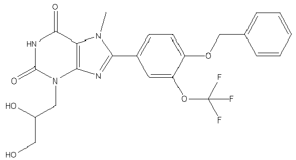 | 8-(4-(benzyloxy)-3-(trifluoromethoxy)phenyl)-3-(2,3-dihydroxypropyl)-7-methyl-3,7-dihydro-1H-purine-2,6-dione |
| **117** | 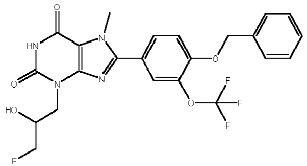 | 8-(4-(benzyloxy)-3-(trifluoromethoxy)phenyl)-3-(3-fluoro-2-hydroxypropyl)-7-methyl-3,7-dihydro-1H-purine-2,6-dione |
| **118** | 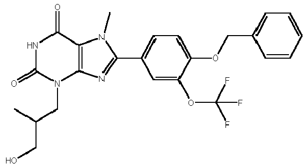 | 8-(4-(benzyloxy)-3-(trifluoromethoxy)phenyl)-3-(3-hydroxy-2-methylpropyl)-7-methyl-3,7-dihydro-1H-purine-2,6-dione |
| **119** | 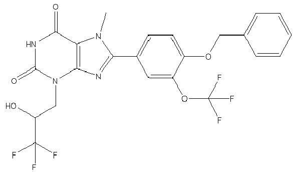 | 8-(4-(benzyloxy)-3-(trifluoromethoxy)phenyl)-7-methyl-3-(3,3,3-trifluoro-2-hydroxypropyl)-3,7-dihydro-1H-purine-2,6-dione |
| **120** | 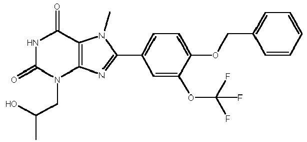 | 8-(4-(benzyloxy)-3-(trifluoromethoxy)phenyl)-3-(2-hydroxypropyl)-7-methyl-3,7-dihydro-1H-purine-2,6-dione |
| **121** | 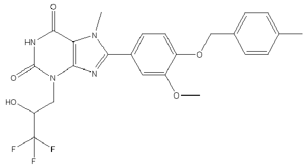 | 8-(3-methoxy-4-((4-methylbenzyl)oxy)phenyl)-7-methyl-3-(3,3,3-trifluoro-2-hydroxypropyl)-3,7-dihydro-1H-purine-2,6-dione |
| **122** | 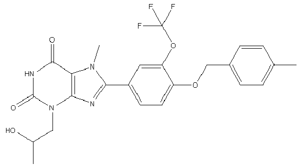 | 3-(2-hydroxypropyl)-7-methyl-8-(4-((4-methylbenzyl)oxy)-3-(trifluoromethoxy)phenyl)-3,7-dihydro-1H-purine-2,6-dione |
| **123** | 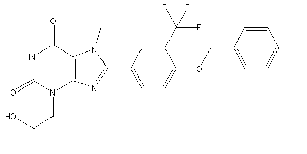 | 3-(2-hydroxypropyl)-7-methyl-8-(4-((4-methylbenzyl)oxy)-3-(trifluoromethyl)phenyl)-3,7-dihydro-1H-purine-2,6-dione |
| **124** | 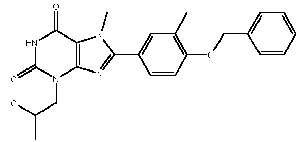 | 8-(4-(benzyloxy)-3-methylphenyl)-3-(2-hydroxypropyl)-7-methyl-3,7-dihydro-1H-purine-2,6-dione |
| **125** | 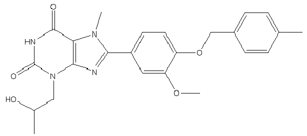 | 3-(2-hydroxypropyl)-8-(3-methoxy-4-((4-methylbenzyl)oxy)phenyl)-7-methyl-3,7-dihydro-1H-purine-2,6-dione |
| **126** | 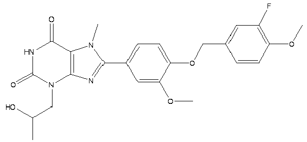 | 8-(4-((3-fluoro-4-methoxybenzyl)oxy)-3-methoxyphenyl)-3-(2-hydroxypropyl)-7-methyl-3,7-dihydro-1H-purine-2,6-dione |
| **127** | 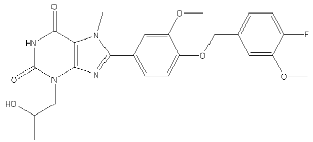 | 8-(4-((4-fluoro-3-methoxybenzyl)oxy)-3-methoxyphenyl)-3-(2-hydroxypropyl)-7-methyl-3,7-dihydro-1H-purine-2,6-dione |
| **128** | 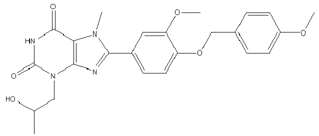 | 3-(2-hydroxypropyl)-8-(3-methoxy-4-((4-methoxybenzyl)oxy)phenyl)-7-methyl-3,7-dihydro-1H-purine-2,6-dione |
| **136** | 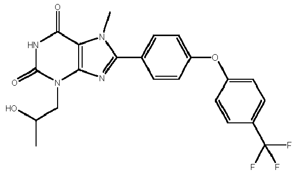 | 3-(2-hydroxypropyl)-7-methyl-8-(4-(4-(trifluoromethyl)phenoxy)phenyl)-3,7-dihydro-1H-purine-2,6-dione |
| **137** | 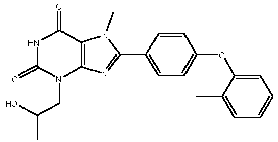 | 3-(2-hydroxypropyl)-7-methyl-8-(4-(o-tolyloxy)phenyl)-3,7-dihydro-1H-purine-2,6-dione |
| **138** | 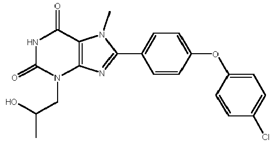 | 8-(4-(4-chlorophenoxy)phenyl)-3-(2-hydroxypropyl)-7-methyl-3,7-dihydro-1H-purine-2,6-dione |
| **139** | 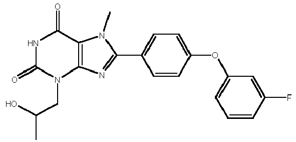 | 8-(4-(3-fluorophenoxy)phenyl)-3-(2-hydroxypropyl)-7-methyl-3,7-dihydro-1H-purine-2,6-dione |
| **140** | 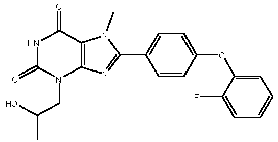 | 8-(4-(2-fluorophenoxy)phenyl)-3-(2-hydroxypropyl)-7-methyl-3,7-dihydro-1H-purine-2,6-dione |
| **141** | 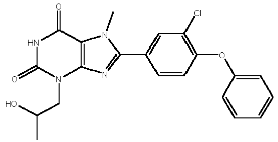 | 8-(3-chloro-4-phenoxyphenyl)-3-(2-hydroxypropyl)-7-methyl-3,7-dihydro-1H-purine-2,6-dione |
| **142** | 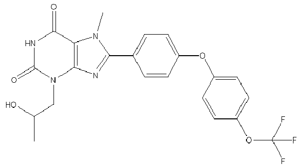 | 3-(2-hydroxypropyl)-7-methyl-8-(4-(4-(trifluoromethoxy)phenoxy)phenyl)-3,7-dihydro-1H-purine-2,6-dione |
| **147** | 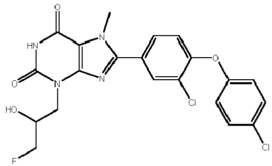 | 8-(3-chloro-4-(4-chlorophenoxy)phenyl)-3-(3-fluoro-2-hydroxypropyl)-7-methyl-3,7-dihydro-1H-purine-2,6-dione |
| **148** | 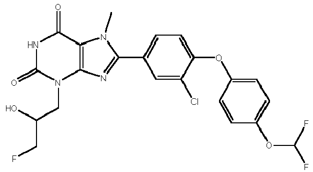 | 8-(3-chloro-4-(4-(difluoromethoxy)phenoxy)phenyl)-3-(3-fluoro-2-hydroxypropyl)-7-methyl-3,7-dihydro-1H-purine-2,6-dione |
| **149** | 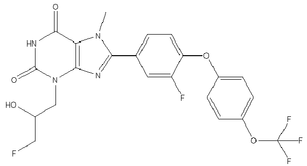 | 3-(3-fluoro-2-hydroxypropyl)-8-(3-fluoro-4-(4-(trifluoromethoxy)phenoxy)phenyl)-7-methyl-3,7-dihydro-1H-purine-2,6-dione |
| **150** | 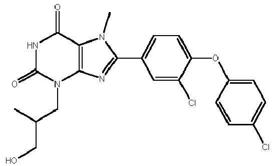 | 8-(3-chloro-4-(4-chlorophenoxy)phenyl)-3-(3-hydroxy-2-methylpropyl)-7-methyl-3,7-dihydro-1H-purine-2,6-dione |
| **151** | 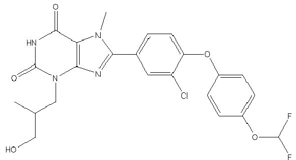 | 8-(3-chloro-4-(4-(difluoromethoxy)phenoxy)phenyl)-3-(3-hydroxy-2-methylpropyl)-7-methyl-3,7-dihydro-1H-purine-2,6-dione |
| **152** | 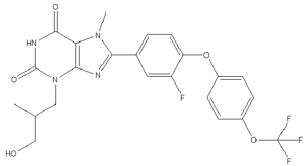 | 8-(3-fluoro-4-(4-(trifluoromethoxy)phenoxy)phenyl)-3-(3-hydroxy-2-methylpropyl)-7-methyl-3,7-dihydro-1H-purine-2,6-dione |
| **153** | 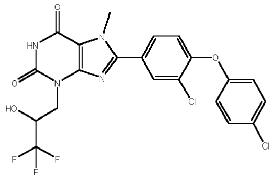 | 8-(3-chloro-4-(4-chlorophenoxy)phenyl)-7-methyl-3-(3,3,3-trifluoro-2-hydroxypropyl)-3,7-dihydro-1H-purine-2,6-dione |
| **155** | 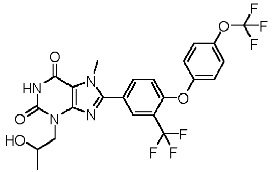 | 3-(2-hydroxypropyl)-7-methyl-8-(4-(4-(trifluoromethoxy)phenoxy)-3-(trifluoromethyl)phenyl)-3,7-dihydro-1H-purine-2,6-dione |
| **156** | 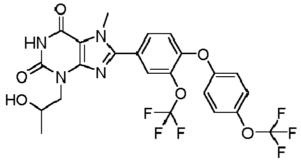 | 3-(2-hydroxypropyl)-7-methyl-8-(3-(trifluoromethoxy)-4-(4-(trifluoromethoxy)phenoxy)phenyl)-3,7-dihydro-1H-purine-2,6-dione |
| **161** | 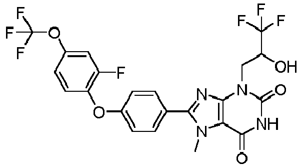 | 8-(4-(2-fluoro-4-(trifluoromethoxy)phenoxy)phenyl)-7-methyl-3-(3,3,3-trifluoro-2-hydroxypropyl)-3,7-dihydro-1H-purine-2,6-dione |
| **162** | 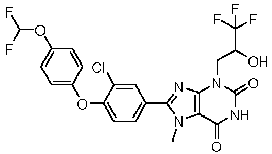 | 8-(3-chloro-4-(4-(difluoromethoxy)phenoxy)phenyl)-7-methyl-3-(3,3,3-trifluoro-2-hydroxypropyl)-3,7-dihydro-1H-purine-2,6-dione |
| **163** | 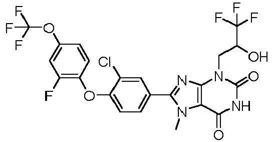 | 8-(3-chloro-4-(2-fluoro-4-(trifluoromethoxy)phenoxy)phenyl)-7-methyl-3-(3,3,3-trifluoro-2-hydroxypropyl)-3,7-dihydro-1H-purine-2,6-dione |
| **164** | 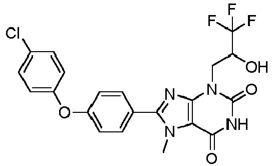 | 8-(4-(4-chlorophenoxy)phenyl)-7-methyl-3-(3, 3, 3-trifl uoro-2-hyd roxypropyl)-3, 7-dihydro-1H-purine-2,6-dione |
| **165** | 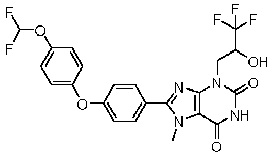 | 8-(4-(4-(difluoromethoxy)phenoxy)phenyl)-7-methyl-3-(3,3,3-trifluoro-2-hydroxypropyl)-3,7-dihydro-1H-purine-2,6-dione |
| **166** | 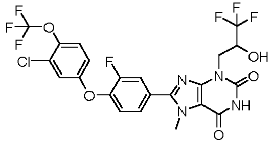 | 8-(4-(3-chloro-4-(trifluoromethoxy)phenoxy)-3-fluorophenyl)-7-methyl-3-(3,3,3-trifluoro-2-hydroxypropyl)-3,7-dihydro-1H-purine-2,6-dione |
| **167** | 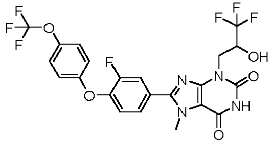 | 8-(3-fluoro-4-(4-(trifluoromethoxy)phenoxy)phenyl)-7-methyl-3-(3,3,3-trifluoro-2-hydroxypropyl)-3,7-dihydro-1H-purine-2,6-dione |
| **176** | 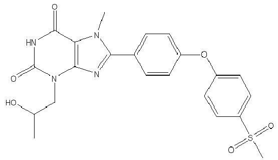 | 3-(2-hydroxypropyl)-7-methyl-8-(4-(4-(methylsulfonyl)phenoxy)phenyl)-3, 7-dihydro-1H-purine-2,6-dione |
| **177** | 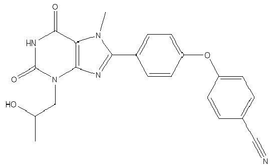 | 4-(4-(3-(2-hydroxypropyl)-7-methyl-2,6-dioxo-2,3,6,7-tetrahydro-1H-purin-8-yl)phenoxy)benzonitrile |
| **178** | 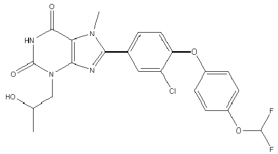 | 8-(3-chloro-4-(4-(difluoromethoxy)phenoxy)phenyl)-3-(2-hydroxypropyl)-7-methyl-3,7-dihydro-1H-purine-2,6-dione |
| **179** | 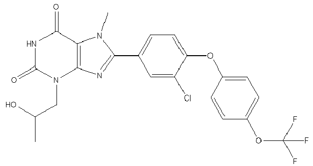 | 8-(3-chloro-4-(4-(trifluoromethoxy)phenoxy)phenyl)-3-(2-hydroxypropyl)-7-methyl-3,7-dihydro-1H-purine-2,6-dione |
| **180** | 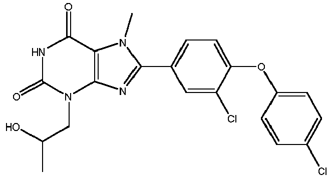 | 8-(3-chloro-4-(4-chlorophenoxy)phenyl)-3-(2-hydroxypropyl)-7-methyl-3,7-dihydro-1H-purine-2,6-dione |
| **181** | 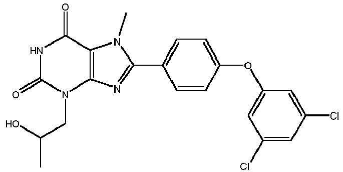 | 8-(4-(3,5-dichlorophenoxy)phenyl)-3-(2-hydroxypropyl)-7-methyl-3,7-dihydro-1H-purine-2,6-dione |
| **182** | 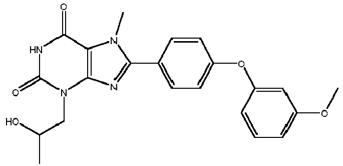 | 3-(2-hydroxypropyl)-8-(4-(3-methoxyphenoxy)phenyl)-7-methyl-3,7-dihydro-1H-purine-2,6-dione |
| **183** | 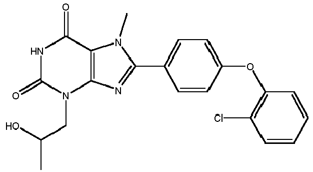 | 8-(4-(2-chlorophenoxy)phenyl)-3-(2-hydroxypropyl)-7-methyl-3,7-dihydro-1H-purine-2,6-dione |
| **184** | 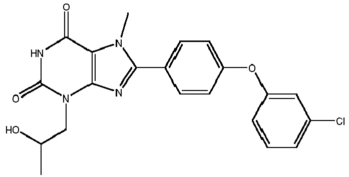 | 8-(4-(3-chlorophenoxy)phenyl)-3-(2-hydroxypropyl)-7-methyl-3,7-dihydro-1H-purine-2,6-dione |
| **185** | 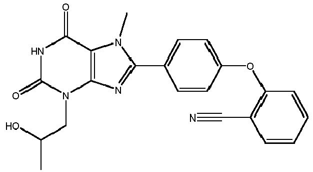 | 2-(4-(3-(2-hydroxypropyl)-7-methyl-2,6-dioxo-2,3,6,7-tetrahydro-1H-purin-8-yl)phenoxy)benzonitrile |
| **186** | 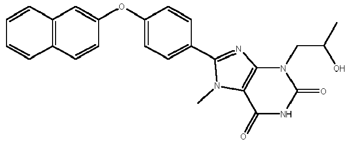 | 3-(2-hydroxypropyl)-7-methyl-8-(4-(naphthalen-2-yloxy)phenyl)-3,7-dihydro-1H-purine-2,6-dione |
| **187** | 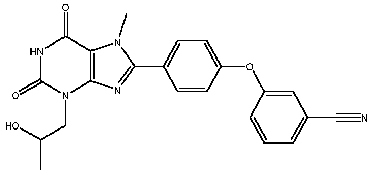 | 3-(4-(3-(2-hydroxypropyl)-7-methyl-2,6-dioxo-2,3,6,7-tetrahydro-1H-purin-8-yl)phenoxy)benzonitrile |
| **188** | 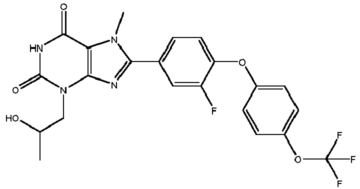 | 8-(3-fluoro-4-(4-(trifluoromethoxy)phenoxy)phenyl)-3-(2-hydroxypropyl)-7-methyl-3,7-dihydro-1H-purine-2,6-dione |
| **190** | 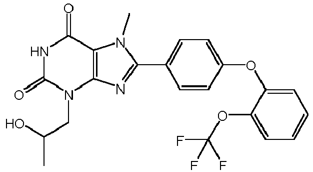 | 3-(2-hydroxypropyl)-7-methyl-8-(4-(2-(trifluoromethoxy)phenoxy)phenyl)-3,7-dihydro-1H-purine-2,6-dione |
| **191** | 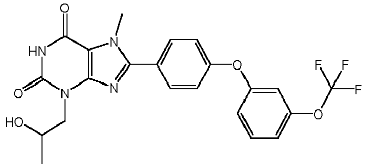 | 3-(2-hydroxypropyl)-7-methyl-8-(4-(3-(trifluoromethoxy)phenoxy)phenyl)-3,7-dihydro-1H-purine-2,6-dione |
| **192** | 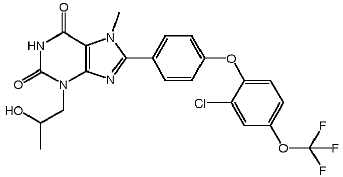 | 8-(4-(2-chloro-4-(trifluoromethoxy)phenoxy)phenyl)-3-(2-hydroxypropyl)-7-methyl-3,7-dihydro-1H-purine-2,6-dione |
| **193** | 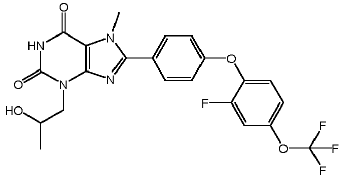 | 8-(4-(2-fluoro-4-(trifluoromethoxy)phenoxy)phenyl)-3-(2-hydroxypropyl)-7-methyl-3,7-dihydro-1H-purine-2,6-dione |
| **194** | 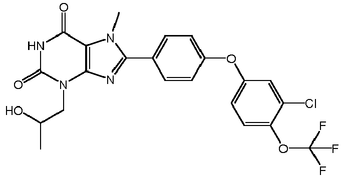 | 8-(4-(3-chloro-4-(trifluoromethoxy)phenoxy)phenyl)-3-(2-hydroxypropyl)-7-methyl-3,7-dihydro-1H-purine-2,6-dione |
| **195** | 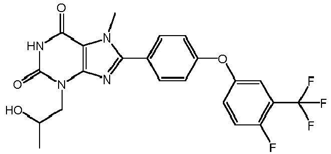 | 8-(4-(4-fluoro-3-(trifluoromethyl)phenoxy)phenyl)-3-(2-hydroxypropyl)-7-methyl-3,7-dihydro-1H-purine-2,6-dione |
| **196** | 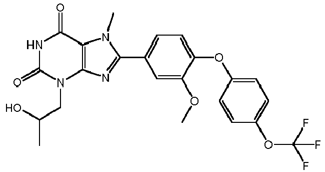 | 3-(2-hydroxypropyl)-8-(3-methoxy-4-(4-(trifluoromethoxy)phenoxy)phenyl)-7-methyl-3,7-dihydro-1H-purine-2,6-dione |
| **197** | 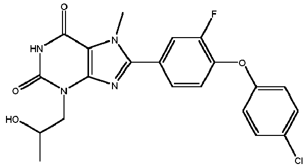 | 8-(4-(4-chlorophenoxy)-3-fluorophenyl)-3-(2-hydroxypropyl)-7-methyl-3,7-dihydro-1H-purine-2,6-dione |
| **198** | 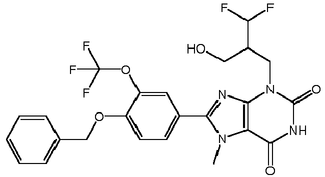 | 8-(4-(benzyloxy)-3-(trifluoromethoxy)phenyl)-3-(3,3-difluoro-2-(hydroxymethyl)propyl)-7-methyl-3,7-dihydro-1H-purine-2,6-dione |
| **199** | 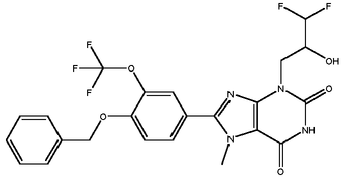 | 8-(4-(benzyloxy)-3-(trifluoromethoxy)phenyl)-3-(3,3-difluoro-2-hydroxypropyl)-7-methyl-3,7-dihydro-1H-purine-2,6-dione |
| **200** | 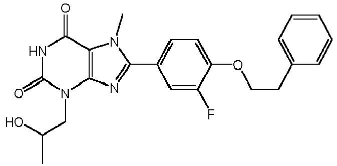 | 8-(3-fluoro-4-phenethoxyphenyl)-3-(2-hydroxypropyl)-7-methyl-3,7-dihydro-1H-purine-2,6-dione |
| **201** | 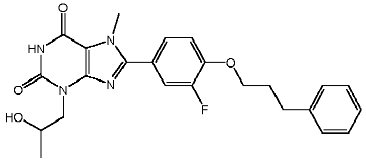 | 8-(3-fluoro-4-(3-phenylpropoxy)phenyl)-3-(2-hydroxypropyl)-7-methyl-3,7-dihydro-1H-purine-2,6-dione |

or an enantiomer, a diastereomer, a tautomer, a mixture of enantiomers, a mixture of diastereomers, a mixture of tautomers, a hydrate, a solvate, a pharmaceutically acceptable salt thereof.

### Syntheses of the compounds

The compound of formula (I) can be prepared by reference to the methods illustrated by the following reaction protocols. The compound of formula (I) can be produced as outlined below by the suitable selection of reagents with appropriate substitution. Solvents, temperatures, pressures, and other reaction conditions may readily be selected by one of ordinary skill in the art. Starting materials are commercially available or readily prepared by one of ordinary skill in the art. Other synthetic routes to prepare the compound of formula (I) can be applied in analogy to methods disclosed in the literature by one of ordinary skill in the art.

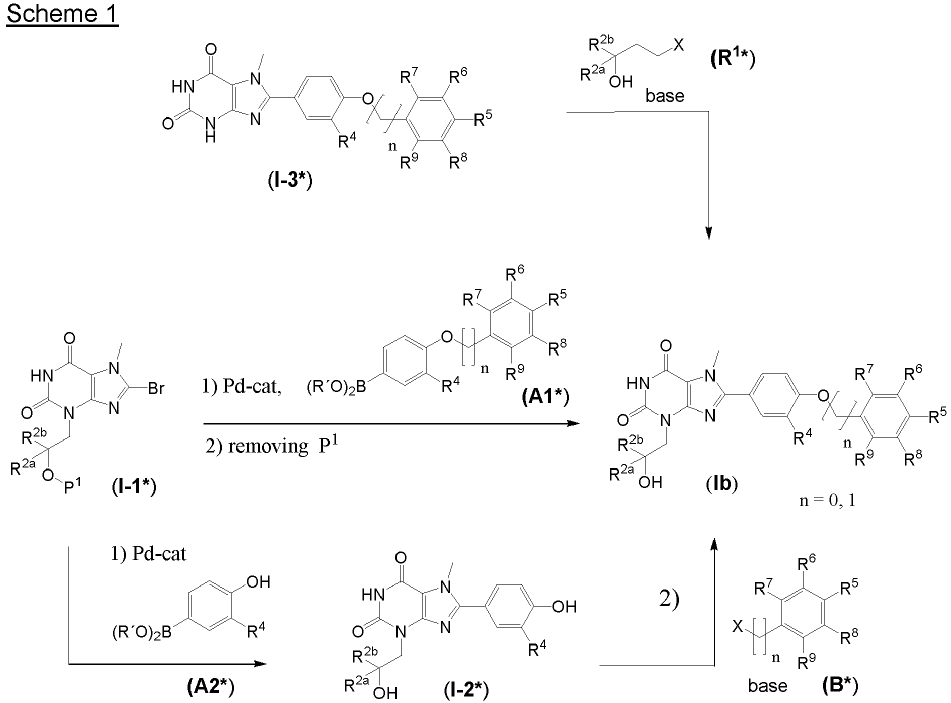

The present invention is also directed to a method for producing the compounds of formula (**Ib**), 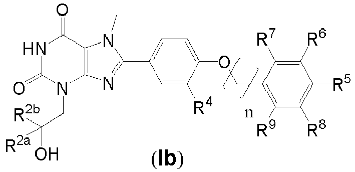 comprising the following steps:
Step A) providing a compound (**I-1***) 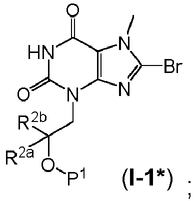
Step B1)
B1a) performing a C-C coupling reaction between the compound (**I-1***) and (**A1***) in a presence of a Pd-catalyst and a base 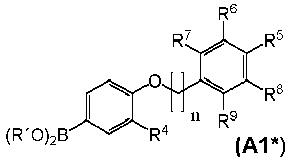 to obtain a compound **(I-4***);
B1b) removing a protecting group P¹ from the compound (**I-4***) 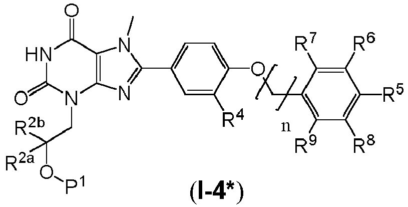 to obtain the compound (**Ib**); 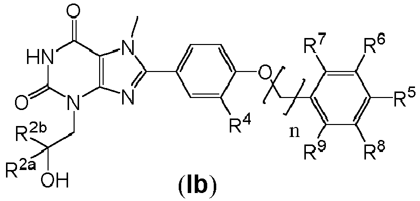 wherein
   **X** represent a leaving group, preferably halogen;
   **P¹** represents a hydroxy protecting group;
   **R'** represents hydrogen, or C₁-₃ alkyl; or two **R'** form together a pinacol moiety;
   **n**, **R^{2a}**, **R^{2b}**, **R⁴**, and **R⁵** to **R⁹** have the same meanings as defined in the formula (**Ib**).

Alternatively, a method for producing the compounds of formula (**Ib**) comprising the following steps:
Step A) providing a compound (**I-1***) 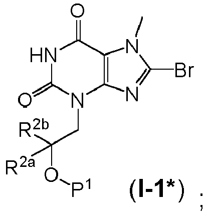
Step B2)
B2a) performing a C-C coupling reaction between the compounds (**I-1***) and (**A2***) in a presence of a Pd-catalyst and a base 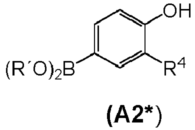 to obtain a compound (**I-2***) 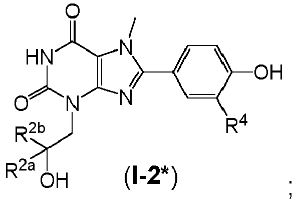
B2b) performing a coupling reaction between the compounds (**I-2***) and (**B***) 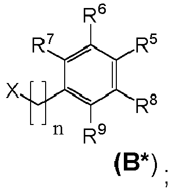 to obtain the compound (**Ib**) 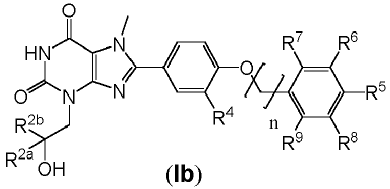 wherein
   **X** represent a leaving group, preferably halogen;
   **P¹** represents a hydroxy protecting group;
   **R'** represents hydrogen, or C₁-₃ alkyl; or two **R'** form together a pinacol moiety;
   **n**, **R^{2a}**, **R^{2b}**, **R⁴**, and **R⁵** to **R⁹** have the same meanings as defined in the formula (**Ib**).

Alternatively, a method for producing the compounds of formula (**Ib**) comprising the following steps:
Step A2) providing a compound (**I-3***) 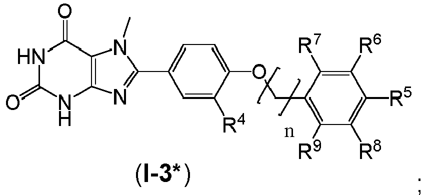
Step B3)
B2a) performing a coupling reaction between the compounds (**I-3***) and (**R¹***) 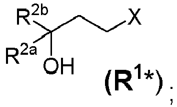 to obtain a compound (**Ib**) 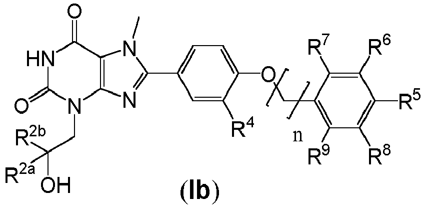 wherein
   **X** represent a leaving group, preferably halogen;
   **n**, **R^{2a}**, **R^{2b}**, **R⁴**, and **R⁵** to **R⁹** have the same meanings as defined in the formula (**Ib**).

In the step **B1a**)**,** Suzuki coupling reaction of brominated compound (**I-1***) with a compound **A1*** as a boronic acid derivative, 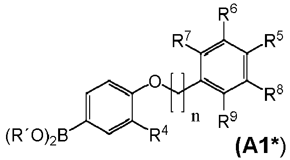 or in the step **B2a**), Suzuki coupling reaction of brominated compound (**I-1***) with a compound **A2*** as a boronic acid derivative 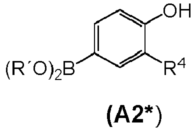 is performed in the presence of the palladium catalyst and the base,
wherein **R'** represents hydrogen, or C₁-₃ alkyl; or two **R'** form together a pinacol moiety;
preferred, the boronic acid derivative (**A1***) or (**A2***) may be a boronic acid (R' = -H) or an ester of the boronic acid, e.g. its isopropyl ester (R' = -CH(CH₃)₂), a pinacol moiety (R'-R' = -C(CH₃)₂-C(CH₃)₂-).
The palladium catalyst is Pd(0) or Pd(II) catalyst. The Pd(0) catalyst may be tetrakis(triphenylphosphine)palladium(0) [Pd(PPh₃)₄], tris(dibenzylideneacetone)di-palladium(0) [Pd₂(dba)₃]. Pd(II) catalyst may be dichlorobis(triphenylphosphine)-palladium(II) [Pd(PPh₃)₂Cl₂], palladium(II) acetate and triphenylphosphine or more preferred [1,1'-bis(diphenylphosphino)ferrocene]palladium dichloride (Pd(dppf)Cl₂). The reaction is preferably carried out in a mixture of a solvent like dioxane, DMF, DME, THF, or isopropanol with water and in the presence of the base like aqueous sodium bicarbonate or K₃PO₄.

P¹ is hydroxy protecting group and preferred is silicon based protecting group selected from trimethylsilyl (TMS), triethylsilyl (TES), isopropyldimethylsilyl (IPMDS), diethylisopropylsilyl (DEIPS), tert-butyldimethylsilyl (TBS), tert-butyldiphenylsilyl (TBDPS), triisopropylsilyl (TIPS), [2-(trimethylsilyl)ethoxy]methyl (SEM), 2-(trimethylsilyl)ethxy carbonate (Teoc), more preferred, tert-butyldimethylsilyl (TBS), or tert-butyldiphenylsilyl (TBDPS).

### Medical Use

Surprisingly, it was found that the compounds of the present invention effectively activate immune cells, in particular, CD4+ and/or CD8+ cells as demonstrated in Table B-1 and natural killer (NK) cells in the context of T cell receptor (TCR) as well as NK cell receptor signalling, respectively. This allows highly selective TCR- or NK cell receptor-mediated activation of the immune system against mutated tissue or tissue infected with foreign pathogens. Hence, by applying the present invention in a clinical set up highly effective treatment can be achieved by minimizing the risk of severe side effects. This is further evident due to the possibility of administering highly potent compounds based on EC₅₀ below nanomolar concentrations. This present invention therefore opens a broad dosing window in order to tip the balance between effective treatment and toxic off target effects for sustained tolerability in the course of multiple treatments.

As indicated above, the present invention also refers to the use of the compound of the present invention in a pharmaceutical context. Herein, a pharmaceutical context may be understood in the broadest sense as any means for improving a patient's health status and/or wellness. The terms "pharmaceutical" and "medicinal" may be understood interchangeably.

A further aspect of the present invention refers to a pharmaceutical composition comprising at least one compound of the present invention and at least one pharmaceutically acceptable carrier.

Preferably, a pharmaceutical composition comprising at least one compound of the present invention and at least one pharmaceutically acceptable carrier, excipient and/or diluent. More preferably, said pharmaceutical composition further compries at least one stimulating agent for activating immune cells.

In this aspect relating to a pharmaceutical composition, the definitions as laid out in detail above also apply *mutatis mutandis.*

A pharmaceutically acceptable carrier according to the present invention may be any additive that is pharmaceutically acceptable, therefore, any additive that is non-toxic to the patient. Exemplarily, a pharmaceutically acceptable carrier may comprise a solvent such as, e.g., water, dimethyl sulfoxide (DMSO), ethanol, vegetable oil, paraffin oil or combinations thereof. Furthermore, a carrier may contain one or more detergent(s), one or more foaming agent(s) (e.g., sodium lauryl sulfate (SLS)/ sodium dodecyl sulfate (SDS)), one or more coloring agent(s) (e.g., TiO₂ , food coloring), one or more vitamin(s), one or more salt(s) (e.g., sodium, potassium, calcium, zinc salts), one or more humectant(s) (e.g., sorbitol, glycerol, mannitol, propylene glycol, polydextrose), one or more enzyme(s), one or more preserving agent(s) (e.g., benzoic acid, methylparabene), one or more texturing agent(s) (e.g., carboxymethyl cellulose (CMC), polyethylene glycol (PEG), sorbitol), one or more emulsifier(s), one or more bulking agent(s), one or more glacing agent(s), one or more separating agent(s), one or more antioxidant(s), one or more herbal and plant extract(s), one or more stabilizing agent(s), one or more polymer(s) (e.g., hydroxypropyl methacrylamide (HPMA), polyethylene imine (PEI), carboxymethyl cellulose (CMC), polyethylene glycol (PEG)), one or more uptake mediator(s) (e.g., polyethylene imine (PEI), dimethyl sulfoxide (DMSO), a cell-penetrating peptide (CPP), a protein transduction domain (PTD), an antimicrobial peptide, etc.) one or more antibody/antibodies, one or more sweetener(s) (e.g., sucrose, acesulfame K, saccharin Na, stevia), one or more counterstain dye(s) (e.g., fluorescein, fluorescein derivatives, Cy dyes, an Alexa Fluor dye(s), S dye(s), rhodamine, quantum dot(s), etc.), one or more homeopathic ingredient(s) one or more gustatory substance(s) and/or one or more fragrance(s).

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

Suitable excipients are binders, disintegrants, lubricants, glidents and/or coloring agents.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminium silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition. Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminium oxide. The amount of the coloring agent may vary from about 0.1 to about 5 weight % of the composition, preferably from about 0.1 to about 1 weight %.

A pharmaceutical composition of the present invention comprises at least one compound of the present invention. Optionally, the pharmaceutical composition may also comprise more than one compound of the present invention such as the combination of two, three, four, five or even more compounds of the present invention. Optionally, the pharmaceutical composition may also comprise one or more other pharmaceutically active agent(s), such as, e.g., one or more further stimulating agent(s) activating immune cells which may be other pharmaceutically active ingredients of the pharmaceutical composition other than the compounds of the present invention. Examples for such further stimulating agent(s) activating immune cells are provided below.

The compound as well as a pharmaceutically acceptable salt thereof and a pharmaceutical composition of the present invention may be used as a medicament.

Therefore, another aspect of the present invention relates to the compound or the pharmaceutical composition of the present invention for use as a medicament.

In this aspect relating to the use as medicament, the definitions as laid out in detail above also apply mutatis mutandis.

In the context of the present invention, the terms "medicament", "therapeutic", "medicine", "drug", "therapeutic agent", "pharmaceutic", "pharmaceutical agent", "prophylactic agent" etc. may be understood in the broadest sense as any kind of compound suitable for being used in a medicinal context, i.e., for treating and/or preventing a pathological condition.

A compound or a pharmaceutical composition comprising such may be administered to the patient by any means known in the art such as, e.g, orally, via injection, nasally, transdermally/percutaneously, etc. Administration may be local administration (e.g., intratumorally, intranodally (i.e., into lymph nodes), intrathecally, intracerebroventricularly (icv), topically or intravitreally) or systemic administration (e.g., intravenously (i.v.), intraarterially (i.a.), intraperitoneally (i.p.), intramusculary (i.m.), subcutaneously (s.c.), orally, nasally). Preferably, administration is oral, intraveneous, subcutaneous, intratumoral or intranodal administration, in particular oral or intraveneous administration.

Administration may be administration once ((acute) single administration) or may be a repeated administration such as, e.g., administration of repeated pulse doses or chronic administration. Repeated administration may exemplarily be administration two times, three times, four times, five times, six times, seven times, eight times, nine times, ten times, more than ten times or even permanently. Between two administrations, there may be a time interval of less than one hour, one hour or more, six hours or more, twelve hours or more, 24 hours or more. Administration may be daily, may be twice daily, three times daily, four times daily, every second day, every three days, weekly, biweekly, monthly, twice a year or yearly. Clinically viable administration schemes may be determined by the person skilled in the art based on balancing efficacy and toxicity. Preferably, the medicament is suitable for treating or preventing pathologic conditions associated with an insufficient immune response. In other words, the present invention also relates to a medicament suitable for increasing an immune response. As used in the context of the activity of an immune response, the terms "activating", "enhancing", "strengthening", "increasing", "triggering", "stimulating" and the like may be understood interchangeably in the broadest sense as the provision of an increased activity of an immune response.

In the context of the present invention, an increase of the immune response is preferably a local increase of the immune response, i.e., an increase of the immune response in proximity of the antigen recognized by the immune cells responsible for the respective immune response.

More specifically, the antigen-stimulated, TCR-ligated immune cells subjected to a compound of the present invention show a significantly increased secretion of several cytokines such as, e.g., IL-2, IFN-γ and/or TNF-α as well as increased proliferation and cytotoxicity shown by increased expression of cytotoxic factors, e.g. granzyme B, whereas corresponding un-stimulated immune cells do not. This leads to a local secretion of cytokines and effective antigen-specific cytotoxicity in the proximity of the neoplasm and/or infectious pathogen improving the local immune response to said neoplasm and/or infectious pathogen. More preferably, the compounds of the present invention provide a therapeutic or prophylactic intervention that increases the local effector efficiency of anti-tumor or anti-viral T cells, B cells and NK cells. The undesired significant increase of the systemic level of cytokines may however be widely avoided in the absence of tumor- or pathogen-associated antigens. When the antigen is exemplarily localized on the surface of neoplastic cells (e.g. cancer cells) and/or antigen presenting cells (e.g., maturated dendritic cells), the activity of immune cells, in particular T cells, contacted with such antigen may be increased. It has been found that upon administration of the compounds of the present invention, stimulated immune cells, in particular activated T cells contacted with their cognate antigen (e.g., a tumor and/or pathogen antigen), show increased local activation of the immune system in the tumor microenvironment and draining lymph nodes. Therefore, the enhanced immune response in the proximity to the neoplasm can be the driving force of increased physiological immune reactions supporting cytotoxicity against neoplasms. This further triggers antigen spreading and neo antigen presentation by APCs inducing a broader spectrum of T cell specific immunity. When the antigen is exemplarily localized on the surface of virus-infected cells (e.g., human papilloma (HPV)- or hepatitis C- infected cells), the activity of immune cells contacted with such antigen may be increased.

Notably, in contrast to vaccination-based strategies (e.g., tumor vaccination), for their activity, the compounds of the present invention do not necessarily require that the immune cells have been contacted with a specific tumor antigen, but stimulation of the cells can also be achieved by other means, e.g. stimulating the TCR / CD3 pathway and/or a costimulatory pathway such as CD28. In the context of the present invention, an increased immune response is preferably characterized by an increase in the secretion of at least one cytokine, more preferably by an increase in the secretion of at least one cytokine selected from the group consisting of IL-2, IFN-γ, TNF-α, IL-1 and IL-6, even more preferably in the secretion of at least one cytokine selected from the group consisting of IL-2, IFN-γ and TNF-α. Particularly preferably, an increased immune response is preferably characterized by an increase in the secretion of at least two cytokines such as, particularly preferred, IL-2 and IFN-γ, IL-2 and TNF-α, or IFN-γ and TNF-α. Also highly preferred is an increase in at least three cytokines such as of IL-2, IFN-γ and TNF-α. Additionally or alternatively, also other markers associated with immunologic activity may be increased in expression such as, e.g., CD40 ligand (CD40L, also known as CD154), granzyme/perforine, CD69, CD25 and/or CD71. Preferably such marker is CD40L.

As laid out above, enhanced IL-2 and IFN-gamma production by tumor infiltrating lymphocytes (TILs), in particular T cells specific for neoplastic and/or infectious antigens, is known to be linked to improved immunity against the neoplastic and/or infectious lesion(s). TNF-alpha has such effects as well. IL-2 may directly activate CD8 cells and natural killer (NK) cells. Therefore, its release may be beneficial at a neoplastic and/or infectious lesion, but also may have a general role for fostering T cell survival. Therefore, its release during antigen-presenting cell (APC) stimulation of T cells (e.g., in the lymph nodes) may also enhance an immune response.

Granzyme/perforine is considered as an effector molecule and consequently a marker for direct killing of neoplastic cells, in particular tumor cells, and may be released specifically proximal to or even in a neoplasm. Likewise, also IFN-gamma and TNF-alpha may activate immune cells (such as, e.g., NK cells and myeolid cells) but also directly upregulate apoptosis pathways in neoplastic cells.

IL-6 is a pleiotropic cytokine, which is particularly known to support B cell survival and its release in the lymph node, therefore, may also support B cell survival.

Release of IL-1 and IL-6 may enhance the development of T helper cells (e.g. Th17 cells), which are known to play a considerably role in immunity against neoplastic and infectious diseases. Therefore, the presence of enhanced levels of II-6 and IL-1 both at a neoplastic and/or infectious lesion and a lymph node may have beneficial effects on the immune response.

CD25, CD69, CD71 and CD40L are well-known surface markers for T cell activation and are known to demonstrate effects of the compounds on the level of individual T cell activation. CD69 is particularly an early marker of T cell activation. CD25 is the IL-2 receptor and high(er) expression typically supports (more) rapid expansion of activated T cells. CD71 is the receptor for transferrin and typically supports T cells to supply with Fe for proliferation. CD40L is a receptor on T helper cells which is known to support both APC and B cell activation and survival and proliferation.

In particular, an increase of IL-2, IFN-γ and/or TNF-α secretion, and/or CD40L expression is also exemplified in the Example section below. All these markers are well-known factors in anti-neoplastic immune response evidencing the anti-neoplastic (in particular, anti-tumor) activity of the compounds of the present invention.

Such increase of the secretion of cytokines by immune cells may be an increase of at least 10%, of at least 20%, of at least 30%, of at least 40%, of at least 50%, of at least 75%, of at least 2fold, of at least 3fold, of at least 4fold, or of at least 5fold compared to the secretion of the corresponding cytokine by immune cells subjected to the same stimulating agent and cultivated under comparable conditions but without being subjected to the compound of the present invention. The person skilled in the art will notice that the rate of an increase will typically also depend on the amount of the compound of the present invention subjected to the respective immune cells in a dose-dependent manner. Accordingly, it will, in many cases also depend on the dose of the compound of the present invention subjected to a patient in a dose-dependent manner. Within a suitable dose range, a higher dose will typically also lead to a higher increase. The person skilled in the art will further know that the dose-dependency also relates to the patient's body weight, the patient's fat and body water content, the patient's individual metabolism rate of deactivating and/or eliminating the compound, the patient's individual immunologic condition etc. Therefore, the person skilled in the art may adjust the dose accordingly.

Additionally or alternatively, also the proliferation rates of immune cells such as T cells, NK cells, B cells and/or monocytes may be increased. Exemplarily, the proliferation of CD4+ and/or CD8+ cells may be increased. Additionally or alternatively, also the maintenance (i.e, the survival rates, activity time or live time) of immune cells such as, e.g., T cells, NK cells, B cells and/or monocytes (e.g., CD4+ and/or CD8+ cells) may be increased.

Notably, the compounds of the present invention may also increase T cell reactivity to tumor antigens presented by MHC I to CD8 T cells or by MHC II to CD4 T cells independently of the tumor type and independently of the tumor antigens. Furthermore, the compounds of the present invention may increase the immunologic activity of the patient's NK cells to aid destruction of tumor cells that have decreased the MHC-I mediated display of tumor antigens. Moreover, strong antigen-specific T cell responses may be further increased by B cells and other immune cells, which may be also targeted by the compounds of the present invention. Thus, the compounds of the present invention may also abolish immunological ignorance towards neoplasia and/or infectious pathogens, so that the patient's specific tumor antigens are recognized as non-self and thus, the patient's own immune system may be re-activated to attack those tumor cells present in the patient, independently of the respective type of neoplastic and/or infectious disease.

In the view of the above, in a further aspect, the present invention relates to the compound, the pharmaceutically acceptable salt thereof, or pharmaceutical composition of the present invention for use in the treatment or prevention of a neoplastic and/or infectious disease.

Disclosed herein is a method of treating or preventing a neoplastic and/or infectious disease in a patient, comprising administering to said patient an amount of a compound or pharmaceutical composition of the present invention sufficient for treating or preventing said neoplastic and/or infectious disease in said patient.

In this aspect relating to such medical use and method of treatment or prevention, respectively, the definitions as laid out in detail above (in particular, in the context of the compound, the pharmaceutical composition and the use thereof as a medicament) also apply *mutatis mutandis.*

As used throughout the present invention, the term "patient" may be understood in the broadest sense as any subject or individual to be prevented or treated by means of a compound or pharmaceutical composition of the present invention, in particular having or being at risk of developing a neoplastic and/or infectious disease, irrespective whether clinical symptoms occur or do not occur. The patient may be any animal, including humans. Preferably, the patient is a mammal (e.g., a human, a mouse, a rat, a cow, a pig, a dog, a cat, a horse, a donkey, a goat, etc.), most preferably a human.

In the context of the present invention, the term "disease" may be understood in the broadest sense as any pathologic condition, irrespective whether clinical symptoms occur or do not occur. Therefore, the disease may be associated with a phenotype or may be latent. Preferably, a disease is a pathologic condition accompanied by one or more clinical symptom(s).

A disease in the context of the present invention may be a chronic and/or an acute disease. Preferably, it is a chronic disease. A chronic disease is persistent or otherwise long-lasting in its effects. In the context of the present invention, a chronic disease also includes a disease with a recurrent course, i.e., a recurrent disease relapsing repeatedly, with periods of remission in between. Accordingly, as used herein, a chronic disease may be understood in the broadest sense as any disease that lasts for at least a week, at least a month, at least three months, at least six month, at least a year or even several years (with or without clinical symptoms). When the patient is a human, a chronic disease is usually understood as lasting for at least one month or preferably at least three months. This understanding may also be applied to the present invention. In this context, it may be understood that, for instance, a neoplasm may typically but not necessarily grow for several months or even years until the first clinical symptoms occur. Nevertheless, the neoplastic disease already exists from the occasion of the first neoplastic cells, typically not associated with any clinical symptoms. Therefore, a recognized neoplastic disease is typically but not necessarily a chronic disease per se. Likewise, an infectious disease like a human immunodeficiency virus (HIV) infection is typically a chronic disease when it starts to provoke clinical symptoms and is first recognized.

As used herein, a neoplastic disease may be understood in the broadest sense as any tissue resulting from miss-controlled cell growth. In many cases a neoplasm leads to at least bulky tissue mass optionally innervated by blood vessels. It may or may not comprise the formation of one or more metastasis/metastases. A neoplastic disease of the present invention may be any neoplasm as classified by the International Statistical Classification of Diseases and Related Health Problems 10th Revision (ICD-10) classes C00-D48.

Exemplarily, a neoplastic disease according to the present invention may be the presence of one or more malignant neoplasm(s) (tumors) (ICD-10 classes C00-C97), may be the presence of one or more in situ neoplasm(s) (ICD-10 classes D00-D09), may be the presence of one or more benign neoplasm(s) (ICD-10 classes D10-D36), or may be the presence of one or more neoplasm(s) of uncertain or unknown behavior (ICD-10 classes D37-D48). Preferably, a neoplastic disease according to the present invention refers to the presence of one or more malignant neoplasm(s), i.e., is malignant neoplasia (ICD-10 classes C00-C97).

In a more preferred embodiment, the neoplastic disease is cancer.

Cancer may be understood in the broadest sense as any malignant neoplastic disease, i.e., the presence of one or more malignant neoplasm(s) in the patient. Cancer may be solid or hematologic malignancy. Preferably, the cancer is such accessible to at least one kind of immunotherapy (including, e.g., therapeutic antibodies targeted against tumor antigens and/or experimental approaches such as, e.g., cancer vaccination). Subtypes of cancer may be classified in different ways such as by the location in the body the main or only tumor bulk is found or by the tissue of origin the tumor(s) is/are derived from.

Exemplarily, such malignant neoplasm according to the present invention may be located on or in the lip, oral cavity and pharynx (ICD-10 classes C00-C14), on or in the digestive organs (ICD-10 classes C15-C26), on or in the respiratory system and intrathoracic organs (ICD-10 classes C30-C39), on or in the bone and articular cartilage (ICD-10 classes C40-C41), on or in the skin (ICD-10 classes C43-C44), on or in the connective and soft tissue (ICD-10 classes C45-C49), on or in the breast and female genital organs (ICD-10 classes C50-C58), on or in the male genital organs (ICD-10 classes C60-C63), on or in the urinary organs (ICD-10 classes C64-C68), on or in the eye, brain and central nervous system (ICD-10 classes C69-C72), on or in the endocrine glands and related structures (ICD-10 classes C73-C75), may be secondary and ill-defined neoplasms (ICD-10 classes C76-C80), may be stated or presumed to be primary, of lymphoid, haematopoetic and related tissue neoplasms (ICD-10 classes C81-C96), and/or may be neoplasms of independent (primary) multiple sites (ICD-10 class C97).

Exemplarily, cancers in the context of the present invention may be selected from the group consisting of carcinoma (i.e., cancers derived from epithelial cells; e.g., adenocarcinoma, squamous cell carcinoma, adenosquamous carcinoma, anaplastic carcinoma, large cell carcinoma, and small cell carcinoma), sarcoma (i.e, cancers derived from connective tissue; e.g., Askin's tumor, sarcoma botryoides, chondrosarcoma, Ewing's sarcoma, malignant hemangioendothelioma, malignant Schwannoma, osteosarcoma, and soft tissue sarcomas), hematologic cancer such as lymphoma, a leukemia or a myeloma. A hematologic cancer contemplated herein includes, but is not limited to lymphoma and leukemia (i.e., cancers derived from hematopoietic (blood-forming) cells; e.g., mature B-cell neoplasms, mature T cell and natural killer (NK) cell neoplasms, Hodgkin lymphoma, immunodeficiency-associated lymphoproliferative disorders, lymphocytic leukemia, myelogenous leukemia), germ cell tumor (i.e., cancers derived from pluripotent cells in the sexual organs; e.g., germinoma (including dysgerminoma and seminoma), dysgerminoma, seminoma), blastoma (i.e., cancers derived from immature "precursor" cells or embryonic tissue; e.g., hepatoblastoma, medulloblastoma, nephroblastoma, neuroblastoma, pancreatoblastoma, pleuropulmonary blastoma, retinoblastoma, glioblastoma), and melanoma and preforms thereof (i.e., cancers derived from melanocytes; e.g., Lentigo maligna , superficial spreading melanoma, acral lentiginous melanoma, mucosal melanoma, nodular melanoma, polypoid melanoma, desmoplastic melanoma, amelanotic melanoma, soft-tissue melanoma), and nonmelanoma skin cancer (i.e., nonmelanoma cancers derived from skin, e.g. basal cell carcinoma, squamous cell carcinoma, dermatofibrosarcoma protuberans, Merkel cell carcinoma, Kaposi's sarcoma, keratoacanthoma, spindle cell tumors, sebaceous carcinomas, microcystic adnexal carcinoma, Paget's disease of the breast, atypical fibroxanthoma, leiomyosarcoma, angiosarcoma) and glioma (i.e., cancers derived from brain or spine cells., e.g., ependymoma, astrocytoma, oligodendrogliomas, brainstem glioma, optic nerve glioma, mixed glioma).

In some embodiments of the invention herein, the cancer is a non-hematologic cancer such as a sarcoma, a carcinoma, or a melanoma. Preferably, non-hematologic cancer may be the formation of one or more solid tumor(s) such as, e.g., those selected from the group consisting of melanoma, neuroblastoma, lung cancer, non-small-cell lung cancer, small cell lung cancer, renal cell carcinoma, epithelial squamous cell cancer, in addition breast cancer, ovarian cancer, cervical cancer, uterine cancer, prostate cancer, testicular cancer, colon cancer, colorectal cancer, hepato-cellular carcinoma, bladder cancer, stomach cancer, esophageal cancer, pancreatic cancer, gastric cancer, liver cancer, mesothelioma, thyroid cancer, adrenal cancer, brain cancer, and head-and-neck cancer.

More preferably, the compounds of the invention, or the pharmaceutical composition of the present invention is useful for the prophylaxis and/or the treatment of the cancer, wherein the cancer is interstitial fibrosis, prostate cancer, colon cancer, melanomas, lung cancer, rectal cancer, breast cancer, multiple myeloma, gastrointestinal cancer, non-small cell lung cancer (NSCLC).

Alternatively, cancer may be the formation of one or more hematopoietic tumor(s) such as, e.g., those selected from the group consisting of multiple myeloma, Non-Hodgkin lymphoma, AML (Acute Myeloid leukemia, DLBCL (Diffuse Large B-cell Lymphoma), and B-CLL (B-cell chronic lymphocytic lymphoma).

In an alternative preferred embodiment, the disease may be an infectious disease.
As used in the context of the present invention, the term "infectious disease" may be understood in the broadest sense as any pathologic condition caused by the invasion of a patient's body by one or more biological agent(s) foreign to the body able to provoke an immune reaction in the patient's body. An infectious disease may or may not be accompanied by an inflammatory response (inflammation). Preferably, an infectious disease in the context of the present invention is accompanied by an inflammatory response. Exemplarily, such immune reaction in the patient's body may be in more detail caused by a biological agent itself (e.g., by the presence of surface antigens thereof), by antigens originating from a biological agent provided on a major histocompatibility complex I or II (MHC I or MHC II), by the multiplication of a biological agent, by the reaction of host tissues to such biological agent, by compounds produced or caused by such biological agent (e.g., toxins, semiochemicals, cytokines, etc.), or by the formation of an antigen from a haptene originating from such biological agent. Such biological agents may be non-living or living agents. Exemplarily, an infectious disease may be caused by biological agents selected from the group consisting of viruses, viroids, prions, microorganisms such as bacteria, nematodes such as roundworms and pinworms, arthropods (e.g., ticks, mites, fleas, and lice), fungi, ringworms, and tapeworms. Preferably, an infectious disease according to the present invention is caused by viruses or bacteria, in particular is a virus infection.

A viral infection in the context of the present invention may be an infection by any virus. The viral infection may be an acute viral infection or a chronic viral infection. Preferably, it is a chronic viral infection. Nonrestrictive examples of clinically important virus families and species in the context of the present invention include Adenovirus, Herpes simplex, type 1, Herpes simplex, type 2, Varicellazoster virus, Epstein-barr virus, Human cytomegalovirus, Human herpesvirus, type 8, Human papillomavirus, BK virus, JC virus, Smallpox, Hepatitis B virus, Human bocavirus, Parvovirus B19, Human astrovirus, Norwalk virus, coxsackievirus, hepatitis A virus, poliovirus, rhinovirus, Severe acute respiratory syndrome virus, Hepatitis C virus, yellow fever virus, dengue virus, West Nile virus, Rubella virus, Hepatitis E virus, Human immunodeficiency virus, Influenza virus, Guanarito virus, Junin virus, Lassa virus, Machupo virus, Sabiá virus, Crimean-Congo hemorrhagic fever virus, Ebola virus, Marburg virus, Measles virus, Mumps virus, Parainfluenza virus, Respiratory syncytial virus, Human metapneumovirus, Hendra virus, Nipah virus, Rabies virus, Hepatitis D, Rotavirus, Orbivirus, Coltivirus, and Banna virus.

In the context of the present invention, those virus infections associated with a down-regulation of the immune response are of particular interest, such as, e.g., a Human immunodeficiency virus (HIV) infection, without symptoms or associated with symptoms (Acquired Immune Deficiency Syndrome (AIDS)).

Further, also such virus infections associated with neoplasia such as, e.g., Herpes simplex virus (HSV), type 1 or 2, are also of particular interest. Here, the compounds or pharmaceutical composition of the present invention may concomitantly be pharmaceutically active against the virus infection as well as the neoplasm resulting from said virus infection.

Further, also such virus infections being latent for a longer time and, thus, hiding from the immune system, such as, e.g., HSV 1 or HSV2, are also of particular interest. Optionally but not necessarily, an infectious disease, in particular a chronic infectious disease (e.g., a chronic virus infection), may be associated with inflammation. In this context, inflammation may be characterized by an increase in the NF-κB activity, C-reactive protein (CRP) level, interferon-gamma (IFN-gamma) level, interleukin 1 (IL-1) level and/or interleukin 8 (IL-8) level.

In the context of a treatment or prevention of a neoplastic and/or infectious disease, the compound(s) of the present invention may be administered as the sole pharmaceutically active agent or may be administered in combination with one or more other pharmaceutically active agent(s). Exemplarily, such other pharmaceutically active agent may be a stimulating agent activating immune cells, may be an anti-proliferative agent (e.g., an anti-cancer agent such as a chemotherapeutic, an antimetabolite, a hormone, an antibody (Ab)), an antiviral agent, and/or an antibiotic.

Preferably, such other pharmaceutically active agent is a biological compound such as a therapeutic monoclonal antibody which has been shown to be efficacious in treating neoplasms. Exemplarily, such therapeutic monoclonal antibody is directed against the PD-1 molecule, the PD-L1 molecule or another ligand of the PD-1 molecule, the CTLA-4 molecule, the TIM3 molecule, the LAG3 molecule, the VISTA molecule or the BTLA-4 molecule.

The application of such combination therapy will depend on the pharmacokinetic and pharmacodynamics properties of the chosen compounds and agents used in such combination therapy (adjunction).

Optionally, the further agent may be administered concomitantly, previously, or subsequently with one or more compound(s) of the present invention. As used herein, a concomitant administration may be an administration in a single composition (e.g., combined in the pharmaceutical composition of the present invention) or in two separate compositions that may also, optionally, be administered via the same or different routes of administration (e.g., via injection, orally, nasally, percutaneously, etc.). As used herein, when administering the compound of the present invention previously or subsequently, there may be a time interval between the administration of said compound(s) and the further agent(s) of less than one hour, one hour or more, three hours or more, six hours or more, twelve hours or more, 24 hours or more, two days or more or a week or more.

As mentioned in the context of the compound of the present invention above, also the one or more further agent(s) may be administered once (single administration) or may be a repeated administration such as, e.g., two times, three times, four times, five times, six times, seven times, eight times, nine times, ten times, more than ten times or even permanently. Between two administrations, there may be a time interval of less than one hour, one hour or more, six hours or more, twelve hours or more, 24 hours or more. Administration may be daily, may be twice daily, three times daily, four times daily, every second day, every three days, weekly, biweekly, monthly, twice a year or yearly. In a preferred embodiment, the compound or pharmaceutical composition of the present invention is administered in combination with one or more further stimulating agent(s) activating immune cells.

Immune cells as used in the context of the present invention may be any immune cells known in the art. Immune cells may be cells of the adaptive immune system (e.g., T cells or B cells, cells of the innate immune system (natural killer (NK) cells, macrophages, monocytes), and/or cells subsumable under both groups (e.g., dendritic cells (DCs); antigen-presenting cells (APCs))) and/or cells known for phagocytic activity such as basophilic, neutrophilic or eosinophilic granulocytes.

In a preferred embodiment, the immune cells are peripheral blood mononuclear cells (PBMCs).

In a preferred embodiment, the immune cells are selected from those bearing T cell antigen receptor (TCR) and a CD4 and/or CD8 co-receptor on their cellular surface. Those immune cells bearing a CD4 co-receptor on their cellular surface may be selected from the group consisting of T helper cells (Th cells), macrophages, and dendritic cells (DCs). Those immune cells bearing a CD8 co-receptor on their cellular surface may be selected from the group consisting of cytotoxic T cells, natural killer (NK) cells, cortical thymocytes, and dendritic cells (DCs).

In another preferred embodiment, the immune cells are selected from the group consisting of T cells, NK cells, monocytes and B cells.

In a particularly preferred embodiment, the immune cells are T cells and/or NK cells.
T cells and NK cells are well-known to bear particular efficiency against neoplasia (particularly anti-tumor immunity) and infectious diseases. NK cells are preferably lymphocytes which can be stimulated via the Fc receptor (FcR). In particular in the context of antibody dependent cellular cytotoxicity (ADCC), this may be of some benefit in the context of the present invention (e.g., as single application or when co-administering a therapeutic antibody against neoplastic and/or infectious antigens).
B cells are well-known to play a considerable role in humoral response to infectious pathogens. It has been further known in the art that also B cells may also play a role in developing humoral immunity against neoplastic cells.

Monocytes may be understood as the transient phenotype of myeloid cells present in large numbers in PBMCs. Moreover, myeloid cells include dendritic cells (DCs). Macrophages may bear both positive and suppressive effects on immunity, in particular anti-neoplastic therapies. Macrophages of particular interest include M1-type macrophages. Myeloid cells may also include myeloid-derived suppressor cells (MDSCs) bearing a negative effect in anti-neoplastic immune response. Myeloid cells can be stimulated via the FC receptor (FcR). In particular in the context of antibody dependent cellular cytotoxicity (ADCC), this may be of some benefit in the context of the present invention (e.g., as single application or when co-administering a therapeutic antibody against neoplastic and/or infectious antigens). It may also be enhancing the effect of naturally developed humoral immunity against tumor antigens in the patient. An activation of immune cells may be understood in the broadest sense as the increase of immunologic activity of such cell and/or the increase of cell proliferation of such cells. In a more preferred embodiment, the further stimulating agent(s) activating immune cells is/are selected from the group consisting of one or more antigen(s) of the neoplasm and/or infectious pathogen to be treated, one or more TCR or CD3 agonist(s), one or more CD28 agonist(s), one or more agonist(s) to other costimulatory T cell surface receptors such as CD40L, CD69, OX40, GITR, CD137, CD27 and/or HVEM, and a combination of two or more thereof.

A TCR / CD3 agonist may be any agent triggering CD3. It may be a peptide or non-peptide agonist binding to the extracellular side of TCR / CD3 in the context or absence of MHC-dependent antigen presentation, may be an agonist binding to the intracellular side of TCR / CD3, or may be an agent activating the intracellular signal transduction pathway triggered by TCR / CD3 engagement. Preferably, a CD3 agonist may be an anti-CD3 antibody, a peptide antigen presented by MHC I or MHC II, an anti-CD3 antibody fragment or an anti-CD3 antibody mimic. Highly preferably, a CD3 agonist is a tumor antigen presented by MHC I or MHC II.

A CD28 agonist may be any agent triggering CD28. It may be an agonist binding to the extracellular side of CD28, may be an agonist binding to the intracellular side of CD28, or may be an agent activating the intracellular signal transduction pathway triggered by CD28. Preferably, a CD28 agonist may be an anti-CD28 antibody, an anti-CD28 antibody fragment, an anti-CD28 antibody mimetic or a protein containing a natural ligand for CD28 such as B7.1 or B7.2. Highly preferably, a CD28 agonist is an (agonistic) anti-CD28 antibody or an Ig fusion protein containing a natural ligand for CD28 such as B7.1 or B7.2.

An antibody in the context of the present invention may be a monoclonal or a polyclonal antibody of any species or origin. It may bind to any epitope(s) comprised in the polypeptide bearing the respective cognate antigen (e.g, CD3 or CD28, respectively) including its posttranslational modifications. The cognate antigen may exemplarily be a linear epitope, a structural epitope, a primary epitope, and/or a secondary epitope. An antibody may be of natural origin, of gene technologic origin and/or of synthetic origin. An antibody fragment may be understood in the broadest sense as any fragment of an antibody that still bears binding affinity to its target polypeptide. Exemplarily, the antibody fragment may be a fragment antigen binding (Fab fragment), a truncated antibody comprising one or both complementarity determining region(s) (CDR(s)) or the variable fragment (Fv) of an antibody. The antibody fragments may be of natural origin, of gene technologic origin and/or of synthetic origin.

An antibody mimetic may be understood in the broadest sense as organic compounds that, like antibodies, can specifically bind antigens and that typically have a molecular mass in a range of from approximately 3 kDa to approximately 25 kDa. Antibody mimetics may be, e.g., Affibody molecules (Affibodies), Affilins, Affitins, Anticalins, Avimers, DARPins, Fynomers, Kunitz domain peptides, single-domain antibodies (e.g., VHH antibodies or VNAR antibodies) Monobodies, Diabodies, Triabodies, flexibodies and tandabs. The antibody mimetics may be of natural origin, of gene technologic origin and/or of synthetical origin.

Peptide antigens may be understood in the broadest sense as organic compounds that specifically bind to MHC I or MHC II molecules and that typically consist of 8-30 amino acids and preferably consist of 9-25 amino acids. The peptides may be of natural origin, of gene technologic origin and/or of synthetical origin.

Preferably, the further stimulating agents activating immune cells are a combination of one or more CD3 agonist(s) and one or more CD28 agonist(s). Particularly preferably, the further stimulating agents activating immune cells are a combination of at least one (agonistic) anti-CD3 antibody and at least one (agonistic) anti-CD28 antibody. As it is evident from the Examples shown below, a stimulation of the immune cells by means of contacting these with (agonistic) anti-CD3 antibodies and/or (agonistic) anti-CD28 antibodies mechanistically simulates T cells, irrespective of the individual TCR-recognized specific antigen. A stimulation with (agonistic) anti-CD3 antibodies and (agonistic) anti-CD28 antibodies very well mimics activation of T cells in a patient's body in vivo.

Additionally or alternatively, the immune cells may also be triggered by an antigen of the neoplasm and/or infectious pathogen (e.g., by means of vaccinating the patient with one or more antigen(s)). Then, the antigen is considered as a stimulating agent. An antigen of the neoplasm and/or infectious pathogen may be, exemplarily, a vaccine comprising one or more antigen(s) of the neoplasm and/or infectious pathogen, such as e.g, a polypeptide-based vaccine, a polynucleotide vaccine, an oligosaccharide vaccine, or a vaccine based on fragments of neoplasms of the same type or on fragments of infectious pathogens of the same type. The person skilled in the art knows numerous methods for providing such vaccines. Several anti-tumor and antiviral vaccines are also commercially available.

Additionally or alternatively, the immune cells may also be triggered by antigen-loaded antigen-presenting cells (APCs). Then, the antigen-loaded APCs are considered as a further stimulating agent. In this context, the antigens are also antigens of the neoplasm and/or infectious pathogen as mentioned before.

Additionally or alternatively, the one or more further stimulating agent(s) may be selected from the group consisting of checkpoint blockade therapeutics (in particular T cell surface receptor-binding agents such as, e.g., those binding one or more selected from the group consisting of CTLA4, PD-1, PDL-1, TIM3, LAG3, BTLA, VISTA and/or a ligand thereof (e.g., anti-CTLA4, anti-PD-1 and/or anti-PDL-1 antibodies)), cytokines (e.g., IL-2, IL-15 and/or IL-7), activating agents of APCs (e.g., CD40 agonists), adoptive cellular agents (in particular adoptive T cells (e.g., chimeric immune receptor T cell therapy such as, e.g., CAR T cell therapy), dendritic cell therapy (e.g., sipuleucel-T) and/or natural killer cell therapies), enhancers of T cell functions (e.g., lenalidomide and related agents), enhancers of natural killer cell functions (e.g., anti-KIR antibodies), and therapeutic antibodies directed against tumor antigens.

Optionally, in particular when the patient is suffering from a neoplastic disease, the patient may be further administered with one or more chemotherapeutic(s), cytokine(s) and/or other anti-neoplastic agent(s) in addition to one or more compound(s) of the present invention. Exemplarily, such chemotherapeutics, cytokines and anti-cancer agents may be selected from the group consisting of polyclonal or monoclonal antibodies (e.g., rituximab, trastuzumab, cetuximab, bevacizumab, basiliximab, daclizumab), anti-metabolites (e.g., 5-fluorouracil, azathioprine, 6-mercaptopurine, mercaptopurine, pyrimidines, thioguanine, fludarabine, floxuridine, cytosine arabinoside (cytarabine), pemetrexed, raltitrexed, pralatrexate, methotrexate), alkylating agents (e.g., mechlorethamine, cyclophosphamide, chlorambucil, Ifosfamide), platins (e.g., cisplatin, carboplatin, oxaliplatin), plant alkaloids and terpenoids (e.g., vinca alkaloids (vincristine, vinblastine, vinorelbine, vindesine), taxanes (e.g., paclitaxel), cytoxan), topoisomerase inhibitors (e.g., camptothecins: irinotecan, topotecan, etoposide, etoposide phosphate, teniposide), melphalan, antineoplastica (e.g., doxorubicin (adriamycin), doxorubicin lipo, epirubicin, bleomycin)), actinomycin D, aminoglutethimide, amsacrine, anastrozole, antagonists of purine and pyrimidine bases, anthracyclines, aromatase inhibitors, asparaginase, antiestrogens, bexarotene, buserelin, busulfan, camptothecin derivatives, capecitabine, carmustine, cladribine, cytarabine, cytosine arabinoside, alkylating cytostatics, dacarbazine, daunorubicin, docetaxel, epirubicin, estramustine, etoposide, exemestane, fludarabine, fluorouracil, folic acid antagonists, formestane, gemcitabine, glucocorticoids, goserelin, hormones and hormone antagonists, hycamtin, hydroxyurea, idarubicin, irinotecan, letrozole, leuprorelin, lomustine, mercaptopurine, miltefosine, mitomycins, mitosis inhibitors, mitoxantrone, nimustine, procarbazine, tamoxifen, temozolomide, teniposide, testolactone, thiotepa, topoisomerase inhibitors, treosulfan, tretinoin, triptorelin, trofosfamide, cytostatically active antibiotics, everolimus, pimecrolimus, tacrolimus, azithromycin, spiramycin, sirolimus (rapamycin), roxithromycin, ascomycin, bafilomycin, erythromycin, midecamycin, josamycin, concancamycin, clarithromycin, troleandomycin, folimycin, tobramycin, mutamycin, dactinomycin, dactinomycin, rebeccamycin, a statin (e.g., cerivastatin, simvastatin, lovastatin, somatostatin, fluvastatin, nystatin, rosuvastatin, atorvastatin, pravastatin, pitavastatin, pentostatin,), 4-hydroxyoxycyclophosphamide, bendamustine, thymosin α-1, aclarubicin, fludarabine-5'-dihydrogen phosphate, hydroxycarbamide, aldesleukin, pegaspargase, cepharanthine, epothilone A and B, azathioprine, mycophenolate mofetil, c-myc antisense, b-myc antisense, betulinic acid, camptothecin, melanocyte stimulating hormone (α-MSH), activated protein C, IL-1β inhibitor, fumaric acid and esters thereof, dermicidin, calcipotriol, taclacitol, lapachol, β-lapachone, podophyllotoxin, betulin, podophyllic acid 2-ethyl hydrazide, sagramostim, (rhuGM-CSF), peginterferon α-2b, lenograstim (r-HuG-CSF), filgrastim, macrogol, cephalomannine, selectin (cytokine antagonist), CETP inhibitor, cadherins, cytokinin inhibitors, COX inhibitor (COX-2 or COX-3 inhibitor), angiopeptin, ciprofloxacin, fluroblastin, bFGF antagonists, probucol, prostaglandins, 1,11-dimethoxyeanthin-6-one, 1-hydroxy-11-methoxycanthin-6-one, scopoletin, colchicine, NO donors, pentaerythrityl tetranitrate, sydnonimines, S-nitroso derivatives, staurosporine, β-estradiol, α-estradiol, estriol, estrone, ethinyl estradiol, fosfestrol, medroxyprogesterone, estradiol cypionates, estradiot benzoates, tranilast, kamebakaurin, verapamil, ciclosporin A, paclitaxel and derivatives thereof such as 6-α-hydroxy paclitaxel, baccatin, taxotere, mofebutazone, acemetacin, diclofenac, lonazolac, dapsone, o-carbamoyl-phenoxy-acetic acid, lidocaine, ketoprofen, mefenamic acid, piroxicam, meloxicam, chloroquine phosphate, penicillamine, hydroxychloroquine, auranofin, sodium aurothiomalate, oxaceprol, celecoxib, β-sitosterol, ademetionine, myrtecaine, polidocanol, nonivamide, levomenthol, benzocaine, aescin, elipticine, Calbiochem D-24851, colcemid, cytochalasin A-E, indanocine, nocodazole, bacitracin, vitronectin receptor antagonists, azelastine, free nucleic acids, nucleic acids incorporated into virus transmitters, DNA and RNA fragments, plasminogen activator inhibitor-1, plasminogen activator inhibitor-2, antisense oligonucleotide, VEGF inhibitors, IGF-1, active agents from the group of antibiotics such as cefadroxil, cefazolin, cefaclor, cefoxitin, gentamicin, penicillins, dicloxacillin, oxacillin, sulfonamides, metronidazole, antithrombotics, argatroban, aspirin, abciximab, synthetic antithrombin, bivalirudin, coumadin, enoxaparin, GpIIb/IIIa platelet membrane receptor, antibodies to factor Xa inhibitor, heparin, hirudin, r-hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin,. vapiprost, interferon α, β and γ, histamine antagonists, serotonin blockers, apoptosis inhibitors, apoptosis regulators, NF-kB or Bcl-xL antisense oligonucleotides, halofuginone, nifedipine, tocopherol, molsidomine, tea polyphenols, epicatechin gallate, epigallocatechin gallate, boswellic acids and derivatives thereof, leflunomide, anakinra, etanercept, sulfasalazine, tetracycline, triamcinolone, procainimide, retinoic acid, quinidine, disopyramide, flecainide, propafenone, sotalol, amiodarone, natural and synthetically obtained steroids such as bryophyllin A, inotodiol, maquiroside A, mansonine, strebloside, hydrocortisone, betamethasone, dexamethasone, fenoprofen, ibuprofen, indomethacin, naproxen, phenylbutazone, acyclovir, ganciclovir, zidovudine, antimycotics, clotrimazole, flucytosine, griseofulvin, ketoconazole, miconazole, terbinafine, chloroquine, mefloquine, quinine, natural terpenoids, hippocaesculin, barringtogenol-C21-angelate 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceanol A, B and C, bruceantinoside C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrine, taxamairin A and B, regenilol, triptolide, cymarin, apocymarin, aristolochic acid, anopterin, hydroxyanopterin, anemonin, protoanemonin, berberine, cheliburin chloride, cicutoxin, sinococuline, combrestatin A and B, cudraisoflavone A, curcumin, dihydronitidine, nitidine chloride, 12-beta-hydroxypregnadiene-3,20-dione bilobol, ginkgol, ginkgolic acid, helenalin, indicine, indicine-N-oxide, lasiocarpine, inotodiol, glycoside 1a, justicidin A and B, larreatin, malloterin, mallotochromanol, isobutyrylmallotochromanol, marchantin A, maytansine, lycoridicin, margetine, pancratistatin, liriodenine, bisparthenolidine, oxoushinsunine, aristolactam-All, periplocoside A, ghalakinoside, deoxypsorospermin, psychorubin, ricin A, sanguinarine, manwu wheat acid, methylsorbifolin, chromones of spathelia, stizophyllin, akagerine, dihydrousambaraensine, hydroxyusambarine, strychnopentamine, strychnophylline, usambarine, usambarensine, daphnoretin, lariciresinol, methoxylariciresinol, syringaresinol, umbelliferone, afromoson, acetylvismione B, desacetylvismione A, vismione A and B), radiation therapy (e.g, Intensity-Modulated Radiation Therapy (IMRT), 3-Dimensional Conformal Radiotherapy (3DCRT), Stereotactic body radiation therapy (SBRT), Stereotactic radiosurgery (SRS), image-guided radiation therapy (IGRT), Particle Therapy (e.g, proton therapy), Brachytherapy, Radioisotope Therapy (RIT) (e.g., with iodine-131, lutetium-177, strontium-89 and samarium (153Sm) lexidronam and/or yttrium-90)), antiangiogenic therapy (e.g., carboxyamidotriazole, TNP-470, CM101, , Suramin, SU5416, Thrombospondin, VEGFR antagonists, angiostatic steroids + heparin, Cartilage-Derived Angiogenesis Inhibitory Factor, matrix metalloproteinase inhibitors, 2-methoxyestradiol, Tecogalan, tetrathiomolybdate, thalidomide, thrombospondin, soluble VEGFR-1 and NRP-1, Angiopoietin 2, angiostatin (e.g., TSP-1 and TSP-2 angiostatin), endostatin, vasostatin, canstatin, calreticulin, platelet factor-4, TIMP and CDAI, Meth-1 and Meth-2, CXCL10prothrombin (kringle domain-2), antithrombin III fragment prolactin, VEGI, SPARC, osteopontin, maspin, proliferin-related protein, restin), kinase inhibitors (e.g., imatinib, imatinib mesylate, gefitinib, erlotinib, pazopanib, apatinib), proteasome inhibitors (e.g., bortezomib), PARP inhibitors (e.g., iniparib, olaparib), and combinations of two or more thereof.

Alternatively or additionally, the patient is suffering from or being at risk of developing a neoplastic and/or infectious disease, may be further administered with one or more cytokines, hormones or analogues thereof (e.g., selective estrogen receptor modulator tamoxifen, IL-2, IFN-α, IFN-β, IFN-γ, IL-4, IL-12, IL-18, platelet factor-4, TNF-α). These cytokines, hormones or analogues thereof may further trigger the patient's immune system. As mentioned before, high doses of many of such agents may provoke severe side effects. However, lower doses may optionally be used to support the treatment or prevention of the present invention.

Optionally, in particular when the patient is suffering from a viral infection, the patient may be further administered with one or more antiviral compound(s) in addition to one or more compound(s) of the present invention. Such antiviral compound may exemplarily be selected from the group consisting of an entry or fusion inhibitor, a nucleoside/nucleotide reverse transcriptase inhibitor, a non-nucleoside reverse transcriptase inhibitor, an integrase inhibitor, and a protease inhibitor.

Optionally, in particular when the patient is suffering from a bacterial infection, the patient may be further administered with one or more antibacterial antibiotic(s) in addition to one or more compound(s) of the present invention. Such antibacterial antibiotic may exemplarily be selected from the group consisting of antibiotics targeting the bacterial cell wall (e.g., penicillins and cephalosporins) or the cell membrane (e.g., polymyxins), interfering with essential bacterial enzymes (e.g., rifamycins, lipiarmycins, quinolones, and sulfonamides), and/or targeting polypeptide synthesis (e.g., macrolides, lincosamides and tetracyclines).

Further, a treatment or prevention according to the present invention may also be combined with other means of treatment such as, e.g., radiation therapy (exemplarily based on x-ray radiation, ultraviolet (UV) radiation (e.g., UV-A, UV-B, and/or UV-C radiation), alpha radiation, beta radiation, gamma radiation, or cosmic radiation), and/or surgery.

As laid out above, it will be understood that the compounds of the present invention may be very well used for the treatment and/or prevention of neoplastic and/or infectious diseases in a patient in vivo. However, a compound of the present invention may not merely be used for in vivo application, but likewise also for any kind of ex vivo and/or in vitro use. Exemplarily, it may also be used for activating immune cells in vivo, ex vivo and in vitro.

Exemplarily, the compounds of the present invention may be used in any method supporting the generation and/or amplification of immune cells ex vivo and/or in vitro, in particular but not necessarily for further use in an adoptive cell therapy (ACT). For ACT preferably antigen-specific T cells may be used. Such methods may also provide activated DCs which may optionally be useful for DC vaccination approaches.

In another aspect, the present invention refers to an *in vitro* or ex *vivo* method for the production of activated immune cells comprising the steps of:
(i) providing immune cells;
(ii) contacting the cells of step (i) with:
   (a) at least one compound or pharmaceutically acceptable salt thereof as defined in any of claims 1 to 9, and optionally
   (b) one or more further stimulating agents activating said immune cells; and
(iii) cultivating the cells of step (ii) under conditions suitable for maintaining the viability of said cells.

In this aspect relating to such method, the definitions as laid out in detail above also apply mutatis mutandis.

The method is conducted ex *vivo* and/or *in vitro,* i.e., is an ex vivo and/or in vitro method. Therefore, in the context of this aspect relating to such method, the immune cells are preferably activated outside of a living being, in particular outside a patient.

Preferably, the immune cells (e.g., T cells and/or natural killer cells) are mature immune cells. Such cells (in particular the T cells) may be CD4+ and/or CD8+ cells. The immune cells may be obtained from any source suitable for this purpose. Alternatively or additionally, the cells may also be B cells such as, e.g., CD19+ B cells. The person skilled in the art knows various ways of obtaining such immune cells. Exemplarily, mature immune cells may be obtained from a blood sample (e.g., a stored blood preservation or fresh blood). Then, peripheral blood mononuclear cells (PBMCs) may exemplarily be obtained from the buffy coat after centrifugation of a blood sample and optionally further isolated/purified, exemplarily, by means of labeling cell type-specific surface markers with fluorescence-labeled antibodies followed by fluorescence activated cell sorting (flow cytometry) or by labeling cell type-specific surface markers with metal bead-labelled antibodies followed by magnetic extraction of the desired cells.

Alternatively, mature immune cells may also be obtained from cell culture. The ways of obtaining a buffy coat and isolating and purifying the cells further is exemplified in the example section below. (Mature) immune cells are also commercially available. Alternatively, immature immune cells or precursors thereof may be used and matured in an intermediate step by well-known means of supplementation with the respective cytokines and growth factors.

The immune cells are subsequently contacted with at least one compound of the present invention and optionally one or more further stimulating agent(s) activating immune cells. The person skilled in the art will immediately notice that these compound(s) and agent(s) may be added to the cells in any kind of solution or medium suitable for the cells. Exemplarily, such solution or medium may also comprise ingredients defined in the context of a pharmaceutical composition above. A further stimulating agent activating immune killer cells may be understood in the broadest sense as defined above. Optionally, the further agent may be administered concomitantly, previously or subsequently with one or more compound(s) of the present invention. The cells may be contacted with the compound(s) and/or agent(s) for less than 30 min, at least 30 min, at least 1 h, for at least 2 h, for at least 5 h, for at least 12 h, for at least 1 day or longer.

Subsequent to or concomitant with contacting the cells with the compound(s) and optional agent(s) (step (ii)), the cells are cultivated under conditions suitable for maintaining the viability of said cells (step (iii)). Therefore, steps (ii) and (iii) may be conducted as one step (simultaneously) or two separate steps (subsequently) or with a partly temporal overlap. Typically, the cells are cultivated in a suitable cell culture medium (e.g., X-Vivo 15) optimized to allow cell culture in the absence of FCS RPMI 1640) optionally supplemented with fetal calf serum (FCS) at 5% CO 2 and a temperature of 30°C-39°C, preferably (approximately) 37°C. Preferably, the cells are cultivated for at least 1 h, for at least 2 h, for at least 5 h, for at least 12 h, for at least 1 day or for at least 3 days.

As a result from the method of the present invention, activated immune cells (e.g., T cells and/or natural killer cells) may be obtained.

These activated immune cells may be optionally isolated by any means known in the art (optional step (iv)). Optionally, as a further step (v), the activated immune cells may subsequently be administered to a patient in need thereof. Alternatively, the isolated activated immune cells obtained from step (iv) or the cells of step (iii) may also be stored and/or preserved (e.g., dispersed in a DMSO-containing medium and stored at - 80°C). Alternatively, the isolated activated immune cells of step (iv) or the cells of step (iii) may be used for any other in vitro and/or in vivo purposes. Exemplarily, such activated immune cells may be used for research purposes intended to further investigate activated immune cells (in particular activated T cells and/or natural killer cells).

Exemplarily, the activated immune cells (in particular activated T cells and/or natural killer cells) may be used for the production of cytokines secreted by the cells. Then, a further step (iv) is the cultivating of the cells until the level(s) of the desired cytokine(s) secreted into the medium reach(es) the desired level, followed by step (v) of isolating and, optionally purifying the desired cytokine(s). The isolation and optional purification of cytokines may be performed by any means known in the art such as, e.g., chromatographic means. Optionally, such cytokine(s) may subsequently be stored and/or preserved (e.g., frozen, dried or freeze-dried).

Furthermore, a compound of the present invention may be further used as a research tool for investigating immune cell activation in more detail.

In a further aspect, the present invention refers to the compound for use, or the pharmaceutically acceptable salt thereof for use, or the pharmaceutical composition thereof for use as a medicament in the prophylaxis or treatment of a neoplastic and/or infectious disease, a heart disease, wherein the heart disease is myocardial infraction, acute coronary syndrome, myocardial ischemia, ischemic cardiomyopathy, myocardial reperfusion injury, non-ischemic cardiomyopathy, or acute or chronic heart failure.

### Description of Figures

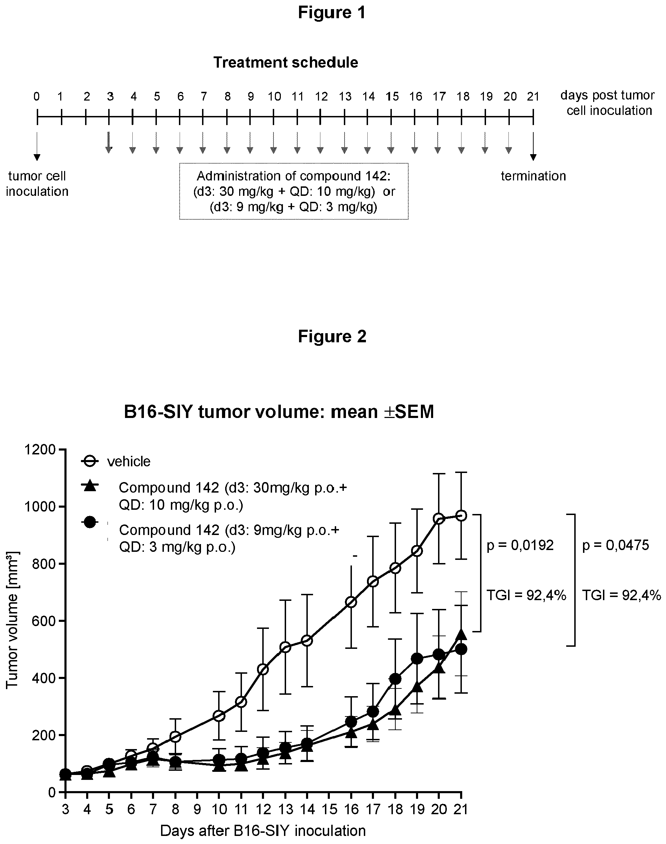

**Fig 1****. Treatment schedules for compound 142 *in vivo* efficacy experiments with p.o. compound administration.** Efficacy experiment #3 treatment schedule for POC study monitoring tumor volume and survival rate of mice treated p.o. Blue arrows indicate p.o. compound **142** administration.
**Fig 2****. Compound 142 impact on *in vivo* tumor growth rate.** Average tumor volumes ± SEM of mice receiving p.o. treatment with compound **142** d3: 30 mg/kg + QD: 10 mg/kg or d3: 9 mg/kg + QD: 3 mg/kg. Statistically significant difference (p < 0.05) between treatment groups and vehicle control group was calculated using 2-way ANOVA analysis.

### Preparative Examples

### General Information:

All reactions involving air- or moisture-sensitive reagents or intermediates were carried out in flame-dried glassware under an argon atmosphere. Dry solvents (THF, toluene, MeOH, DMF, DCM) were used as commercially available. ¹H-NMR and ¹³C-NMR were recorded on a Bruker DRX400 (400 MHz). Multiplicities are indicated as: br s (broadened singlet), s (singlet), d (doublet), t (triplet), q (quartet), quin (quintet), m (multiplet); and coupling constants (J) are given in Hertz (Hz). HPLC - electrospray mass spectra (HPLC ES-MS) were obtained using Waters Acquity Performance Liquid Chromatography (UPLC) equipped SQ 3100 Mass detector spectrometer. Column: Acquity UPLC BEH C18 1.7um, 2.1x50mm. Flow: 0.5ml/min. Eluents: A: H₂O with 0.05% formic acid and B: ACN with 0.05% TFA. All chemicals and solvents were purchased from commercial sources like Sigma-Aldrich, Fluka, TCI, Acros Organics, ABCR, Alfa Aesar, Enamine, VWR, Combi-Blocks, Apollo Scientific, Aquilla Pharmatech, Ark Pharm, D-L Chiral Chemicals, ChemBridge, Renno Tech, Accela, KeyOrganics, Pharmablock and Chem Impex. Unless otherwise noted, all commercially available compounds were used as received without further purifications.

**Abbreviations used in the description and in the Examples that follow are:**
mCPBA (meta-chloroperoxybenzoic acid), chx (cyclohexane), DAST (diethylaminosulfur trifluloride), DBU (1,8-diazabicyclo[5.4.0]undec-7-ene), DCM (dichloromethane), DIPEA (N,N-diisopropylethylamine), DMF (dimethylformamide), DMSO (dimethylsulfoxide), LCMS (liquid chromatography mass spectroscopy), Ms (mesyl, methanesulfonyl), p-TSA (PTSA, p-toluenesulfonic acid), Pd(dppf)Cl₂ ([1,1'-bis(diphenylphosphino)ferrocene]palladium(II) dichloride), SEM ([2-(trimethylsilyl)ethoxy]methyl), TBDMS (tert-*Butyldimethysilyl*), TFA (trifluoroacetic acid), THF (tetrahydrofuran), TMAD (*N*,*N*,*N*',*N*'-tetramethylazodicarboxamide), TMB (1,3,5-trimethoxybenzene), TLC (thin layer chromatography), TPP (triphenyl phosphine), Tos (tosyl, p-toluenesulfonyl);
ACK (Ammonium-Chloride-Potassium), CD (Cluster of differentiation), CCR (C-C motif receptor), DMEM (Dulbecco's Modified Eagle's Medium), FBS (Fetal Bovine Serum), EDTA (Ethylene-Diamine-Tetra-Acetic acid), FACS (Fluorescence activated cell sorting), HPbCD (2-Hydroxypropyl-β-cyclodextrin), HPMC (Hydroxypropylmethylcellulose), PEG400 (polyethylene glycol), Pen-Strep (Penicillin-Streptomycin), PBMCs (Peripheral blood mononuclear cells), PBS (Phosphate Buffered Saline).

### General Information

### General procedures and synthetic routes to disclosed compounds

In the following section some general procedures are described enabling persons skilled in the art to synthesize many key intermediates and final compounds disclosed in this patent. The synthetic approach is not limited to the outlined synthetic routes and reactions. Substances described herein can also be obtained by other conditions or reaction sequences as published in the literature.

### Synthetic routes

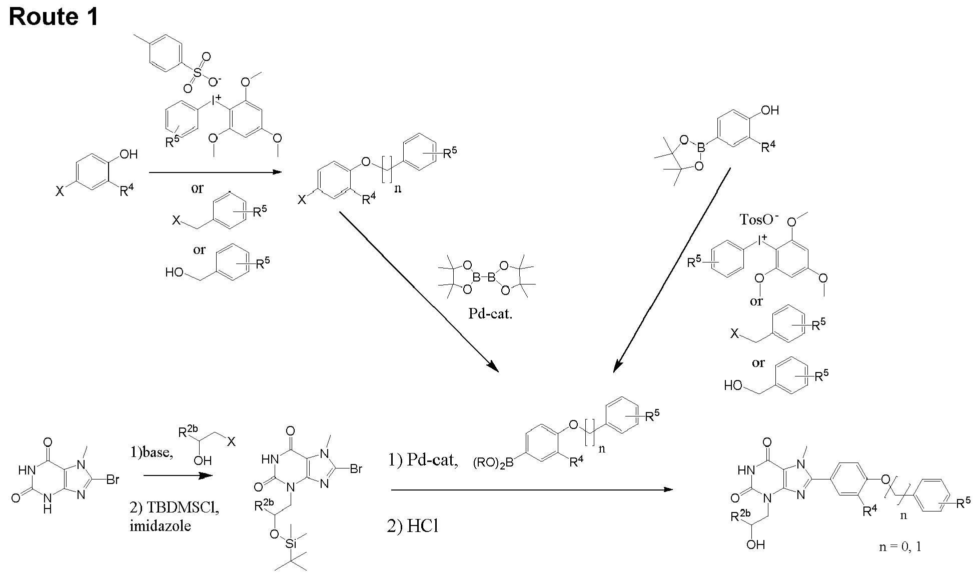

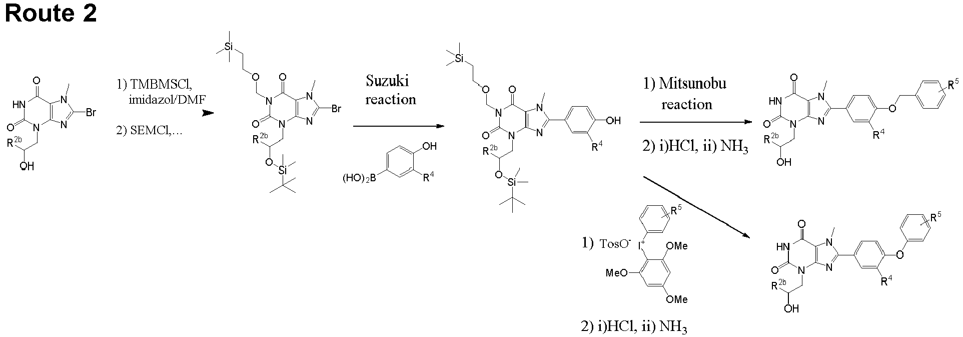

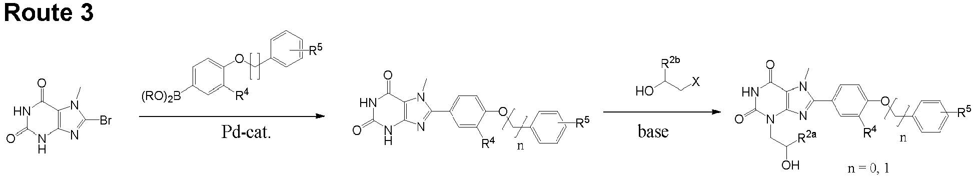

### General procedures

### General Procedure A: Mitsunobu reaction

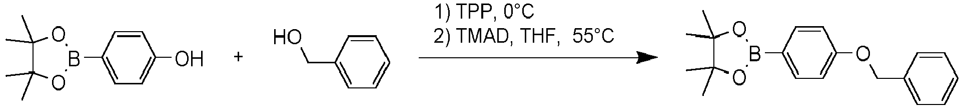

Dissolve a phenol (1.0 eq.) and the alcohol (1.5 eq.) in dry THF (0.1 M). Dry the solution with molecular sieve 4 Å. Remove the molecular sieve, cool down to 0°C, add TPP (2.2 eq.) and seal the reaction vessel. Stir at 0°C for 30 min, then add TMAD (2.6 eq.) and stir for additional 30 min at 0°C. Heat to 55°C and stir overnight. After completion of the reaction add Celite and evaporate solvent. Purify *via* normal phase column chromatography (silica, cyclohexane/ethyl acetate gradient).

### General Procedure B: nucleophilic substitution

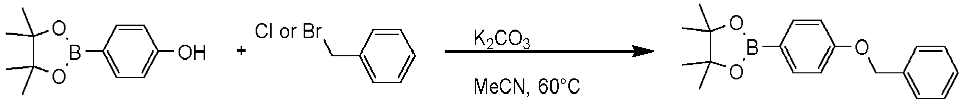

Dissolve a phenol (1.0 eq.), halide (1.5 eq.) and K₂CO₃ (3.0 eq.) in dry acetonitrile (0.1 M). Stir the mixture in a sealed reaction vessel at 60°C overnight. After reaction is completed add Celite and remove volatiles under reduced pressure. Purify via normal phase column chromatography (silica, cyclohexane/ethyl acetate gradient). An alternative suitable base is Cs₂CO₃, and acetonitrile can be replaced by THF, DMF or acetone. Depending on the availability of the starting materials the equivalents of phenol and halide can be inverted.

### General Procedure C: aryl-trimethoxyphenyl iodonium salts

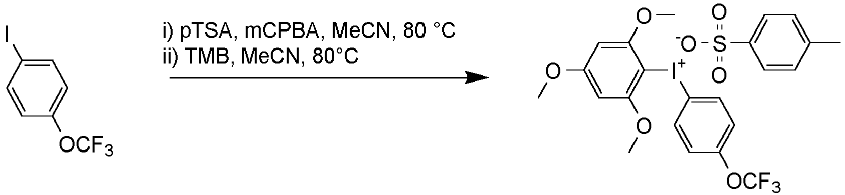

Dissolve an iodoarene (1.0 eq.) in dry acetonitrile (0.15 M). Acidify the solution with pTSA (1.1 eq.). Then add mCPBA (1.1 eq.) and stir at 80 °C for 1-2 h. Upon completion of the oxidation step add 1,3,5-trimethoxybenzene (TMB) and stir for additional 30 min at 80 °C. After completion add Celite and remove volatiles under reduced pressure. Purify via normal phase column chromatography (silica, DCM/methanol gradient).

### General Procedure D: O-arylation with aryl-trimethoxyphenyl iodonium salts

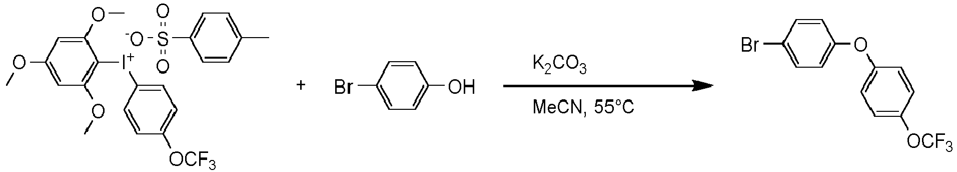

Suspend a phenol (1.2 eq.) and K₂CO₃ (3.0 eq.) in dry acetonitrile (0.25 M). Heat the mixture to 55°C and add the iodonium salt to the stirred solution. Continue stirring at 55°C overnight. Add Celite and remove volatiles under reduced pressure. Purify via normal phase column chromatography (silica, cyclohexane/ethyl acetate gradient). To extend the scope of the reaction, the phenol and K₂CO₃ can be replaced by an aliphatic alcohol and NaH.

### General Procedure E: Miyaura borylation

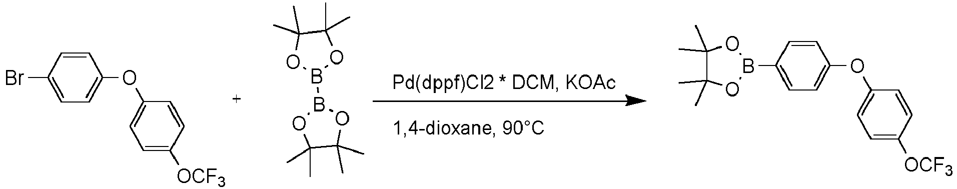

Dissolve an aryl halide (1.0 eq.), bis(pinacolato)diboron (1.2 eq.), Pd(dppf)Cl₂*DCM (0.1 eq.) and KOAc (3.0 eq.) in dry 1,4-dioxane (0.1 M). Seal the reaction vessel and stir at 90°C overnight. After completion add Celite and remove volatiles under reduced pressure. Purify via normal phase column chromatography (silica, cyclohexane/ethyl acetate gradient).

### General Procedure F: Suzuki cross coupling

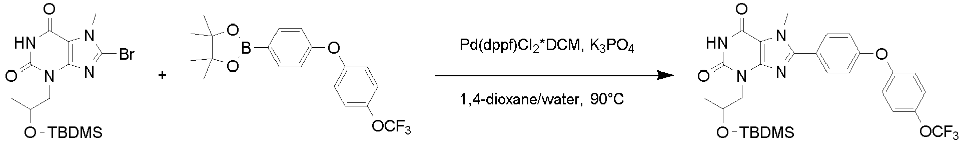

Dissolve a 8-bromoxanthine derivative (1.0 eq.), boronic ester (1.2 eq.), Pd(dppf)Cl₂*DCM (0.1 eq.) and K₃PO₄ (3.0 eq.) in a 4:1 mixture of 1,4-dioxane and water (0.1 M). Seal the reaction vessel and stir at 90°C overnight. After complete reaction add Celite and remove volatiles under reduced pressure. Purify via normal phase column chromatography (silica, cyclohexane/ethyl acetate/methanol gradient). The boronic ester can be replaced by a boronic acid.

### General-Procedure G: TBDMS deprotection of an alcohol

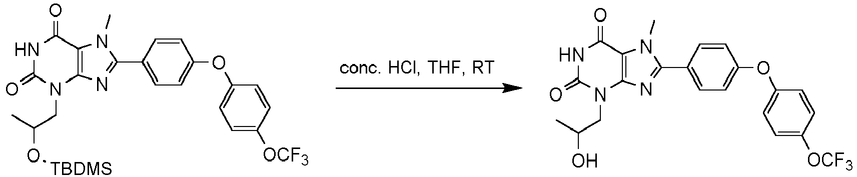

Dissolve a TBDMS-protected alcohol (1.0 eq.) in THF (0.1M). Add concentrated hydrochloric acid (15 eq.) and stir at room temperature overnight. After complete deprotection remove volatiles under reduced pressure. Dissolve the residue in DMSO and purify via reversed-phase HPLC (C18 column, water (0.1 %TFA) and ACN (0.1 %TFA) gradient). Desired fractions were lyophilized to give the final compound.

### General Procedure H: SEM protection

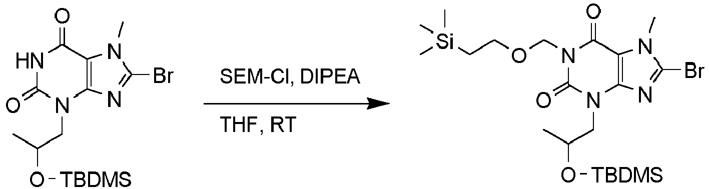

Dissolve xanthine derivative (1.0 eq.) in dry THF (0.2 M) and DIPEA (6.0 eq.). Add SEM-Cl (4.0 eq.) and continue stirring at room temperature overnight. Add more DIPEA and SEM-Cl in case incomplete reaction. After completion add saturated NaHCOs solution, concentrate the mixture under reduced pressure, add Celite and remove volatiles under reduced pressure. Purify via normal phase column chromatography (silica, cyclohexane/ethyl acetate gradient).

### General Procedure I: TBDMS and SEM-deprotection

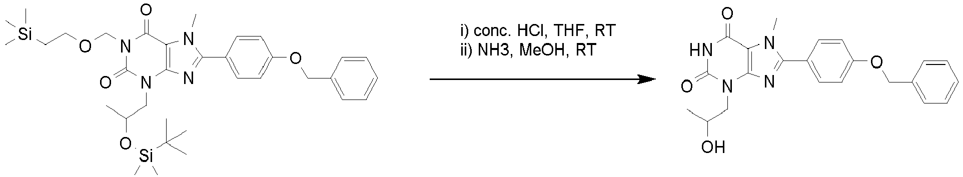

Dissolve a SEM and TBDMS protected compound (1.0 eq.) in THF (0.1 M). Add concentrated hydrochloric acid (15.0 eq.) and stir at room temperature overnight. Remove volatiles under reduced pressure and add ammonia in methanol (25.0 eq, 7 M). Stir at room temperature for 30min. Aagain remove volatiles under reduced pressure and dissolve the residue in DMSO and purify by reversed-phase HPLC (C18 column, water (0.1 %TFA) / acetonitrile (0.1 %TFA) gradient). The desired fractions were lyophilized to give the final compound.

### EXAMPLES

### Preparation of 8-bromo-3-(2-hydroxypropyl)-7-methyl-3,7-dihydro-1H-purine-2,6-dione (1)

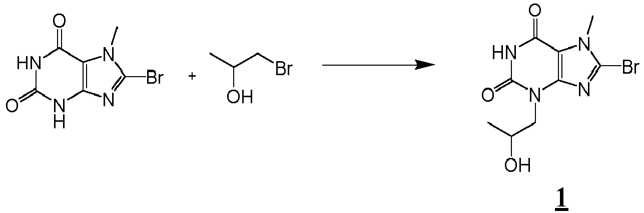

To a stirred solution of 8-bromo-7-methyl-3,7-dihydro-1H-purine-2,6-dione (5 g, 20.49 mmol) in DMF (50 mL) at room temperature were added 1-bromo-2-propanol (4.24 g, 30.73 mmol) and DBU (3.1 g, 20.49 mmol). The resultant reaction mixture was stirred for 16 h at 90 °C. After completion of reaction the reaction mass was cooled to room temperature. The reaction mixture was concentrated under reduced pressure to get crude reaction mass. It mass was diluted with DCM (100 mL) and precipitated solid was collected. The crude was dried under vacuum to afford compound **1** (3.66 g, 59%) as an off white solid. 1H NMR (400 MHz, DMSO-d6) δ: 11.16 (brs, 1H), 4.80 (d, J = 5.2 Hz, 1H), 4.06-4.00 (m, 1H), 3.88-3.78 (m, 4H), 3.69 (dd, J= 13.4, 5.6 Hz,1H), 1.05 (d, J = 6.0 Hz, 3H). LCMS (ESI+): found 303.1 [M+H]⁺, calculated 302.00 for C9H11BrN4O3.

### Preparation of 5-(difluoromethyl)-2,2-dimethyl-1,3-dioxane 69

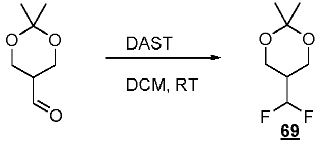

To a stirred solution of 2,2-dimethyl-1,3-dioxane-5-carbaldehyde (300 mg, 2.08 mmol), in DCM (6 mL) were added DAST (0.55 mL, 4.16 mmol) at room temperature, the reaction mixture was stirred at room temperature for 1 h. The reaction mixture was concentrated. The crude was purified by column chromatography, compound eluted in 10% EtOAc in petroleum ether. Fractions containing the compound were combined and evaporated to get **69** (0.2 g, 58%) as brown liquid. 1H NMR (400 MHz, CDCl3) δ ppm: 6.09 (td, J = 56.0, 7.2 Hz, 1H), 4.04-4.08 (m, 2H), 3.99-3.90 (m, 2H), 1.89-1.98 (m, 1H), 1.46 (s, 3H), 1.40 (s, 3H).

### Preparation of 2-(difluoromethyl)propane-1,3-diol 70

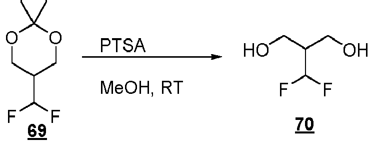

To a stirred solution of compound **69** (1.3 g, 7.82 mmol, 1.0 eq.) in MeOH:H₂O (3:1 26 mL) was added p-TSA (1.61 g 9.38 mmol, 1.2 eq.) at 0 °C, the reaction mixture was stirred at RT for 2 h. Reaction mixture was evaporated to remove excess volatiles, diluted in EtOAc and washed with water. The separated organic layer was dried over Na₂SO₄, filtered and concentrated under reduced pressure to afford compound **70** (400 mg, 41%) as a thick pale yellow syrup. 1H NMR (400 MHz, CDCl₃) δ ppm: 6.09 (td, J = 56.0, 5.2 Hz, 1H), 4.02-3.95 (m, 4H), 2.05-2.24 (m, 2H).

### Preparation of 3,3-difluoro-2-(hydroxymethyl)propyl methanesulfonate 111

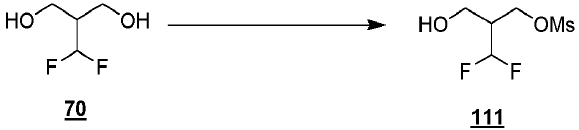

To a stirred solution of compound **70** (200 mg, 1.58 mmol, 1.0 eq.) in DCM (4 mL) was added triethylamine (0.22 mL, 1.58 mmol, 1.0 eq.), methane sulfonyl chloride (0.12 mL, 1.58 mmol, 1.0 eq.) at 0 °C. The reaction mixture was stirred at RT for 2 h. Reaction mixture was evaporated under reduced pressure to remove excess volatiles to afford compound **111** (120 mg, 37%). Crude compound was taken to next step without purification as thick pale-yellow syrup. 1H NMR (400 MHz, CDCl₃) δ ppm: 6.12-5.82 (m, 1H), 4.49-4.39 (m, 3H), 3.92-3.89 (m, 1H), 3.06-3.08 (m, 3H), 2.73-2.44 (m, 1H).

### Preparation of 8-bromo-3-(3,3-difluoro-2-(hydroxymethyl)propyl)-7-methyl-3,7-dihydro-1H-purine-2,6-dione 71

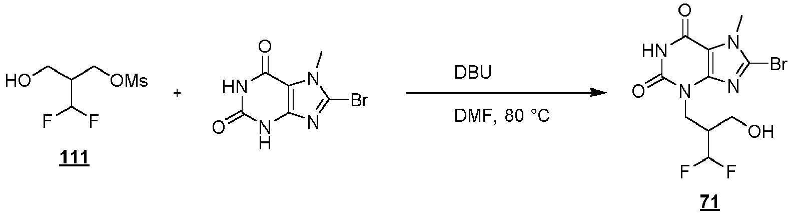

To a stirred solution of compound **111** (100 mg, 0.49 mmol, 1.0 eq.) in DMF (1 mL) was added 8-bromo-7-methyl-3,7-dihydro-1H-purine-2,6-dione (179 mg, 0.73 mmol, 1.5 eq.) and DBU (73 mg, 0.49 mmol, 1.0 eq.) The reaction mixture was stirred at 90 °C for 16 h. The reaction mixture was allowed to cool to room temperature. It was diluted with water, extracted with EtOAc (2 x 10 mL). The organic layer was washed with brine (30 mL), dried over anhydrous Na₂SO₄, filtered and concentrated under reduced pressure. The crude was purified by column chromatography using 3% methanol in dichloromethane as mobile phase to obtain compound **71** (100 mg, 58%) as a colorless solid. 1H NMR (400 MHz, CDCl₃) δ ppm: 11.31 (s, 1H), 6.11 (td, J = 56.0, 4 Hz, 1H), 4.84 (t, J = 5.2 Hz, 1H), 4.13 (dd, J = 14 Hz, 1H), 3.98 (dd, J= 14 Hz, 1H), 3.82 (s, 3H), 3.52 (t, J= 5.2 Hz, 2H). LCMS (ESI+): found 355.3 [M+H]⁺, calculated 354.0 for C10H11BrF2N4O3.

Building blocks disclosed in table **A-1** can be made e.g. in analogy to the methods described for derivatives **1** or **71**.

**Table A-1:**

| **Cpd no** | **structure** | **name** | **formula** | **exact mass** | **LCMS [M+H]⁺ found** |
|---|---|---|---|---|---|
| **1** | 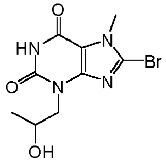 | 8-bromo-3-(2-hydroxypropyl)-7-methyl-3,7-dihydro-1H-purine-2,6-dione | C9H11BrN4 O3 | 302.00 | 303 |
| **2** | 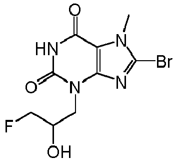 | 8-bromo-3-(3-fluoro-2-hydroxypropyl)-7-methyl-3,7-dihydro-1H-purine-2,6-dione | C9H10BrFN4 O3 | 319.99 | 321 |
| **3** | 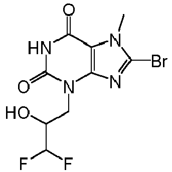 | 8-bromo-3-(3,3-difluoro-2-hydroxypropyl)-7-methyl-3,7-dihydro-1H-purine-2,6-dione | C9H9BrF2N4 O3 | 337.98 | 339 |
| **4** | 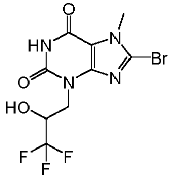 | 8-bromo-7-methyl-3-(3,3,3-trifluoro-2-hydroxypropyl)-3,7-dihydro-1H-purine-2,6-dione | C9H8BrF3N4 O3 | 355.97 | 357 |
| **5** | 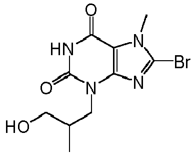 | 8-bromo-3-(3-hydroxy-2-methyl propyl)-7-methyl-3,7-dihydro-1H-purine-2,6-dione | C10H13BrN4 O3 | 316.02 | 317 |
| **7** | 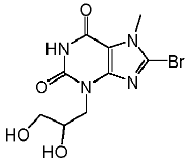 | 8-bromo-3-(2,3-dihydroxypropyl)-7-methyl-3,7-dihydro-1H-purine-2,6-dione | C9H11BrN4 O4 | 318.0 | 319 |
| **71** | 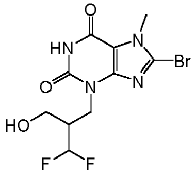 | 8-bromo-3-(3,3-difluoro-2-(hydroxymethyl)propyl )-7-methyl-3,7-dihydro-1H-purine-2,6-dione | C10H11BrF2 N4O3 | 352.00 | 353 |

### Preparation of 8-bromo-3-(2-((tert-butyldimethylsilyl)oxy)propyl)-7-methyl-3,7-dihydro-1H-purine-2,6-dione (8)

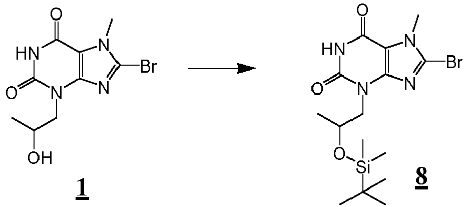

To a stirred solution of compound **1** (3.5 g, 11.55 mmol) in DMF (35 mL) at room temperature were added imidazole (5.2 g, 34.7 mmol) and TBDMSCI (2.36 g, 34.7 mmole). The resultant reaction mixture was stirred for 16 h at room temperature. After completion of reaction (monitored by LC-MS), the reaction mass was cooled to room temperature. The reaction mixture was evaporated under reduced pressure. The crude mass was purified by normal phase column chromatography (silica, cyclohexane/ethyl acetate gradient) to afford **8** (4.22 g, 87.5%) as a white solid. 1H NMR (400 MHz, DMSO-d₆) δ: 11.28 (s, 1H), 4.22-4.19 (m, 1H), 3.95-3.90 (m, 1H), 3.81 (s, 3H), 3.70 (dd, J = 13.2, 4.0 Hz, 1H), 1.12 (d, J = 6 Hz, 3H), 0.70 (s, 9H), -0.05 (s, 3H), -0.25 (s, 3H). LCMS (ESI+): found 417.3 [M+H]⁺, calculated 416.1 for C15H25BrN4O3Si.

### Preparation of 8-bromo-3-(2-(((tert-butyldimethylsilyl)oxy)methyl)-3,3-difluoropropyl)-7-methyl-3,7-dihydro-1H-purine-2,6-dione 13

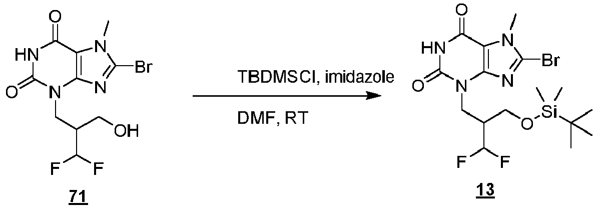

To a stirred solution of compound **71** (100 mg, 0.28 mmol, 1.0 equiv.), in dry DMF (2 mL) were added imidazole (39 mg, 0.57 mmol, 2.0 eq.) and TBDMS-Cl (86 mg, 0.57 mmol, 2.0 eq.). Then the reaction mixture was stirred at RT for 16 h. The reaction mixture was diluted with water and extracted with EtOAc (2 x 10 mL). The organic layer was washed with brine (30 mL), dried over anhydrous Na₂SO₄, filtered and concentrated under reduced pressure. The crude was purified by column chromatography using 3% Methanol in dichloromethane to obtain compound **13** (75 mg, 58%) as a colorless solid. LCMS (ESI+): found 467.3 [M+H]⁺, calculated 466.01 for C16H25BrF2N4O3Si. 1H NMR (400 MHz, DMSO-d₆) δ ppm: 11.30 (s, 1H), 6.11 (td, J = 56.0, 4.4 Hz, 1H), 4.13-4.04 (m, 2H), 3.81 (s, 3H), 3.72 (d, J = 4.8 Hz, 2H), 2.67-2.51 (m, 1H), 0.81 (s, 9H), -0.010 (s, 3H), -0.018 (s, 3H).

Building blocks in the following table **A-2** can be synthesized e.g. as exemplified for compound **8** or **13.**

**Table A-2:**

| **Cpd no** | **structure** | **name** | **formula** | **exact mass** | **[M+H]⁺ found** |
|---|---|---|---|---|---|
| **8** | 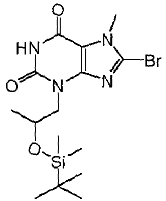 | 8-bromo-3-(2-((tertbutyldimethylsilyl)oxy) propyl)-7-methyl-3,7-dihydro-1H-purine-2,6-dione | C15H25BrN4 O3Si | 416.1 | 417 |
| **9** | 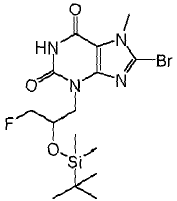 | 8-bromo-3-(2-((tertbutyldimethylsilyl)oxy) -3-fluoropropyl)-7-methyl-3,7-dihydro-1H-purine-2,6-dione | C15H24BrFN 4O3Si | 434.1 | 435 |
| **10** | 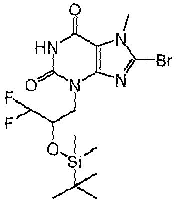 | 8-bromo-3-(2-((tertbutyldimethylsilyl)oxy) -3,3-difluoropropyl)-7-methyl-3,7-dihydro-1H-purine-2,6-dione | C15H23BrF2 N4O3Si | 452.1 | 453 |
| **11** | 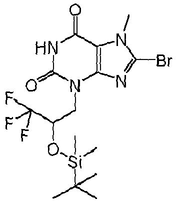 | 8-bromo-3-(2-((tertbutyldimethylsilyl)oxy) -3,3,3-trifluoropropyl)-7-methyl-3,7-dihydro-1H-purine-2,6-dione | C15H22BrF3 N4O3Si | 470.06 | 471 |
| **12** | 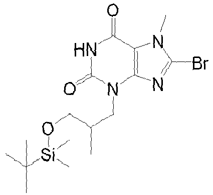 | 8-bromo-3-(3-((tertbutyldimethylsilyl)oxy) -2-methylpropyl)-7-methyl-3,7-dihydro-1 H-purine-2,6-dione | C16H27BrN4 O3Si | 430.1 | 431 |
| **13** | 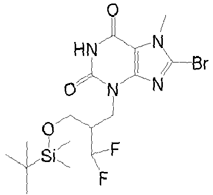 | 8-bromo-3-(2-(((tertbutyldimethylsilyl)oxy) methyl)-3,3-difluoropropyl)-7-methyl-3,7-dihydro-1H-purine-2,6-dione | C16H25BrF2 N4O3Si | 466.01 | 467 |
| **14** | 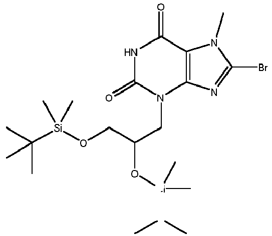 | 3-(2,3-bis((tert-butyldimethylsilyl)\oxy) propyl)-8-bromo-7-methyl-3,7-dihydro-1H-purine-2,6-dione | C21H39BrN4 O4Si2 | 546.17 | 547 |

### Preparation of 8-(4-(benzyloxy)-3-methoxyphenyl)-7-methyl-3,7-dihydro-1H-purine-2,6-dione 15

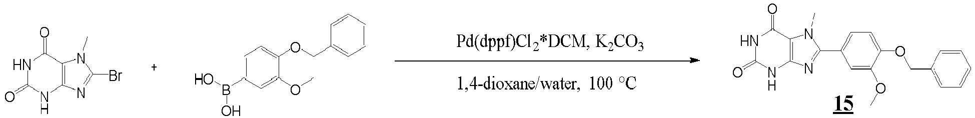

500 mg 8-bromo-7-methyl-3,7-dihydro-1H-purine-2,6-dione, 790 mg (4-(benzyloxy)-3-methoxyphenyl)boronic acid, 170 mg Pd(dppf)Cl2*DCM and 423 mg K₂CO₃ were suspended under a nitrogen atmosphere in 20 ml 1,4-dioxane/water (1:1) and heated to 100 °C until reaction was complete. After cooling to room temperature 15 ml water were added to precipitate the product which was collected by filtration. The crude was then triturated with 30 ml acetonitrile overnight. The solid was collected, washed and dried to yield 680 mg of compound **15.** LCMS (ESI-): found 377 [M-H]⁻, calculated 378.13 for C20H18N4O4.

### Preparation of 8-(4-(benzyloxy)-3-methoxyphenyl)-3-(2-hydroxypropyl)-7-methyl-3,7-dihydro-1H-purine-2,6-dione 16

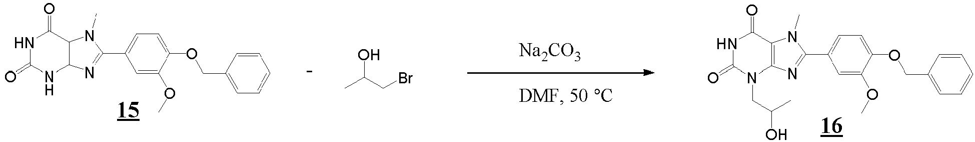

150 mg compound **15,** 0.051 ml 1-bromo-2-propanol, 46 mg Na₂CO₃ were suspended under a nitrogen atmosphere in 3.9 ml DMF and stirred at 50 °C for 72 h. After cooling to room temperature some water was added to precipitate the product which was collected, washed and dried. Pure compound **16** was obtained after purification by preparative TLC (DCM/MeOH, 15:1). LCMS (ESI+): found 437 [M+H]⁺, calculated 436.17 for C23H24N4O5.

Compounds in the following table **A-3** were synthesized e.g. as exemplified by compound **16.**

**Table A-3:**

| **Cpd no** | **structure** | **name** | **formula** | **exact mass** | **[M+H]⁺ found** |
|---|---|---|---|---|---|
| **16** | 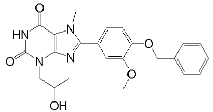 | 8-(4-(benzyloxy)-3-methoxyphenyl)-3-(2-hydroxypropyl)-7-methyl-3,7-dihydro-1H-purine-2,6-dione | C23H24N4O 5 | 436.17 | 437 |
| **52** | 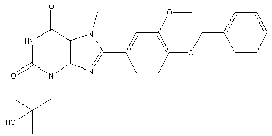 | 8-(4-(benzyloxy)-3-methoxyphenyl)-3-(2-hydroxy-2-methyl propyl)-7-methyl-3,7-dihydro-1H-purine-2,6-dione | C24H26N4O 5 | 450.19 | 451 |
| **53** | 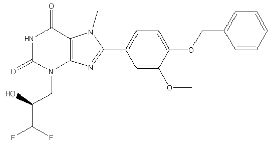 | (S)-8-(4-(benzyloxy)-3-methoxyphenyl)-3-(3,3-difluoro-2-hydroxypropyl)-7-methyl-3,7-dihydro-1H-purine-2,6-dione | C23H22F2N 4O5 | 472.16 | 473 |
| **54** | 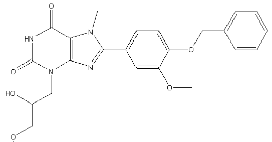 | 8-(4-(benzyloxy)-3-methoxyphenyl)-3-(2-hydroxy-3-methoxypropyl)-7-methyl-3,7-dihydro-1H-purine-2,6-dione | C24H26N4O 6 | 466.19 | 467 |
| **55** | 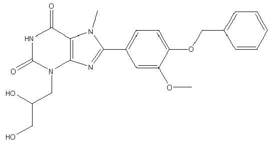 | 8-(4-(benzyloxy)-3-methoxyphenyl)-3-(2,3-dihydroxypropyl)-7-methyl-3,7-dihydro-1H-purine-2,6-dione | C23H24N4O 6 | 452.17 | 453 |
| **56** | 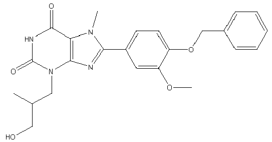 | 8-(4-(benzyloxy)-3-methoxyphenyl)-3-(3-hydroxy-2-methyl propyl)-7-methyl-3,7-dihydro-1H-purine-2,6-dione | C24H26N4O 5 | 450.19 | 451 |
| **57** | 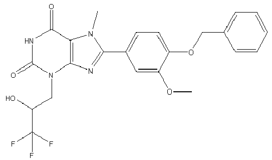 | 8-(4-(benzyloxy)-3-methoxyphenyl)-7-methyl-3-(3,3,3-trifluoro-2-hydroxypropyl)-3,7-dihydro-1H-purine-2,6-dione | C23H21F3N 4O5 | 490.15 | 491 |
| **58** | 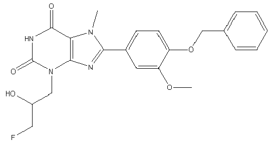 | 8-(4-(benzyloxy)-3-methoxyphenyl)-3-(3-fluoro-2-hydroxypropyl)-7-methyl-3,7-dihydro-1H-purine-2,6-dione | C23H23FN4 O5 | 454.17 | 455 |
| **59** | 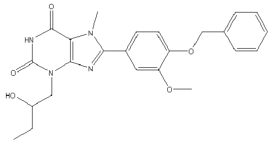 | 8-(4-(benzyloxy)-3-methoxyphenyl)-3-(2-hydroxybutyl)-7-methyl-3,7-dihydro-1H-purine-2,6-dione | C24H26N4O 5 | 450.19 | 451 |
| **60** | 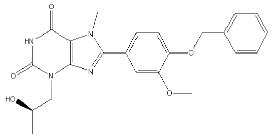 | (R)-8-(4-(benzyloxy)-3-methoxyphenyl)-3-(2-hydroxypropyl)-7-methyl-3,7-dihydro-1H-purine-2,6-dione | C23H24N4O 5 | 436.17 | 437 |
| **61** | 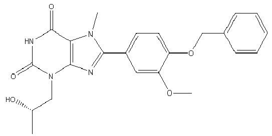 | (S)-8-(4-(benzyloxy)-3-methoxyphenyl)-3-(2-hydroxypropyl)-7-methyl-3,7-dihydro-1H-purine-2,6-dione | C23H24N4O 5 | 436.17 | 437 |

### Preparation of (4-(trifluoromethoxy)phenyl)(2,4,6-trimethoxyphenyl)iodonium 4-methylbenzenesulfonate 104

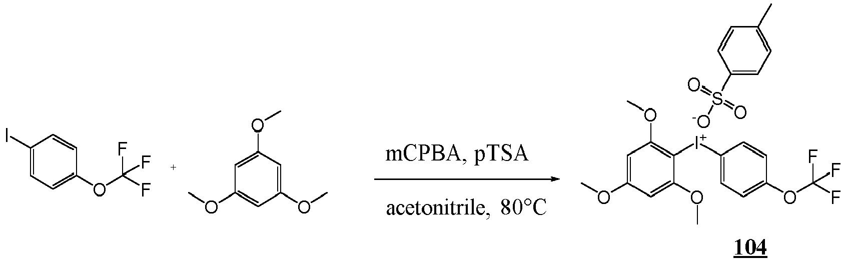

20.0 g 1-iodo-4-(trifluoromethoxy)benzene were dissolved in 250 ml dry acetonitrile. 13.15 g pTSA and 26.0 g mCPBA were added and the reaction was stirred at 80 °C for 2 h. Upon completion of the oxidation 12.8 g 1,3,5-trimethoxybenzene were added and stirring was continued for additional 30 min at 80 °C. The mixture was concentrated under reduced pressure and the residue was absorbed on Celite. Volatiles were removed under reduced pressure. Purification was achieved via normal phase column chromatography (silica, DCM/methanol gradient). LCMS (ESI+): found 454.8 [M]⁺, calculated 455,00 for C16H15F3IO4⁺.

### Preparation of (2-(trifluoromethoxy)phenyl)(2,4,6-trimethoxyphenyl)iodonium 4-methylbenzenesulfonate 108

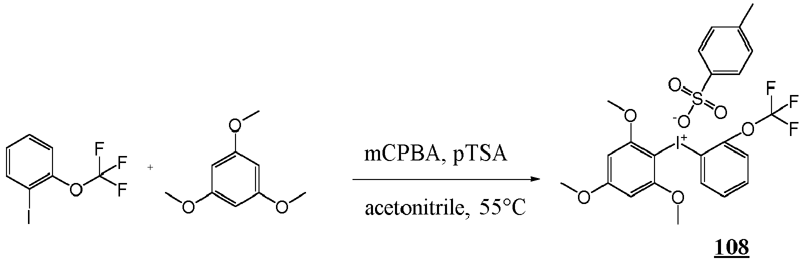

150 mg 1-iodo-2-(trifluoromethoxy)benzene were dissolved in 3 ml dry acetonitrile. 117 mg pTSA and 167 mg mCPBA were added and the reaction was stirred at 55 °C for 1 h. Upon completion of the oxidation 96 mg 1,3,5-trimethoxybenzene were added and stirring was continued for additional 30 min at 55 °C. The mixture was absorbed on Celite. Volatiles were removed under reduced pressure. Purification was achieved via normal phase column chromatography (silica, DCM/methanol gradient). LCMS (ESI+): found 454.6 [M]⁺, calculated 455.0 for C16H15F3IO4⁺.

Compounds in the following table **A-4** were synthesized as exemplified by compound **104, 108** or literature. Other iodonium salts also were commercially available.

**Table A-4:**

| **Cpd no** | **structure** | **name** | **formula** | **exact mass** | **[M-TosO⁻]⁺ found** |
|---|---|---|---|---|---|
| **104** | 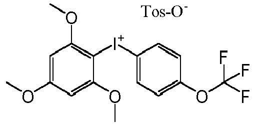 | (4-(trifluoromethoxy)phenyl)(2, 4,6-trimethoxyphenyl)iodonium 4-methylbenzenesulfonate | C23H22F 3IO7S | 626.01 | 454.8 |
| **107** | 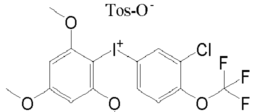 | (3-chloro-4-(trifluoromethoxy)phenyl)(2, 4,6-trimethoxyphenyl)iodonium 4-methylbenzenesulfonate | C23H21Cl F3IO7S | 659.97 | 488.6 |
| **108** | 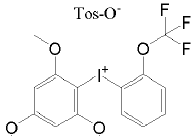 | (2-(trifluoromethoxy)phenyl)(2, 4,6-trimethoxyphenyl)iodonium 4-methylbenzenesulfonate | C23H22F 3IO7S | 626.01 | 454.6 |
| **109** | 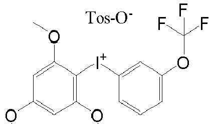 | (3-(trifluoromethoxy)phenyl)(2, 4,6-trimethoxyphenyl)iodonium 4-methylbenzenesulfonate | C23H22F 3IO7S | 626.01 | 454.7 |
| **129** | 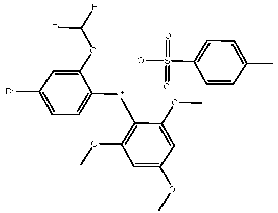 | (4-bromo-2-(difluoromethoxy)phenyl)(2, 4,6-trimethoxyphenyl)iodonium 4-methylbenzenesulfonate | C23H22Br F2IO7S | 685.93 | 515.0 |
| **158** | 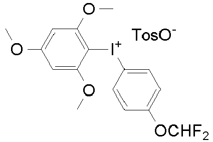 | (4-(difluoromethoxy)phenyl)(2, 4,6-trimethoxyphenyl)iodonium 4-methylbenzenesulfonate | C23H23F 2IO7S | 608.02 | 436.9 |
| **159** | 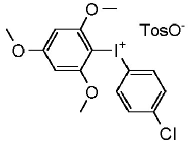 | (4-chlorophenyl)(2,4,6-trimethoxyphenyl)iodonium 4-methylbenzenesulfonate | C22H22Cl IO6S | 575.99 | 405.0 |
| **160** | 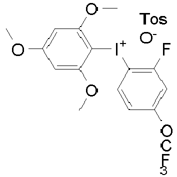 | (2-fluoro-4-(trifluoromethoxy)phenyl)(2, 4,6-trimethoxyphenyl)iodonium 4-methylbenzenesulfonate | C23H21F 4IO7S | 644.00 | 472.8 |
| **168** | 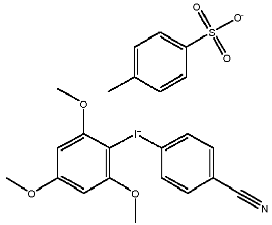 | (4-cyanophenyl)(2,4,6-trimethoxyphenyl)iodonium 4-methylbenzenesulfonate | C23H22IN O6S | 567.02 | 396 |
| **169** | 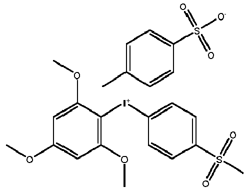 | (4-(methylsulfonyl)phenyl)(2,4, 6-trimethoxyphenyl)iodonium 4-methylbenzenesulfonate | C23H25IO 8S2 | 620.00 | 448.7 |
| **170** | 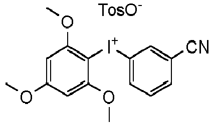 | (3-cyanophenyl)(2,4,6-trimethoxyphenyl)iodonium 4-methylbenzenesulfonate | C23H22IN O6S | 567.02 | 395.8 |
| **171** | 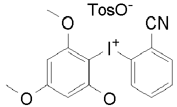 | (2-cyanophenyl)(2,4,6-trimethoxyphenyl)iodonium 4-methylbenzenesulfonate | C23H22IN O6S | 567.02 | 395.7 |
| **172** | 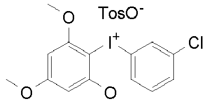 | (3-chlorophenyl)(2,4,6-trimethoxyphenyl)iodonium 4-methylbenzenesulfonate | C22H22Cl IO6S | 575.99 | 404.8 |
| **173** | 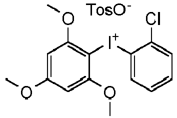 | (2-chlorophenyl)(2,4,6-trimethoxyphenyl)iodonium 4-methylbenzenesulfonate | C22H22Cl IO6S | 575.99 | 404.9 |
| **174** | 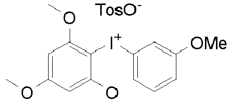 | (3-methoxyphenyl)(2,4,6-trimethoxyphenyl)iodonium 4-methylbenzenesulfonate | C23H25IO 7S | 572.04 | 400.9 |
| **175** | 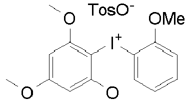 | (2-methoxyphenyl)(2,4,6-trimethoxyphenyl)iodonium 4-methylbenzenesulfonate | C23H25IO 7S | 572.04 | 400.9 |
| **189** | 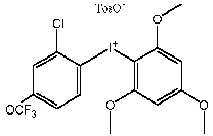 | (2-chloro-4-(trifluoromethoxy)phenyl)(2, 4,6-trimethoxyphenyl)iodonium 4-methylbenzenesulfonate | C23H21Cl F3IO7S | 659.97 | 488.6 |

### Preparation of 1-bromo-4-(4-(trifluoromethoxy)phenoxy)benzene 105

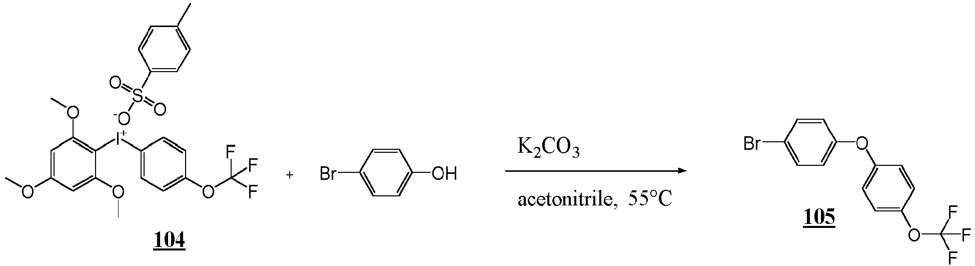

8.29 g 4-bromophenol, 20.0 g iodonium tosylate **104,** 17.65 g K₂CO₃ in 250 ml acetonitrile were heated to 55°C overnight. Some solvent was removed under reduced pressure and the residue was absorbed on Celite. Purification of compound **105** was achieved by normal phase column chromatography (silica, cyclohexane/ethyl acetate gradient). GCMS (EI): m/z found 334.2 [M] ⁺, calculated 333.96 for C13H8BrF3O2

### Preparation of 4,4,5,5-tetramethyl-2-(4-(4-(trifluoromethoxy)phenoxy)phenyl)-1,3,2-dioxaborolane 106

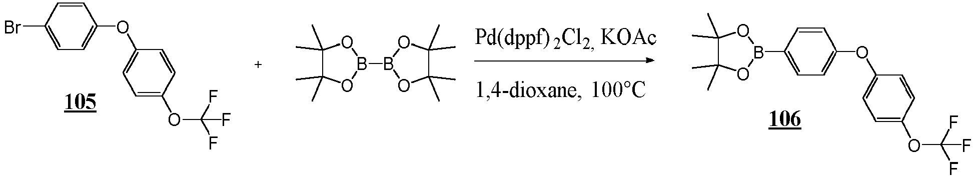

10 g bromide **105,** 11.43 g bis(pinacolato)diborane, 1.22 g [1,1'-bis(diphenylphosphino) ferrocene]palladium dichloride dichloromethane and 11.91 g potassium acetate were mixed in 200 ml 1,4-dioxane. The reaction was stirred at 100 °C until complete. Some Soolvent was removed under reduced pressure and the residue absorbed on Celite. Purification was achieved by normal phase column chromatography (silica, cyclohexane/ethyl acetate gradient). LCMS (ESI+): found 381.2 [M+H]⁺, calculated 380.14 for C19H20BF3O4.

### Preparation of 4-bromo-2-(difluoromethoxy)-1-((4-methylbenzyl)oxy)benzene 130

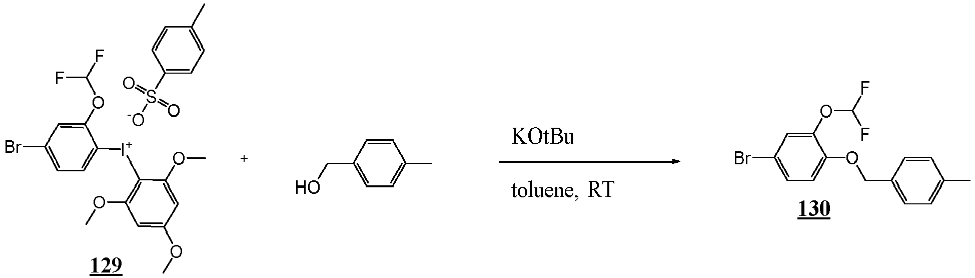

52 mg p-tolylmethanol and 53 mg KOtBu were added to 2 ml ice cold toluene under a nitrogen atmosphere. The mixture was allowed to warm to room temperature. 388 mg iodonium salt **129** were added and stirring at room temperature was continued for 1.5 h. the reaction mixture was a absorbed on Celite and purified by normal phase column chromatography (silica, cyclohexane/ethyl acetate gradient). LCMS (ESI+): found 343.4 [M+H]⁺, calculated 342.01 for C15H13BrF2O2.

### Preparation of 2-(3-(difluoromethoxy)-4-((4-methylbenzyl)oxy)phenyl)-4,4,5,5-tetramethyl-1,3,2-dioxaborolane 131

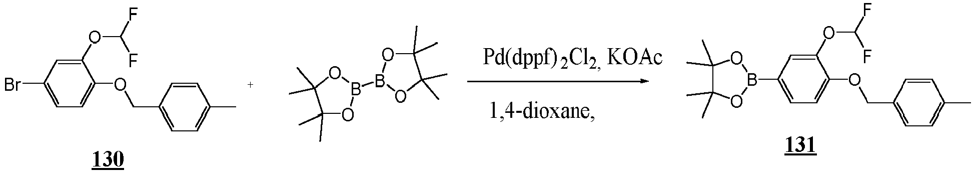

85 mg bromide **130,** 94 mg bis(pinacolato)diborane, 42 mg [1,1'-bis(diphenylphosphino)ferrocene]palladium dichloride dichloromethane and 49 mg potassium acetate were mixed in 1.5mL 1,4-dioxane. The reaction was stirred at 85 °C until complete and then directly absorbed on Celite. Purification was achieved by normal phase column chromatography (silica, cyclohexane/ethyl acetate gradient). LCMS (ESI+): found 391.3 [M+H]⁺, calculated 390.18 for C21H25BF2O4.

### Preparation of 2-(4-((4-(difluoromethyl)benzyl)oxy)-3-fluorophenyl)-4,4,5,5-tetramethyl-1,3,2-dioxaborolane 17

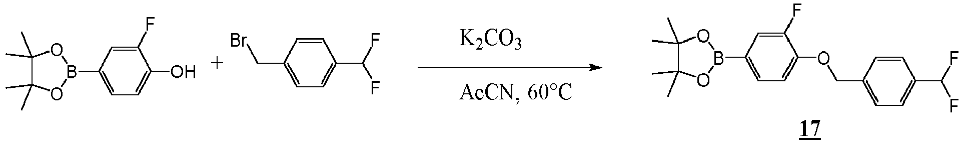

500 mg 2-fluoro-4-(4,4,5,5-tetramethyl-1,3,2-dioxaborolan-2-yl)phenol, 603 mg (1.3 equ.) 1-(bromomethyl)-4-(difluoromethyl)benzene and 138 mg K₂CO₃ were heated in 10 ml dry acetonitrile to 60 °C and stirred until reaction was complete. The mixture was absorbed on Celite, dried and purified by normal phase column chromatography (silica, cyclohexane/ethyl acetate gradient) to obtain compound **17**. LCMS (ESI+): found 379 [M+H]⁺, calculated 378.16 for C20H22BF3O3.

### Preparation of 2-(4-(benzyloxy)-3-methoxyphenyl)-4,4,5,5-tetramethyl-1,3,2-dioxaborolane 20

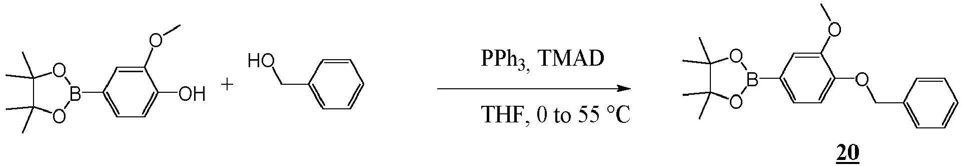

324 mg benzyl alcohol and 500 mg 2-methoxy-4-(4,4,5,5-tetramethyl-1,3,2-dioxaborolan-2-yl)phenol in 5 ml THF were dried over molecular sieve 3 Å and then transferred to 1.15 g triphenylphosphine in 5 ml THF at 0°C under nitrogen. 860 mg TMAD were added. After 30 min the reaction mixture was heated to 55°C until reaction was complete. The mixture was absorbed on Celite, dried and purified by normal phase column chromatography (silica, cyclohexane/ethyl acetate gradient) to obtain compound **20.** LCMS (ESI+): found 341 [M+H]⁺, calculated 340.18 for C20H25BO4.

### Preparation of 1-(benzyloxy)-4-bromo-2-(trifluoromethoxy)benzene 23

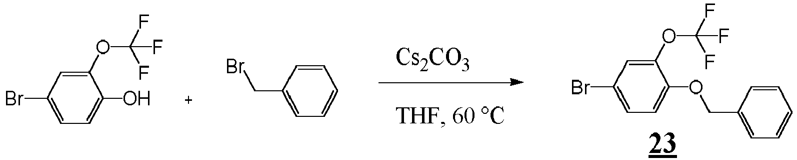

Benzyl bromide (4.16 g), 4-bromo-2-(trifluoromethoxy)phenol (2.5 g) and Cs₂CO₃ (9.5 g) were mixed in 30 ml THF and stirred at 60 °C until the reaction was complete. The mixture was absorbed on Celite, dried and purified by normal phase column chromatography (silica, cyclohexane/ethyl acetate gradient) to obtain compound **23.** LCMS (ESI+): found 348.6 [M+H]⁺, calculated 347.98 for C14H10BrF3O2.

### Preparation of 2-(4-(benzyloxy)-3-(trifluoromethoxy)phenyl)-4,4,5,5-tetramethyl-1,3,2-dioxaborolane 24

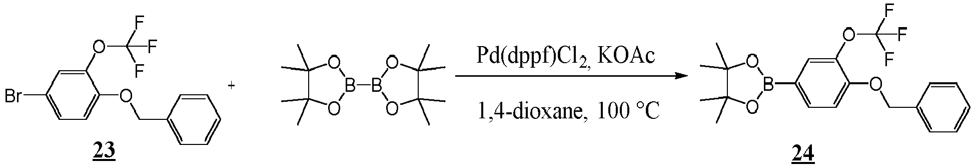

3.5 g bromo derivative **23, 0.411** g Pd(dppf)Cl2*DCM, 4.0 g potassium acetate and 3.84 g bis(pinacolato)diboron in 30 ml 1,4-dioxane were heated to 100°C until reaction was complete. Most of the solvent was evaporated, the residue absorbed on Celite and purified via normal phase column chromatography (silica, cyclohexane/ethyl acetate gradient) to afford pure compound **24.** LCMS (ESI+): found 395 [M+H]⁺, calculated 394.16 for C20H22BF3O4.

Building blocks in the following table A-5 were synthesized e.g. as exemplified by compound **17, 20, 24, 106, 131.**

**Table A-5:**

| **Cpd no** | **structure** | **name** | **formula** | **exact mass** | **[M+H]⁺ found** |
|---|---|---|---|---|---|
| **17** | 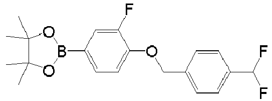 | 2-(4-((4-(difluoromethyl)benzyl)oxy )-3-fluorophenyl)-4,4,5,5-tetramethyl-1,3,2-dioxaborolane | C20H22B F3O3 | 378.16 | 379.3 |
| **20** | 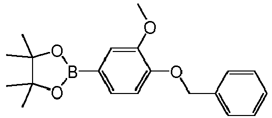 | 2-(4-(benzyloxy)-3-methoxyphenyl)-4,4,5,5-tetramethyl-1,3,2-dioxaborolane | C20H25B O4 | 340.18 | 341.3 |
| **21** | 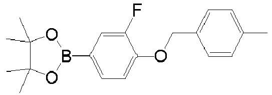 | 2-(3-fluoro-4-((4-methylbenzyl)oxy)phenyl)-4,4,5,5-tetramethyl-1,3,2-dioxaborolane | C20H24B FO3 | 342.18 | 343.1 |
| **22** | 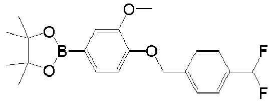 | 2-(4-((4-(difluoromethyl)benzyl)oxy )-3-methoxyphenyl)-4,4,5,5-tetramethyl-1,3,2-dioxaborolane | C21H25B F2O4 | 390.18 | 391.3 |
| **24** | 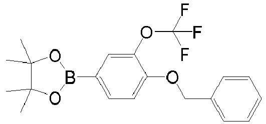 | 2-(4-(benzyloxy)-3-(trifluoromethoxy)phenyl)-4,4,5,5-tetramethyl-1,3,2-dioxaborolane | C20H22B F3O4 | 394.16 | 395.2 |
| **25** | 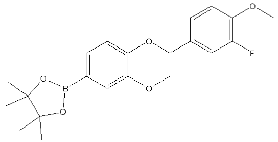 | 2-(4-((3-fluoro-4-methoxybenzyl)oxy)-3-methoxyphenyl)-4,4,5,5-tetramethyl-1,3,2-dioxaborolane | C21H26B FO5 | 388.19 | 389.3 |
| **26** | 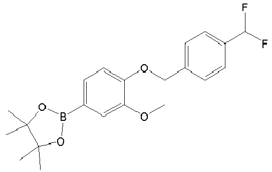 | 2-(4-((4-(difluoromethyl)benzyl)oxy )-3-methoxyphenyl)-4,4,5,5-tetramethyl-1,3,2-dioxaborolane | C21H25B F2O4 | 390.18 | 391.1 |
| **27** | 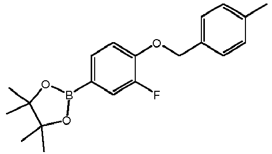 | 2-(3-fluoro-4-((4-methylbenzyl)oxy)phenyl)-4,4,5,5-tetramethyl-1,3,2-dioxaborolane | C20H24B FO3 | 342.18 | 343.1 |
| **28** | 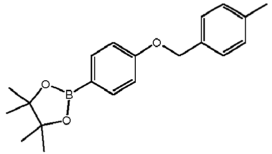 | 4,4,5,5-tetramethyl-2-(4-((4-methylbenzyl)oxy)phenyl)-1,3,2-dioxaborolane | C20H25B O3 | 324.19 | 325.3 |
| **29** | 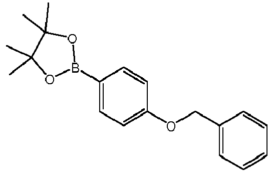 | 2-(4-(benzyloxy)phenyl)-4,4,5,5-tetramethyl-1,3,2-dioxaborolane | C19H23B O3 | 310.17 | 311.3 |
| **30** | 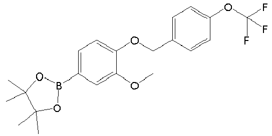 | 2-(3-methoxy-4-((4-(trifluoromethoxy)benzyl)o xy)phenyl)-4,4,5,5-tetramethyl-1,3,2-dioxaborolane | C21H24B F3O5 | 424.17 | 425.4 |
| **31** | 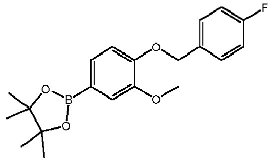 | 2-(4-((4-fluorobenzyl)oxy)-3-methoxyphenyl)-4,4,5,5-tetramethyl-1,3,2-dioxaborolane | C20H24B FO4 | 358.18 | 359.2 |
| **32** | 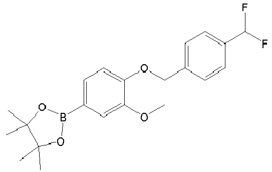 | 2-(4-((4-(difluoromethyl)benzyl)oxy )-3-methoxyphenyl)-4,4,5,5-tetramethyl-1,3,2-dioxaborolane | C21H25B F2O4 | 390.18 | 391.2 |
| **33** | 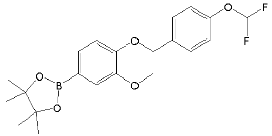 | 2-(4-((4-(difluoromethoxy)benzyl)o xy)-3-methoxyphenyl)-4,4,5,5-tetramethyl-1,3,2-dioxaborolane | C21H25B F2O5 | 406.18 | 407.2 |
| **34** | 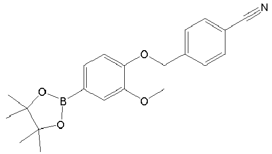 | 4-((2-methoxy-4-(4,4,5,5-tetramethyl-1,3,2-dioxaborolan-2-yl)phenoxy)methyl)benzon itrile | C21H24B NO4 | 365.18 | 366.1 |
| **35** | 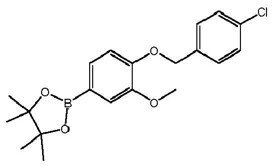 | 2-(4-((4-chlorobenzyl)oxy)-3-methoxyphenyl)-4,4,5,5-tetramethyl-1,3,2-dioxaborolane | C20H24B ClO4 | 374.15 | 375.1 |
| **36** | 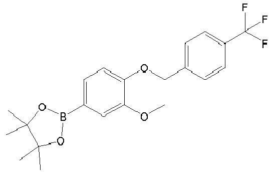 | 2-(3-methoxy-4-((4-(trifluoromethyl)benzyl)oxy )phenyl)-4,4,5,5-tetramethyl-1,3,2-dioxaborolane | C21H24B F3O4 | 408.17 | 409.3 |
| **37** | 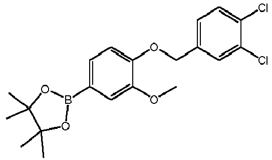 | 2-(4-((3,4-dichlorobenzyl)oxy)-3-methoxyphenyl)-4,4,5,5-tetramethyl-1,3,2-dioxaborolane | C20H23B Cl2O4 | 408.11 | 409.3 |
| **38** | 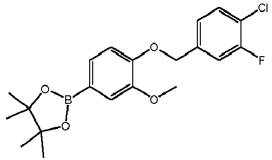 | 2-(4-((4-chloro-3-fluorobenzyl)oxy)-3-methoxyphenyl)-4,4,5,5-tetramethyl-1,3,2-dioxaborolane | C20H23B ClFO4 | 392.14 | 393.3 |
| **39** | 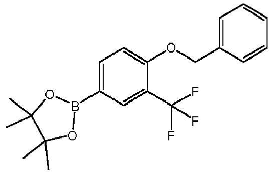 | 2-(4-(benzyloxy)-3-(trifluoromethyl) phenyl)-4,4,5,5-tetramethyl-1,3,2-dioxaborolane | C20H22B F3O3 | 378.16 | 379.5 |
| **40** | 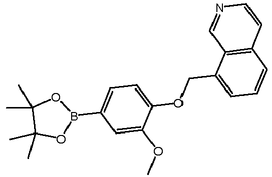 | 8-((2-methoxy-4-(4,4,5,5-tetramethyl-1,3,2-dioxaborolan-2-yl)phenoxy)methyl)isoquin oline | C23H26B NO4 | 391.20 | 392.1 |
| **41** | 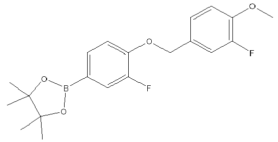 | 2-(3-fluoro-4-((3-fluoro-4-methoxybenzyl)oxy)phenyl )-4,4,5,5-tetramethyl-1,3,2-dioxaborolane | C20H23B F2O4 | 376.17 | 377.3 |
| **45** | 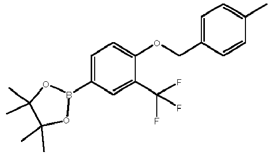 | 4,4,5,5-tetramethyl-2-(4-((4-methylbenzyl)oxy)-3-(trifluoromethyl) phenyl)-1,3,2-dioxaborolane | C21H24B F3O3 | 392.18 | 392.9 |
| **46** | 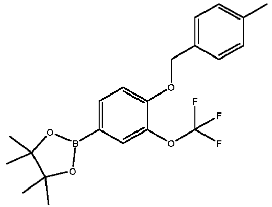 | 4,4,5,5-tetramethyl-2-(4-((4-methylbenzyl)oxy)-3-(trifluoromethoxy)phenyl)-1,3,2-dioxaborolane | C21H24B F3O4 | 408.17 | 409.4 |
| **47** | 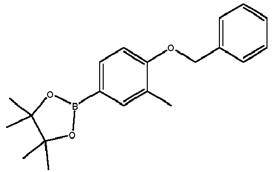 | 2-(4-(benzyloxy)-3-methylphenyl)-4,4,5,5-tetramethyl-1,3,2-dioxaborolane | C20H25B O3 | 324.19 | 325.3 |
| **48** | 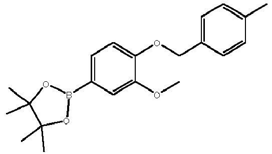 | 2-(3-methoxy-4-((4-methylbenzyl)oxy)phenyl)-4,4,5,5-tetramethyl-1,3,2-dioxaborolane | C21H27B O4 | 354.20 | 355.3 |
| **49** | 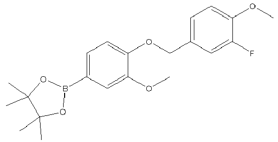 | 2-(4-((3-fluoro-4-methoxybenzyl)oxy)-3-methoxyphenyl)-4,4,5,5-tetramethyl-1,3,2-dioxaborolane | C21H26B FO5 | 388.19 | 389.2 |
| **50** | 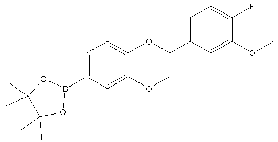 | 2-(4-((4-fluoro-3-methoxybenzyl)oxy)-3-methoxyphenyl)-4,4,5,5-tetramethyl-1,3,2-dioxaborolane | C21H26B FO5 | 388.19 | 389.2 |
| **51** | 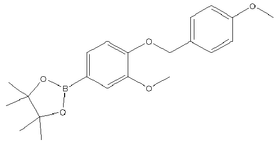 | 2-(3-methoxy-4-((4-methoxybenzyl)oxy)phenyl )-4,4,5,5-tetramethyl-1,3,2-dioxaborolane | C21H27B O5 | 370.20 | 371.2 |
| **112** | 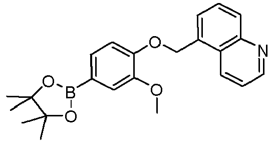 | 5-((2-methoxy-4-(4,4,5,5-tetramethyl-1,3,2-dioxaborolan-2-yl)phenoxy)methyl)quinoli ne | C23H26B NO4 | 391.20 | 392.3 |
| **131** | 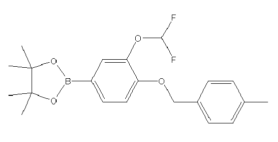 | 2-(3-(difluoromethoxy)-4-((4-methylbenzyl)oxy)phenyl)-4,4,5,5-tetramethyl-1,3,2-dioxaborolane | C21H25B F2O4 | 390.18 | 391.3 |
| **132** | 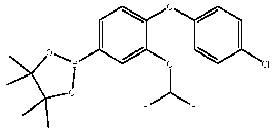 | 2-(4-(4-chlorophenoxy)-3-(difluoromethoxy) phenyl)-4,4,5,5-tetramethyl-1,3,2-dioxaborolane | C19H20B ClF2O4 | 396.11 | 397.4 |
| **133** | 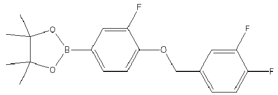 | 2-(4-((3,4-difluorobenzyl)oxy)-3-fluorophenyl)-4,4,5,5-tetramethyl-1,3,2-dioxaborolane | C19H20B F3O3 | 364.15 | 365.2 |
| **134** | 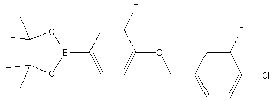 | 2-(4-((4-chloro-3-fluorobenzyl)oxy)-3-fluorophenyl)-4,4,5,5-tetramethyl-1,3,2-dioxaborolane | C19H20B ClF2O3 | 380.12 | 381.1 |
| **135** | 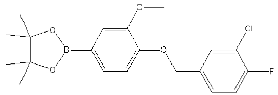 | 2-(4-((3-chloro-4-fluorobenzyl)oxy)-3-methoxyphenyl)-4,4,5,5-tetramethyl-1,3,2-dioxaborolane | C20H23B ClFO4 | 392.14 | 393.5 |
| **143** | 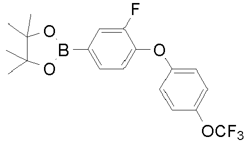 | 2-(3-fluoro-4-(4-(trifluoromethoxy)phenoxy )phenyl)-4,4,5,5-tetramethyl-1,3,2-dioxaborolane | C19H19B F4O4 | 398.13 | 399.2 |
| **144** | 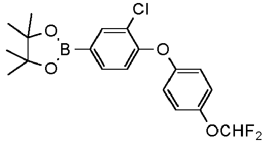 | 2-(3-chloro-4-(4-(difluoromethoxy)phenoxy) phenyl)-4,4,5,5-tetramethyl-1,3,2-dioxaborolane | C19H20B ClF2O4 | 396.11 | 397.2 |
| **145** | 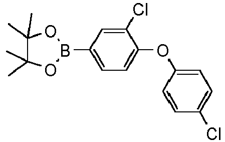 | 2-(3-chloro-4-(4-chlorophenoxy)phenyl)-4,4,5,5-tetramethyl-1,3,2-dioxaborolane | C18H19B Cl2O3 | 364.08 | 365.4 |
| **146** | 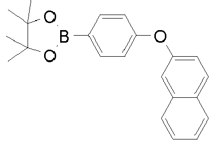 | 4,4,5,5-tetramethyl-2-(4-(naphthalen-2-yloxy)phenyl)-1,3,2-dioxaborolane | C22H23B O3 | 346.17 | 347.5 |

### Preparation of 8-(4-((4-(difluoromethyl)benzyl)oxy)-3-fluorophenyl)-7-methyl-3-(3,3,3-trifluoro-2-hydroxypropyl)-3,7-dihydro-1H-purine-2,6-dione 18

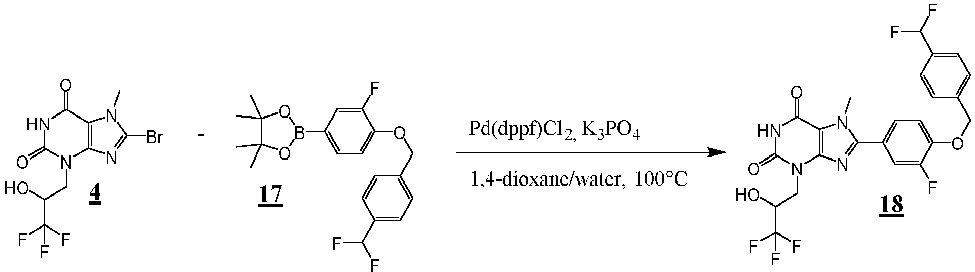

To 730 mg bromide **4,** 70 mg boronic ester **17,** 100 mg K₃PO₄ and 26.6 mg Pd(dppf)Cl₂ under nitrogen 3 ml 1,4-dioxane/water (5:1) were added and the mixture was heated to 100 °C until starting material was consumed. The mixture was absorbed on Celite, dried and purified by normal phase column chromatography (silica, cyclohexane/ethyl acetate gradient) to obtain compound **18.** LCMS (ESI+): found 529.3 [M+H]⁺, calculated 528.12 for C23H18F6N4O4.

### Preparation of 8-(4-(benzyloxy)-3-methoxyphenyl)-3-(3,3-difluoro-2-hydroxypropyl)-7-methyl-3,7-dihydro-1H-purine-2,6-dione 68

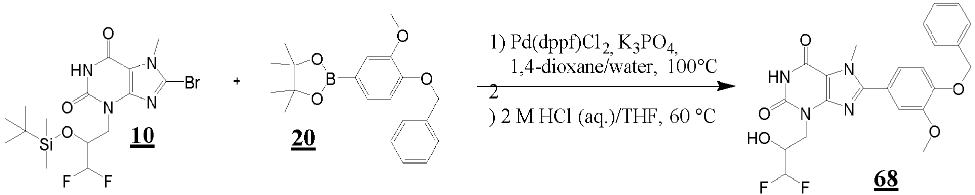

To 50 mg bromide **10,** 66 mg boronic ester **20,** 70 mg K₃PO₄ and 18.7 mg Pd(dppf)Cl₂ under nitrogen 3 ml 1,4-dioxane/water (5:1) were added and the mixture was heated to 100 °C until starting material was consumed. The mixture was absorbed on Celite, dried and purified by normal phase column chromatography (silica, cyclohexane/ethyl acetate gradient) to obtain the protected intermediate. The protecting group was removed by dissolving the intermediate in 1.5 ml THF/200 µl 2M aqueous hydrochloric acid and stirring the mixture at 60 °C overnight. The mixture was absorbed on Celite and purified by reversed phase column chromatography (RP18, water/acetonitrile gradient) to give compound **68.** LCMS (ESI+): found 473.3 [M+H]⁺, calculated 472.16 for C23H22F2N4O5.

Compounds in the following table **A-6** were synthesized as exemplified by compound **18, 68** or others using 8-bromoxanthine derivatives e.g. disclosed in tables **A-1** or **A-2** and boronic acids or esters e.g. commercially available or disclosed in table A-5 or made in analogy to the herein disclosed or to literature procedures.

**Table A-6:**

| **Cpd no** | **structure** | **name** | **formula** | **exact mass** | **[M+H] ⁺ found** |
|---|---|---|---|---|---|
| **18** | 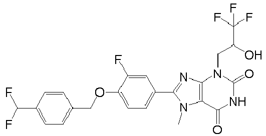 | 8-(4-((4-(difluoromethyl)benzyl)oxy)-3-fluorophenyl)-7-methyl-3-(3,3,3-trifluoro-2-hydroxypropyl)-3,7-dihydro-1H-purine-2,6-dione | C23H18F6N4O4 | 528.12 | 529.3 |
| **19** | 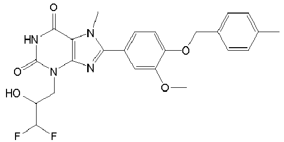 | 3-(3,3-difluoro-2-hydroxypropyl)-8-(3-methoxy-4-((4-methylbenzyl)oxy)phenyl)-7-methyl-3,7-dihydro-1H-purine-2,6-dione | C24H24F2N4O5 | 486.17 | 487.3 |
| **62** | 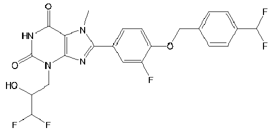 | 3-(3,3-difluoro-2-hydroxypropyl)-8-(4-((4-(difluoromethyl)benzyl)oxy)-3-fluorophenyl)-7-methyl-3,7-dihydro-1H-purine-2,6-dione | C23H19F5N4O4 | 510.13 | 511.3 |
| **63** | 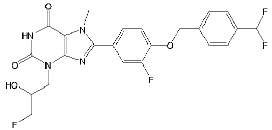 | 8-(4-((4-(difluoromethyl)benzyl)oxy)-3-fluorophenyl)-3-(3-fluoro-2-hydroxypropyl)-7-methyl-3,7-dihydro-1H-purine-2,6-dione | C23H20F4N4O4 | 492.14 | 493.3 |
| **64** | 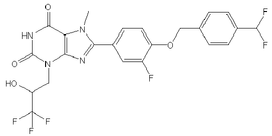 | 8-(4-((4-(difluoromethyl)benzyl)oxy)-3-fluorophenyl)-7-methyl-3-(3,3,3-trifluoro-2-hydroxypropyl)-3,7-dihydro-1H-purine-2,6-dione | C23H18F6N4O4 | 528.12 | 529.3 |
| **65** | 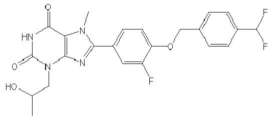 | 8-(4-((4-(difluoromethyl)benzyl)oxy)-3-fluorophenyl)-3-(2-hydroxypropyl)-7-methyl-3,7-dihydro-1H-purine-2,6-dione | C23H21F3N4O4 | 474.15 | 475.3 |
| **66** | 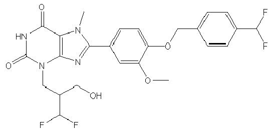 | 3-(3,3-difluoro-2-(hydroxymethyl)propyl)-8-(4-((4-(difluoromethyl)benzyl)oxy)-3-methoxyphenyl)-7-methyl-3,7-dihydro-1H-purine-2,6-dione | C25H24F4N4O5 | 536.17 | 537.3 |
| **67** | 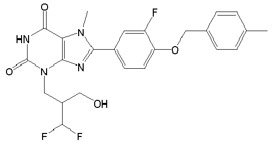 | 3-(3,3-difluoro-2-(hydroxymethyl)propyl)-8-(3-fluoro-4-((4-methylbenzyl)oxy)phenyl)-7-methyl-3,7-dihydro-1H-purine-2,6-dione | C24H23F3N4O4 | 488.17 | 489.3 |
| **68** | 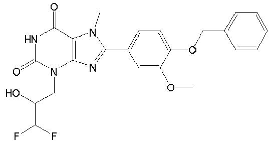 | 8-(4-(benzyloxy)-3-methoxyphenyl)-3-(3,3-difluoro-2-hydroxypropyl)-7-methyl-3,7-dihydro-1H-purine-2,6-dione | C23H22F2N4O5 | 472.16 | 473.3 |
| **72** | 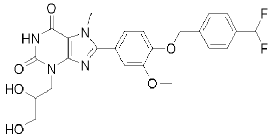 | 8-(4-((4-(difluoromethyl)benzyl)oxy)-3-methoxyphenyl)-3-(2,3-dihydroxypropyl)-7-methyl-3,7-dihydro-1H-purine-2,6-dione | C24H24F2N4O6 | 502.17 | 503.3 |
| **73** | 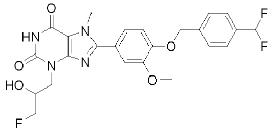 | 8-(4-((4-(difluoromethyl)benzyl)oxy)-3-methoxyphenyl)-3-(3-fluoro-2-hydroxypropyl)-7-methyl-3,7-dihydro-1H-purine-2,6-dione | C24H23F3N4O | 504.16 | 505.3 |
| **74** | 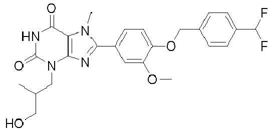 | 8-(4-((4-(difluoromethyl)benzyl)oxy)-3-methoxyphenyl)-3-(3-hydroxy-2-methylpropyl)-7-methyl-3,7-dihydro-1H-purine-2,6-dione | C25H26F2N4O5 | 500.19 | 501.3 |
| **75** | 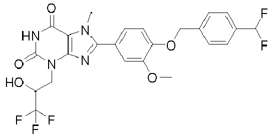 | 8-(4-((4-(difluoromethyl)benzyl)oxy)-3-methoxyphenyl)-7-methyl-3-(3,3,3-trifluoro-2-hydroxypropyl)-3,7-dihydro-1H-purine-2,6-dione | C24H21F5N4O5 | 540.14 | 541.3 |
| **76** | 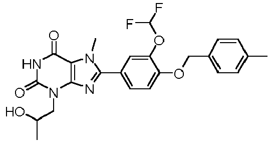 | 8-(3-(difluoromethoxy)-4-((4-methylbenzyl)oxy)phenyl)-3-(2-hydroxypropyl)-7-methyl-3,7-dihydro-1H-purine-2,6-dione | C24H24F2N4O5 | 486.17 | 487.3 |
| 77 | 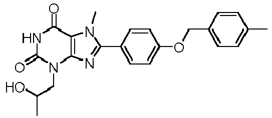 | 3-(2-hydroxypropyl)-7-methyl-8-(4-((4-methylbenzyl)oxy)phenyl)-3,7-dihydro-1H-purine-2,6-dione | C23H24N4O4 | 420.18 | 421.2 |
| **78** | 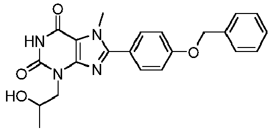 | 8-(4-(benzyloxy)phenyl)-3-(2-hydroxypropyl)-7-methyl-3,7-dihydro-1H-purine-2,6-dione | C22H22N4O4 | 406.16 | 407.3 |
| **79** | 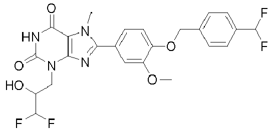 | 3-(3,3-difluoro-2-hydroxypropyl)-8-(4-((4-(difluoromethyl)benzyl)oxy)-3-methoxyphenyl)-7-methyl-3,7-dihydro-1H-purine-2,6-dione | C24H22F4N4O5 | 522.15 | 523.2 |
| **80** | 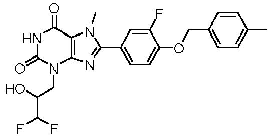 | 3-(3,3-difluoro-2-hydroxypropyl)-8-(3-fluoro-4-((4-methylbenzyl)oxy)phenyl)-7-methyl-3,7-dihydro-1H-purine-2,6-dione | C23H21F3N4O4 | 474.15 | 475.3 |
| **81** | 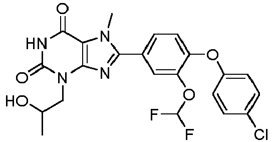 | 8-(4-(4-chlorophenoxy)-3-(difluoromethoxy)phenyl)-3-(2-hydroxypropyl)-7-methyl-3,7-dihydro-1H-purine-2,6-dione | C22H19CIF2N4O5 | 492.10 | 493.2 |
| **82** | 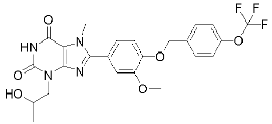 | 3-(2-hydroxypropyl)-8-(3-methoxy-4-((4-(trifluoromethoxy)benzyl)oxy)pheny l)-7-methyl-3,7-dihydro-1H-purine-2,6-dione | C24H23F3N4O6 | 520.16 | 521.4 |
| **83** | 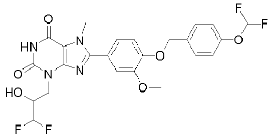 | 3-(3,3-difluoro-2-hydroxypropyl)-8-(4-((4-(difluoromethoxy)benzyl)oxy)-3-methoxyphenyl)-7-methyl-3,7-dihydro-1H-purine-2,6-dione | C24H22F4N4O6 | 538.15 | 539.4 |
| **84** | 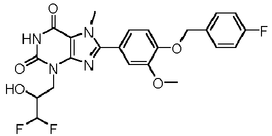 | 3-(3,3-difluoro-2-hydroxypropyl)-8-(4-((4-fluorobenzyl)oxy)-3-methoxyphenyl)-7-methyl-3,7-dihydro-1H-purine-2,6-dione | C23H21F3N4O5 | 490.15 | 491.4 |
| **85** | 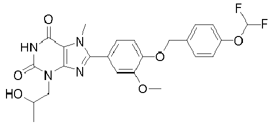 | 8-(4-((4-(difluoromethoxy)benzyl)oxy)-3-methoxyphenyl)-3-(2-hydroxypropyl)-7-methyl-3,7-dihydro-1H-purine-2,6-dione | C24H24F2N4O6 | 502.17 | 503.2 |
| **86** | 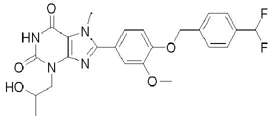 | 8-(4-((4-(difluoromethyl)benzyl)oxy)-3-methoxyphenyl)-3-(2-hydroxypropyl)-7-methyl-3,7-dihydro-1H-purine-2,6-dione | C24H24F2N4O5 | 486.17 | 487.2 |
| **87** | 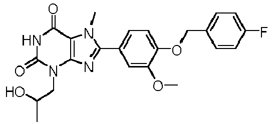 | 8-(4-((4-fluorobenzyl)oxy)-3-methoxyphenyl)-3-(2-hydroxypropyl)-7-methyl-3,7-dihydro-1H-purine-2,6-dione | C23H23FN4O5 | 454.17 | 455.2 |
| **88** | 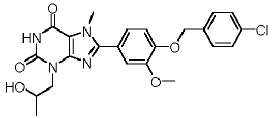 | 8-(4-((4-chlorobenzyl)oxy)-3-methoxyphenyl)-3-(2-hydroxypropyl)-7-methyl-3,7-dihydro-1H-purine-2,6-dione | C23H23CIN4O5 | 470.14 | 471.2 |
| **89** | 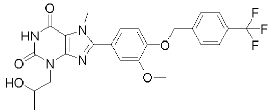 | 3-(2-hydroxypropyl)-8-(3-methoxy-4-((4-(trifluoromethyl)benzyl)oxy)phenyl)-7-methyl-3,7-dihydro-1H-purine-2,6-dione | C24H23F3N4O5 | 504.16 | 505.2 |
| **90** | 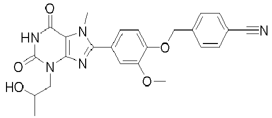 | 4-((4-(3-(2-hydroxypropyl)-7-methyl-2,6-dioxo-2,3,6,7-tetrahydro-1H-purin-8-yl)-2-methoxyphenoxy)methyl)benzonitril e | C24H23N5O5 | 461.17 | 462.2 |
| **91** | 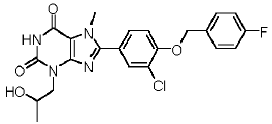 | 8-(3-chloro-4-((4-fluorobenzyl)oxy)phenyl)-3-(2-hydroxypropyl)-7-methyl-3,7-dihydro-1H-purine-2,6-dione | C22H20CIFN4O4 | 458.12 | 459.2 |
| **92** | 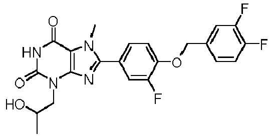 | 8-(4-((3,4-difluorobenzyl)oxy)-3-fluorophenyl)-3-(2-hydroxypropyl)-7-methyl-3,7-dihydro-1H-purine-2,6-dione | C22H19F3N4O4 | 460.14 | 461.4 |
| **93** | 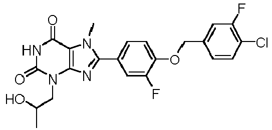 | 8-(4-((4-chloro-3-fluorobenzyl)oxy)-3-fluorophenyl)-3-(2-hydroxypropyl)-7-methyl-3,7-dihydro-1H-purine-2,6-dione | C22H19CIF2N4O4 | 476.11 | 477.2 |
| **94** | 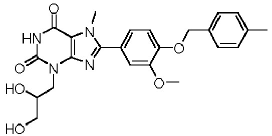 | 3-(2,3-dihydroxypropyl)-8-(3-methoxy-4-((4-methylbenzyl)oxy)phenyl)-7-methyl-3,7-dihydro-1H-purine-2,6-dione | C24H26N4O6 | 466.19 | 467.2 |
| **95** | 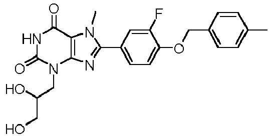 | 3-(2,3-dihydroxypropyl)-8-(3-fluoro-4-((4-methylbenzyl)oxy)phenyl)-7-methyl-3,7-dihydro-1H-purine-2,6-dione | C23H23FN4O5 | 454.17 | 455.2 |
| **96** | 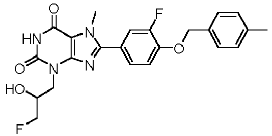 | 3-(3-fluoro-2-hydroxypropyl)-8-(3-fluoro-4-((4-methylbenzyl)oxy)phenyl)-7-methyl-3,7-dihydro-1H-purine-2,6-dione | C23H22F2N4O4 | 456.16 | 457.2 |
| **97** | 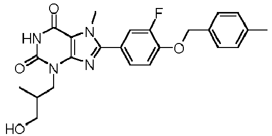 | 8-(3-fluoro-4-((4-methylbenzyl)oxy)phenyl)-3-(3-hydroxy-2-methylpropyl)-7-methyl-3,7-dihydro-1H-purine-2,6-dione | C24H25FN4O4 | 452.19 | 453.2 |
| **98** | 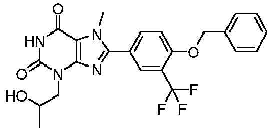 | 8-(4-(benzyloxy)-3-(trifluoromethyl)phenyl)-3-(2-hydroxypropyl)-7-methyl-3,7-dihydro-1H-purine-2,6-dione | C23H21F3N4O4 | 474.15 | 475.2 |
| **99** | 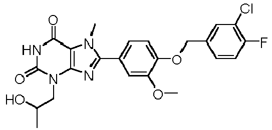 | 8-(4-((3-chloro-4-fluorobenzyl)oxy)-3-methoxyphenyl)-3-(2-hydroxypropyl)-7-methyl-3,7-dihydro-1H-purine-2,6-dione | C23H22CIFN4O5 | 488.13 | 489.2 |
| **100** | 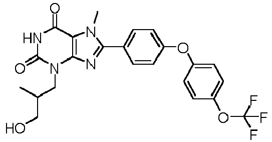 | 3-(3-hydroxy-2-methylpropyl)-7-methyl-8-(4-(4-(trifluoromethoxy)phenoxy)phenyl)-3,7-dihydro-1H-purine-2,6-dione | C23H21F3N4O5 | 490.15 | 491.2 |
| **101** | 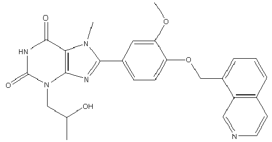 | 3-(2-hydroxypropyl)-8-(4-(isoquinolin-8-ylmethoxy)-3-methoxyphenyl)-7-methyl-3,7-dihydro-1H-purine-2,6-dione | C26H25N5O5 | 487.19 | 488.2 |
| **102** | 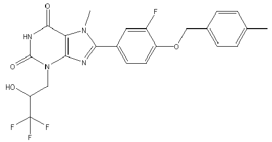 | 8-(3-fluoro-4-((4-methylbenzyl)oxy)phenyl)-7-methyl-3-(3,3,3-trifluoro-2-hydroxypropyl)-3,7-dihydro-1H-purine-2,6-dione | C23H20F4N4O4 | 492.14 | 493.2 |
| **103** | 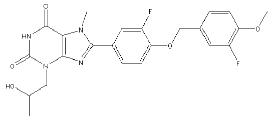 | 8-(3-fluoro-4-((3-fluoro-4-methoxybenzyl)oxy)phenyl)-3-(2-hydroxypropyl)-7-methyl-3,7-dihydro-1H-purine-2,6-dione | C23H22F2N4O5 | 472.16 | 473.2 |
| **113** | 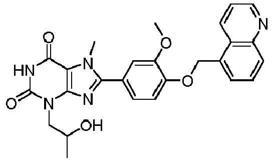 | 3-(2-hydroxypropyl)-8-(3-methoxy-4-(quinolin-5-ylmethoxy)phenyl)-7-methyl-3,7-dihydro-1H-purine-2,6-dione | C26H25N5O5 | 487.19 | 488 |
| **114** | 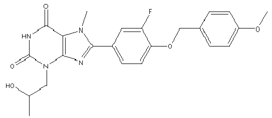 | 8-(3-fluoro-4-((4-methoxybenzyl)oxy)phenyl)-3-(2-hydroxypropyl)-7-methyl-3,7-dihydro-1H-purine-2,6-dione | C23H23FN4O5 | 454.17 | 455.3 |
| **115** | 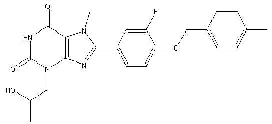 | 8-(3-fluoro-4-((4-methylbenzyl)oxy)phenyl)-3-(2-hydroxypropyl)-7-methyl-3,7-dihydro-1H-purine-2,6-dione | C23H23FN4O4 | 438.17 | 439.2 |
| **116** | 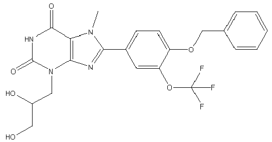 | 8-(4-(benzyloxy)-3-(trifluoromethoxy)phenyl)-3-(2,3-dihydroxypropyl)-7-methyl-3,7-dihydro-1H-purine-2,6-dione | C23H21F3N4O6 | 506.14 | 507.4 |
| **117** | 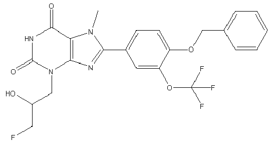 | 8-(4-(benzyloxy)-3-(trifluoromethoxy)phenyl)-3-(3-fluoro-2-hydroxypropyl)-7-methyl-3,7-dihydro-1H-purine-2,6-dione | C23H20F4N4O5 | 508.14 | 509.2 |
| **118** | 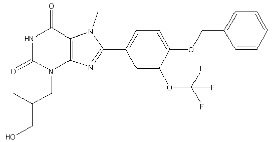 | 8-(4-(benzyloxy)-3-(trifluoromethoxy)phenyl)-3-(3-hydroxy-2-methylpropyl)-7-methyl-3,7-dihydro-1H-purine-2,6-dione | C24H23F3N4O5 | 504.16 | 505.3 |
| **119** | 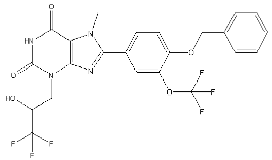 | 8-(4-(benzyloxy)-3-(trifluoromethoxy)phenyl)-7-methyl-3-(3,3,3-trifluoro-2-hydroxypropyl)-3,7-dihydro-1H-purine-2,6-dione | C23H18F6N4O5 | 544.12 | 545.2 |
| **120** | 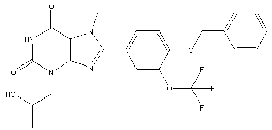 | 8-(4-(benzyloxy)-3-(trifluoromethoxy)phenyl)-3-(2-hydroxypropyl)-7-methyl-3,7-dihydro-1H-purine-2,6-dione | C23H21F3N4O5 | 490.15 | 491.2 |
| **121** | 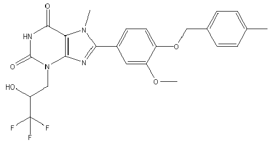 | 8-(3-methoxy-4-((4-methylbenzyl)oxy)phenyl)-7-methyl-3-(3,3,3-trifluoro-2-hydroxypropyl)-3,7-dihydro-1H-purine-2,6-dione | C24H23F3N4O5 | 504.16 | 505.5 |
| **122** | 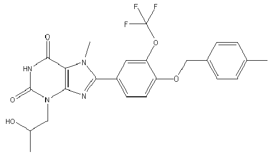 | 3-(2-hydroxypropyl)-7-methyl-8-(4-((4-methylbenzyl)oxy)-3-(trifluoromethoxy)phenyl)-3, 7-dihydro-1H-purine-2,6-dione | C24H23F3N4O5 | 504.16 | 505.2 |
| **123** | 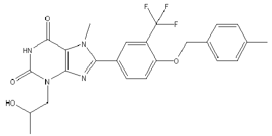 | 3-(2-hydroxypropyl)-7-methyl-8-(4-((4-methylbenzyl)oxy)-3-(trifluoromethyl)phenyl)-3,7-dihydro-1H-purine-2,6-dione | C24H23F3N4O4 | 488.17 | 489.1 |
| **124** | 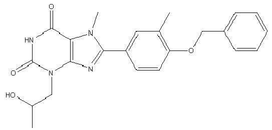 | 8-(4-(benzyloxy)-3-methylphenyl)-3-(2-hydroxypropyl)-7-methyl-3,7-dihydro-1H-purine-2,6-dione | C23H24N4O4 | 420.18 | 421.2 |
| **125** | 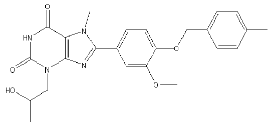 | 3-(2-hydroxypropyl)-8-(3-methoxy-4-((4-methylbenzyl)oxy)phenyl)-7-methyl-3,7-dihydro-1H-purine-2,6-dione | C24H26N4O5 | 450.19 | 451.2 |
| **126** | 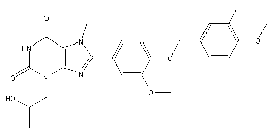 | 8-(4-((3-fluoro-4-methoxybenzyl)oxy)-3-methoxyphenyl)-3-(2-hydroxypropyl)-7-methyl-3,7-dihydro-1H-purine-2,6-dione | C24H25FN4O6 | 484.18 | 485.3 |
| **127** | 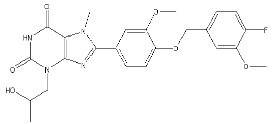 | 8-(4-((4-fluoro-3-methoxybenzyl)oxy)-3-methoxyphenyl)-3-(2-hydroxypropyl)-7-methyl-3,7-dihydro-1H-purine-2,6-dione | C24H25FN4O6 | 484.18 | 485 |
| **128** | 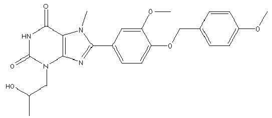 | 3-(2-hydroxypropyl)-8-(3-methoxy-4-((4-methoxybenzyl)oxy)phenyl)-7-methyl-3,7-dihydro-1H-purine-2,6-dione | C24H26N4O6 | 466.19 | 467.2 |
| **136** | 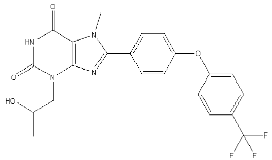 | 3-(2-hydroxypropyl)-7-methyl-8-(4-(4-(trifluoromethyl)phenoxy)phenyl)-3,7-dihydro-1H-purine-2,6-dione | C22H19F3N4O4 | 460.14 | 461.4 |
| **137** | 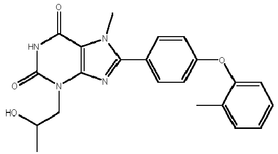 | 3-(2-hydroxypropyl)-7-methyl-8-(4-(o-tolyloxy)phenyl)-3,7-dihydro-1H-purine-2,6-dione | C22H22N4O4 | 406.16 | 407.3 |
| **138** | 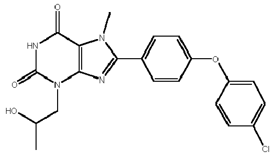 | 8-(4-(4-chlorophenoxy)phenyl)-3-(2-hydroxypropyl)-7-methyl-3,7-dihydro-1H-purine-2,6-dione | C21H19CIN4O4 | 426.11 | 427.2 |
| **139** | 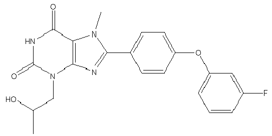 | 8-(4-(3-fluorophenoxy)phenyl)-3-(2-hydroxypropyl)-7-methyl-3,7-dihydro-1H-purine-2,6-dione | C21H19FN4O4 | 410.14 | 411.2 |
| **140** | 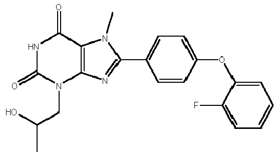 | 8-(4-(2-fluorophenoxy)phenyl)-3-(2-hydroxypropyl)-7-methyl-3,7-dihydro-1H-purine-2,6-dione | C21H19FN4O4 | 410.14 | 411.2 |
| **141** | 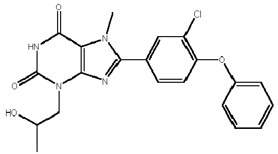 | 8-(3-chloro-4-phenoxyphenyl)-3-(2-hydroxypropyl)-7-methyl-3,7-dihydro-1H-purine-2,6-dione | C21H19CIN4O4 | 426.11 | 427.2 |
| **142** | 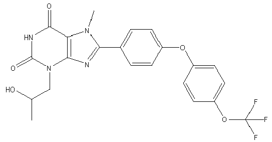 | 3-(2-hydroxypropyl)-7-methyl-8-(4-(4-(trifluoromethoxy)phenoxy)phenyl)-3,7-dihydro-1H-purine-2,6-dione | C22H19F3N4O5 | 476.13 | 477.2 |
| **147** | 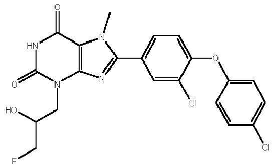 | 8-(3-chloro-4-(4-chlorophenoxy)phenyl)-3-(3-fluoro-2-hydroxypropyl)-7-methyl-3,7-dihydro-1H-purine-2,6-dione | C21H17Cl2FN4O4 | 478.06 | 479.1 |
| **148** | 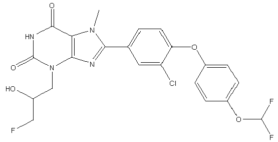 | 8-(3-chloro-4-(4-(difluoromethoxy)phenoxy)phenyl)-3-(3-fluoro-2-hydroxypropyl)-7-methyl-3,7-dihydro-1H-purine-2,6-dione | C22H18CIF3N4O5 | 510.09 | 511.2 |
| **149** | 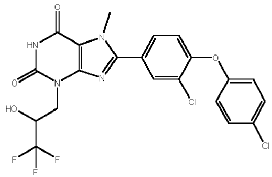 | 3-(3-fluoro-2-hydroxypropyl)-8-(3-fluoro-4-(4-(trifluoromethoxy)phenoxy)phenyl)-7-methyl-3,7-dihydro-1H-purine-2,6-dione | C22H17F5N4O5 | 512.11 | 513.2 |
| **150** | 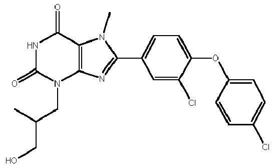 | 8-(3-chloro-4-(4-chlorophenoxy)phenyl)-3-(3-hydroxy-2-methylpropyl)-7-methyl-3,7-dihydro-1H-purine-2,6-dione | C22H20Cl2N4O4 | 474.09 | 475.1 |
| **151** | 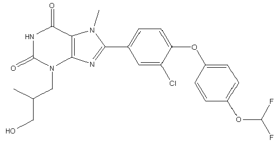 | 8-(3-chloro-4-(4-(difluoromethoxy)phenoxy)phenyl)-3-(3-hydroxy-2-methylpropyl)-7-methyl-3,7-dihydro-1H-purine-2,6-dione | C23H21CIF2N4O5 | 506.12 | 507.2 |
| **152** | 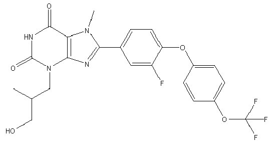 | 8-(3-fluoro-4-(4-(trifluoromethoxy)phenoxy)phenyl)-3-(3-hydroxy-2-methylpropyl)-7-methyl-3,7-dihydro-1H-purine-2,6-dione | C23H20F4N4O5 | 508.14 | 509.2 |
| **153** | 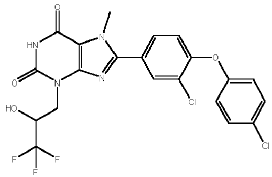 | 8-(3-chloro-4-(4-chlorophenoxy)phenyl)-7-methyl-3-(3,3,3-trifluoro-2-hydroxypropyl)-3,7-dihydro-1H-purine-2,6-dione | C21H15Cl2F3N4O4 | 514.04 | 515.1 |
| **198** | 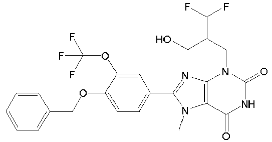 | 8-(4-(benzyloxy)-3-(trifluoromethoxy)phenyl)-3-(3,3-difluoro-2-(hydroxymethyl)propyl)-7-methyl-3,7-dihydro-1H-purine-2,6-dione | C24H21F5N4O5 | 540.14 | 541.3 |
| **199** | 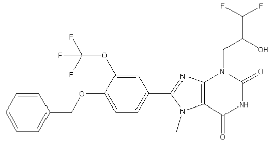 | 8-(4-(benzyloxy)-3-(trifluoromethoxy)phenyl)-3-(3,3-difluoro-2-hydroxypropyl)-7-methyl-3,7-dihydro-1H-purine-2,6-dione | C23H19F5N4O5 | 526.13 | 527.3 |

### Preparation of 8-bromo-3-(2-((tert-butyldimethylsilyl)oxy)propyl)-7-methyl-1 -((2-(trimethylsilyl)ethoxy)methyl)-3,7-dihydro-1H-purine-2,6-dione 42

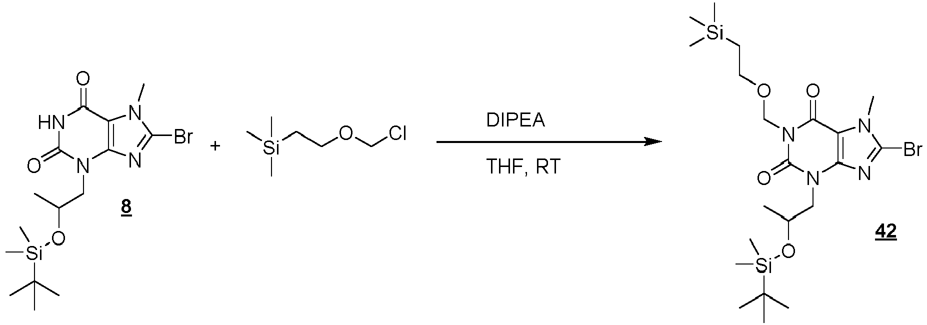

3.0 g compound **8** and 3.74 ml DIPEA were dissolved in 20 ml THF. 2.55 ml SEMCI were added at room temperature und the reaction mixture was stirred overnight. Additional SEMCI (0.5 ml) and DIPEA (1 ml) were added and the reaction was stirred at room temperature overnight again. Ethyl acetate was added and the organic phase was washed with saturated NaHCO₃ solution, dried over MgSO₄ and volatiles were removed under reduced pressure. The crude was pure enough to be used in the next reactions without further purification. LCMS (ESI+): found 547.3 [M+H]⁺, calculated 546.17 for C21H39BrN4O4Si2.

### Preparation of 3-(2-((tert-butyldimethylsilyl)oxy)propyl)-8-(3-fluoro-4-hydroxyphenyl)-7-methyl-1-((2-(trimethylsilyl)ethoxy)methyl)-3,7-dihydro-1H-purine-2,6-dione 43

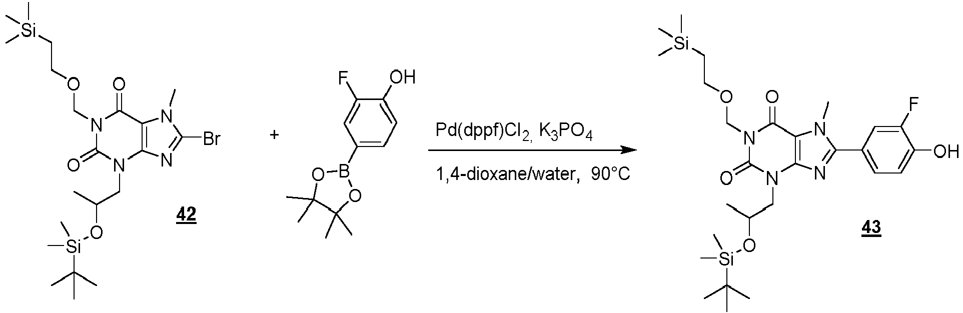

2.5 g bromide **42,** 1.3 g 2-fluoro-4-(4,4,5,5-tetramethyl-1,3,2-dioxaborolan-2-yl)phenol, 0.39 g Pd(dppf)Cl₂*DCM and 2.91 g K₃PO₄ under an inert atmosphere in 30 ml 1,4-dioxane/water (4:1) were stirred at 90 °C overnight. The mixture was concentrated under reduced pressure and absorbed on Celite. Purification was achieved by normal phase column chromatography (silica, cyclohexane/ethyl acetate gradient) to yield phenol **43.** LCMS (ESI+): found 579.4 [M+H]⁺, calculated 578.28 for C27H43FN4O5Si2.

### Preparation of 8-(4-(benzyloxy)-3-fluorophenyl)-3-(2-hydroxypropyl)-7-methyl-3,7-dihydro-1H-purine-2,6-dione 44

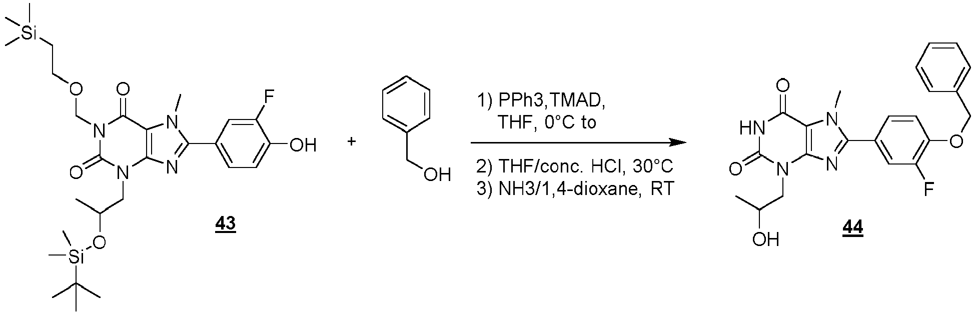

30 mg phenol **43** and 12 mg benzyl alcohol were dissolved in 1.5 ml THF and dried over 4 Å molecular sieves. This solution was then transferred to 30 mg triphenylphosphine in 0.5 ml THF under a nitrogen atmosphere at 0°C. After 30 min TMAD was added and after additional 30 min at 0°c the mixture was heated to 55 °C overnight. The mixture was absorbed on Celite and purified via normal phase column chromatography (silica, cyclohexane/ethyl acetate/methanol gradient) to give the protected intermediate. To remove both protecting groups the crude was dissolved in 750 µl THF and 750 µl concentrated aqueous hydrochloric acid were added. After stirring at 30 °C volatiles were removed and the crude was dissolved in 2 ml ammonia (0.5 M solution in dry 1,4-dioxane) at room temperature. After 5 min volatiles were removed again, the crude dissolved in some DMSO and directly purified by reversed-phase HPLC (C18 column, water (0.1 %TFA) / acetonitrile (0.1 %TFA) gradient). The desired fractions were lyophilized to yield title compound **44.** LCMS (ESI+): found 425.3 [M+H]⁺, calculated 424.15 for C22H21FN4O4.

### Preparation of 8-(4-(4-chlorophenoxy)-3-fluorophenyl)-3-(2-hydroxypropyl)-7-methyl-3,7-dihydro-1H-purine-2,6-dione 197

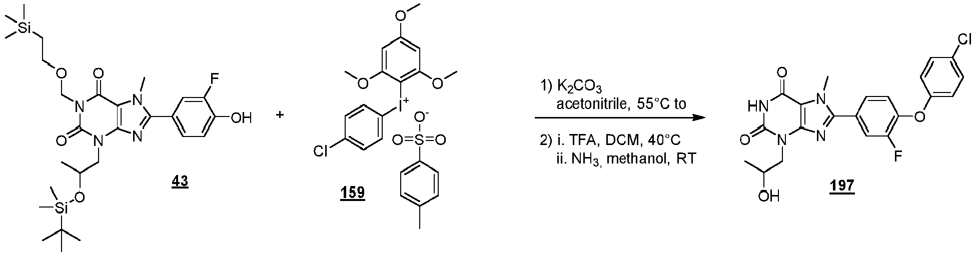

130 mg phenol **43,** 218 mg iodonium salt **159** and 62 mg K₂CO₃ in 4 ml acetonitrile were stirred at 55 °C until full conversion of the phenol. The mixture was absorbed on Celite and purified by normal phase column chromatography (silica, cyclohexane/ethyl acetate gradient). The protected intermediate was dissolved in 3 ml DCM and 500 µl TFA and stirred at 40 °C overnight. Volatiles were removed and the crude was dissolved in 3 ml ammonia (7 M solution in methanol) at room temperature. After 5 min volatiles were removed again, the crude dissolved in some DMSO and directly purified by reversed-phase HPLC (C18 column, water (0.1 %TFA) / acetonitrile (0.1 %TFA) gradient). The desired fractions were lyophilized to yield title compound **197.** LCMS (ESI+): found 445.4 [M+H]⁺, calculated 444.1 for C21H18ClFN4O4.

### Preparation of 3-(2-((tert-butyldimethylsilyl)oxy)propyl)-8-(4-hydroxy-3-(trifluoromethyl)phenyl)-7-methyl-3,7-dihydro-1H-purine-2,6-dione 154

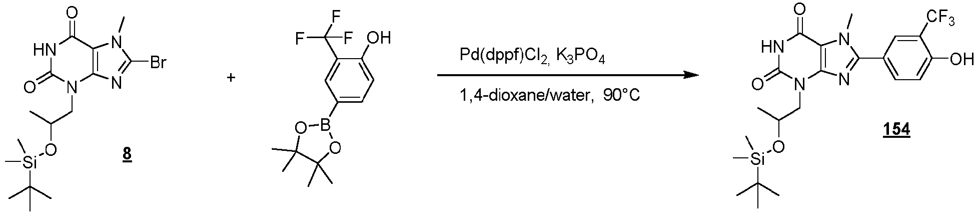

100 mg bromide **8,** 104 mg 4-(4,4,5,5-tetramethyl-1,3,2-dioxaborolan-2-yl)-2-(trifluoromethyl)phenol, 30.4 mg Pd(dppf)₂Cl*DCM and 153 mg K₃PO₄ under inert atmosphere were mixed with 2.5 ml 1,4-dioxane/water (4:1) and stirred at 90 °C overnight. The mixture was concentrated under reduced pressure and absorbed on Celite. Purification was achieved by normal phase column chromatography (silica, cyclohexane/ethyl acetate gradient) to yield phenol **154.** LCMS (ESI+): found 499.2 [M+H]⁺, calculated 498.19 for C22H29F3N4O4Si.

### Preparation of 3-(2-hydroxypropyl)-7-methyl-8-(3-(trifluoromethoxy)-4-(4-(trifluoromethoxy)phenoxy)phenyl)-3,7-dihydro-1H-purine-2,6-dione 155

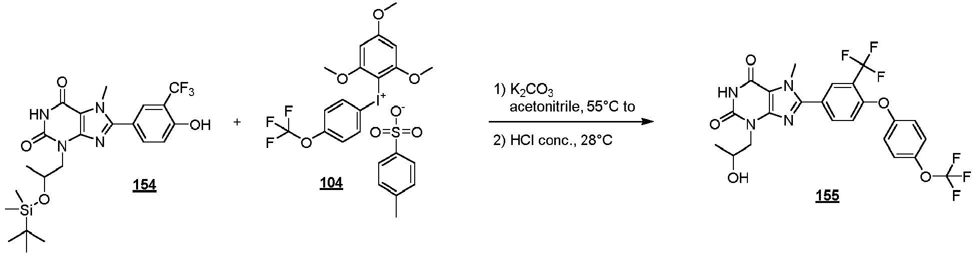

100 mg phenol **154,** 138 mg iodonium salt **104** and 111 mg K₂CO₃ in 4 ml acetonitrile were stirred at 55 °C until full conversion of the phenol. The mixture was absorbed on Celite and purified by normal phase column chromatography (silica, cyclohexane/ethyl acetate/methanol gradient). The protected intermediate was dissolved in 2 ml THF and 500 µl concentrated hydrochloric acid and stirred at 28 °C overnight. Purification was achieved by reversed-phase HPLC (C18 column, water (0.1 %TFA) / acetonitrile (0.1 %TFA) gradient). The desired fractions were lyophilized to yield title compound **155.** LCMS (ESI+): found 545.2 [M+H]⁺, calculated 544.12 for C23H18F6N4O5.

### Preparation of 3-(2-((tert-butyldimethylsilyl)oxy)propyl)-8-(4-hydroxy-3-(trifluoromethyl)phenyl)-7-methyl-3,7-dihydro-1H-purine-2,6-dione 157

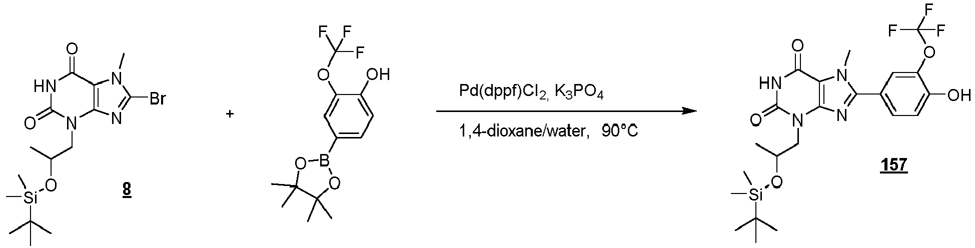

100 mg bromide **8,** 104 mg 4-(4,4,5,5-tetramethyl-1,3,2-dioxaborolan-2-yl)-2-(trifluoromethoxy)phenol, 30.4 mg Pd(dppf)Cl₂*DCM and 153 mg K₃PO₄ under inert atmosphere were mixed with 2.5 ml 1,4-dioxane/water (4:1) and stirred at 90 °C overnight. The mixture was concentrated under reduced pressure and absorbed on Celite. Purification was achieved by normal phase column chromatography (silica, cyclohexane/ethyl acetate gradient) to yield phenol **157.** LCMS (ESI+): found 515.5 [M+H]⁺, calculated 514.19 for C22H29F3N4OOSi.

### Preparation of 3-(2-hydroxypropyl)-7-methyl-8-(3-(trifluoromethoxy)-4-(4-(trifluoromethoxy)phenoxy)phenyl)-3,7-dihydro-1H-purine-2,6-dione 156

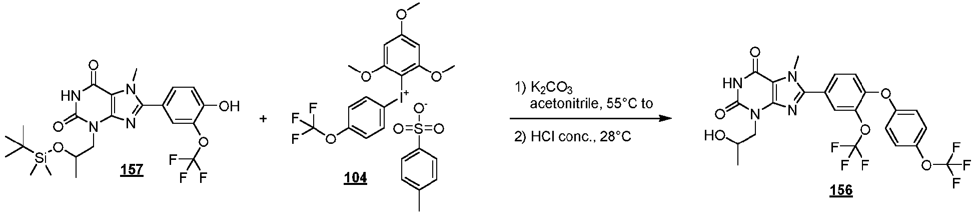

100 mg phenol **157,** 138 mg iodonium salt **104** and 111 mg K₂CO₃ in 4 ml acetonitrile were stirred at 55 °C until full conversion of the phenol. The mixture was absorbed on Celite and purified by normal phase column chromatography (silica, cyclohexane/ethyl acetate/methanol gradient). The protected intermediate was dissolved in 2 ml THF and 500 µl concentrated hydrochloric acid and stirred at 28 °C overnight. Purification was achieved by reversed-phase HPLC (C18 column, water (0.1 %TFA) / acetonitrile (0.1 %TFA) gradient). The desired fractions were lyophilized to yield title compound **156.** LCMS (ESI+): found 561.1 [M+H]⁺, calculated 560.11 for C23H18F6N4O6.

Compounds in the following table **A-7** were synthesized as exemplified by compound **44, 155, 156, 197** or others.

**Table A-7:**

| **Cpd no** | **structure** | **name** | **formula** | **exact mass** | **[M+H]⁺ found** |
|---|---|---|---|---|---|
| **44** | 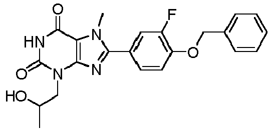 | 8-(4-(benzyloxy)-3-fluorophenyl)-3-(2-hydroxypropyl)-7-methyl-3,7-dihydro-1H-purine-2,6-dione | C22H21F N4O4 | 424.15 | 425.3 |
| **155** | 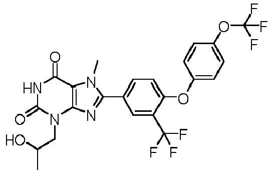 | 3-(2-hydroxypropyl)-7-methyl-8-(4-(4-(trifluoromethoxy)phenoxy )-3-(trifluoromethyl)phenyl)-3,7-dihydro-1H-purine-2,6-dione | C23H18F 6N4O5 | 544.12 | 545.2 |
| **156** | 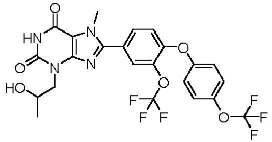 | 3-(2-hydroxypropyl)-7-methyl-8-(3-(trifluoromethoxy)-4-(4-(trifluoromethoxy)phenoxy )phenyl)-3,7-dihydro-1H-purine-2,6-dione | C23H18F 6N4O6 | 560.11 | 561.1 |
| **161** | 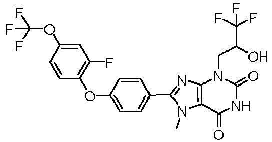 | 8-(4-(2-fluoro-4-(trifluoromethoxy)phenoxy )phenyl)-7-methyl-3-(3,3,3-trifluoro-2-hydroxypropyl)-3,7-dihydro-1H-purine-2,6-dione | C22H15F 7N4O5 | 548.09 | 549.2 |
| **162** | 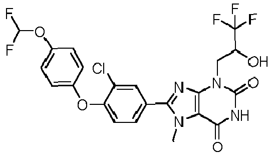 | 8-(3-chloro-4-(4-(difluoromethoxy)phenoxy )phenyl)-7-methyl-3-(3,3,3-trifluoro-2-hydroxypropyl)-3,7-dihydro-1H-purine-2,6-dione | C22H16 ClF5N4O 5 | 546.07 | 547.2 |
| **163** | 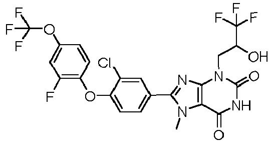 | 8-(3-chloro-4-(2-fluoro-4-(trifluoromethoxy)phenoxy )phenyl)-7-methyl-3-(3,3,3-trifluoro-2-hydroxypropyl)-3,7-dihydro-1H-purine-2,6-dione | C22H14 ClF7N4O 5 | 582.05 | 583.1 |
| **164** | 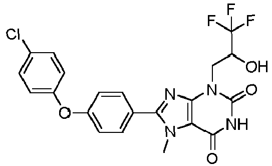 | 8-(4-(4-chlorophenoxy)phenyl)-7-methyl-3-(3,3,3-trifluoro-2-hydroxypropyl)-3,7-dihydro-1H-purine-2,6-dione | C21H16 ClF3N4O 4 | 480.08 | 481.1 |
| **165** | 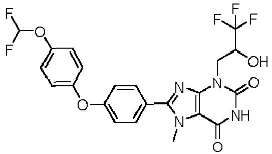 | 8-(4-(4-(difluoromethoxy)phenoxy )phenyl)-7-methyl-3-(3,3,3-trifluoro-2-hydroxypropyl)-3,7-dihydro-1H-purine-2,6-dione | C22H17F 5N4O5 | 512.11 | 513.2 |
| **166** | 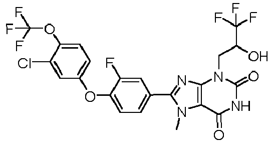 | 8-(4-(3-chloro-4-(trifluoromethoxy)phenoxy )-3-fluorophenyl)-7-methyl-3-(3,3,3-trifluoro-2-hydroxypropyl)-3,7-dihydro-1H-purine-2,6-dione | C22H14 ClF7N4O 5 | 582.05 | 583.3 |
| **167** | 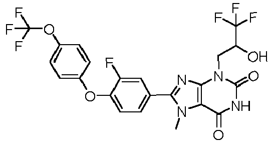 | 8-(3-fluoro-4-(4-(trifluoromethoxy)phenoxy )phenyl)-7-methyl-3-(3,3,3-trifluoro-2-hydroxypropyl)-3,7-dihydro-1H-purine-2,6-dione | C22H15F 7N4O5 | 548.09 | 549.2 |
| **176** | 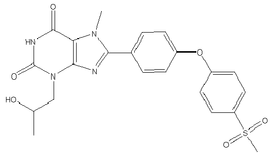 | 3-(2-hydroxypropyl)-7-methyl-8-(4-(4-(methylsulfonyl)phenoxy)p henyl)-3,7-dihydro-1H-purine-2,6-dione | C22H22 N4O6S | 470.13 | 471.2 |
| **177** | 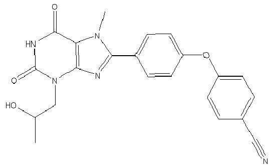 | 4-(4-(3-(2-hydroxypropyl)-7-methyl-2,6-dioxo-2,3,6,7-tetrahydro-1H-purin-8-yl)phenoxy)benzonitrile | C22H19 N5O4 | 417.14 | 418.2 |
| **178** | 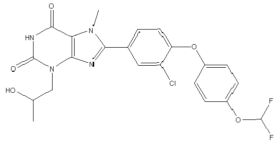 | 8-(3-chloro-4-(4-(difluoromethoxy)phenoxy )phenyl)-3-(2-hydroxypropyl)-7-methyl-3,7-dihydro-1H-purine-2,6-dione | C22H19 ClF2N4O 5 | 492.10 | 493.2 |
| **179** | 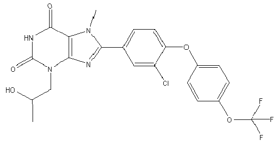 | 8-(3-chloro-4-(4-(trifluoromethoxy)phenoxy )phenyl)-3-(2-hydroxypropyl)-7-methyl-3,7-dihydro-1H-purine-2,6-dione | C22H18 ClF3N4O 5 | 510.09 | 511.1 |
| **180** | 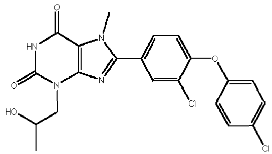 | 8-(3-chloro-4-(4-chlorophenoxy)phenyl)-3-(2-hydroxypropyl)-7-methyl-3,7-dihydro-1H-purine-2,6-dione | C21H18 Cl2N404 | 460.07 | 461.1 |
| **181** | 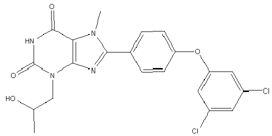 | 8-(4-(3,5-dichlorophenoxy)phenyl)-3-(2-hydroxypropyl)-7-methyl-3,7-dihydro-1H-purine-2,6-dione | C21H18 Cl2N4O4 | 460.07 | 461 |
| **182** | 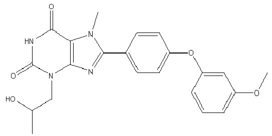 | 3-(2-hydroxypropyl)-8-(4-(3-methoxyphenoxy)phenyl)-7-methyl-3,7-dihydro-1H-purine-2,6-dione | C22H22 N4O5 | 422.16 | 423.3 |
| **183** | 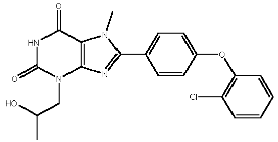 | 8-(4-(2-chlorophenoxy)phenyl)-3-(2-hydroxypropyl)-7-methyl-3,7-dihydro-1H-purine-2,6-dione | C21H19 ClN4O4 | 426.11 | 427.3 |
| **184** | 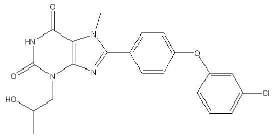 | 8-(4-(3-chlorophenoxy)phenyl)-3-(2-hydroxypropyl)-7-methyl-3,7-dihydro-1H-purine-2,6-dione | C21H19 ClN4O4 | 426.11 | 427.2 |
| **185** | 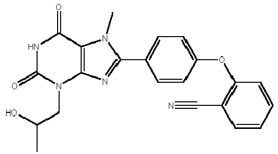 | 2-(4-(3-(2-hydroxypropyl)-7-methyl-2,6-dioxo-2,3,6,7-tetrahydro-1H-purin-8-yl)phenoxy)benzonitrile | C22H19 N5O4 | 417.14 | 418.2 |
| **186** | 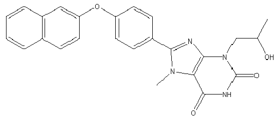 | 3-(2-hydroxypropyl)-7-methyl-8-(4-(naphthalen-2-yloxy)phenyl)-3,7-dihydro-1H-purine-2,6-dione | C25H22 N4O4 | 442.16 | 443.4 |
| **187** | 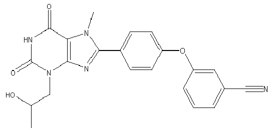 | 3-(4-(3-(2-hydroxypropyl)-7-methyl-2,6-dioxo-2,3,6,7-tetrahydro-1H-purin-8-yl)phenoxy)benzonitrile | C22H19 N5O4 | 417.14 | 418.2 |
| **188** | 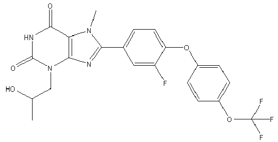 | 8-(3-fluoro-4-(4-(trifluoromethoxy)phenoxy )phenyl)-3-(2-hydroxypropyl)-7-methyl-3,7-dihydro-1H-purine-2,6-dione | C22H18F 4N4O5 | 494.12 | 495.1 |
| **190** | 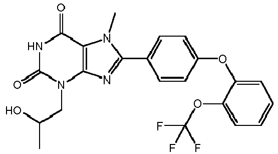 | 3-(2-hydroxypropyl)-7-methyl-8-(4-(2-(trifluoromethoxy)phenoxy )phenyl)-3,7-dihydro-1H-purine-2,6-dione | C22H19F 3N4O5 | 476.13 | 477.1 |
| **191** | 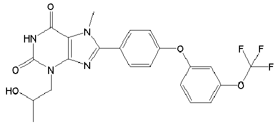 | 3-(2-hydroxypropyl)-7-methyl-8-(4-(3-(trifluoromethoxy)phenoxy )phenyl)-3,7-dihydro-1H-purine-2,6-dione | C22H19F 3N4O5 | 476.13 | 477.1 |
| **192** | 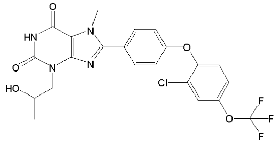 | 8-(4-(2-chloro-4-(trifluoromethoxy)phenoxy )phenyl)-3-(2-hydroxypropyl)-7-methyl-3,7-dihydro-1H-purine-2,6-dione | C22H18 ClF3N4O 5 | 510.09 | 511.1 |
| **193** | 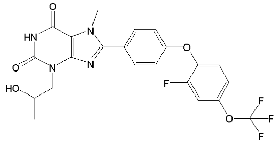 | 8-(4-(2-fluoro-4-(trifluoromethoxy)phenoxy )phenyl)-3-(2-hydroxypropyl)-7-methyl-3,7-dihydro-1H-purine-2,6-dione | C22H18F 4N4O5 | 494.12 | 495.1 |
| **194** | 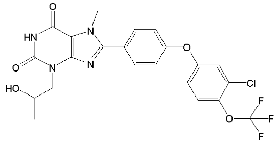 | 8-(4-(3-chloro-4-(trifluoromethoxy)phenoxy )phenyl)-3-(2-hydroxypropyl)-7-methyl-3,7-dihydro-1H-purine-2,6-dione | C22H18 ClF3N4O 5 | 510.09 | 511.1 |
| **195** | 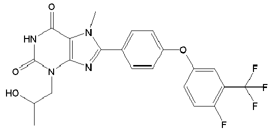 | 8-(4-(4-fluoro-3-(trifluoromethyl)phenoxy)p henyl)-3-(2-hydroxypropyl)-7-methyl-3,7-dihydro-1H-purine-2,6-dione | C22H18F 4N4O4 | 478.13 | 479.2 |
| **196** | 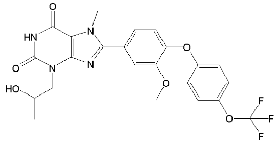 | 3-(2-hydroxypropyl)-8-(3-methoxy-4-(4-(trifluoromethoxy)phenoxy )phenyl)-7-methyl-3,7-dihydro-1H-purine-2,6-dione | C23H21F 3N4O6 | 506.14 | 507.1 |
| **197** | 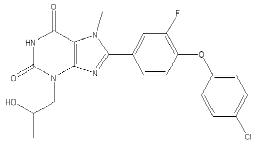 | 8-(4-(4-chlorophenoxy)-3-fluorophenyl)-3-(2-hydroxypropyl)-7-methyl-3,7-dihydro-1H-purine-2,6-dione | C21H18 ClFN4O4 | 444.10 | 445.1 |
| **200** | 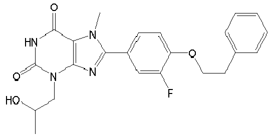 | 8-(3-fluoro-4-phenethoxyphenyl)-3-(2-hydroxypropyl)-7-methyl-3,7-dihydro-1H-purine-2,6-dione | C23H23F N4O4 | 438.17 | 439.2 |
| **201** | 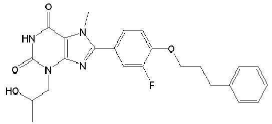 | 8-(3-fluoro-4-(3-phenylpropoxy)phenyl)-3-(2-hydroxypropyl)-7-methyl-3,7-dihydro-1H-purine-2,6-dione | C24H25F N4O4 | 452.19 | 453,3 |

### Biological Examples

### Example B-1: Immunostimulation assays with CD4+ and CD8+ T-cells

Selection of potent compounds based on structure activity relation (SAR) using human, anti-CD3/28 stimulated T-cells. Enhanced activation of stimulated T-cells is measured by CD69 expression by flowcytometry.

### 1. Materials and Equipment

### 1.1 Test Compounds

### 1.2 Materials for Bioassay

| Product | Distributor | Cat.# |
|---|---|---|
| LymphoPrep | StemCell | 07851 |
| CD4 Microbeads | Miltenyi | 130-045-101 |
| CD8 Microbeads | Miltenyi | 130-045-201 |
| Dynabeads human T-activator CD3/28 | Gibco ^{™} | 11131D |

### 2. Procedure / Description

### 2.1 Collection of PBMCs from buffy Coat

White blood cell enriched buffy coats were ordered from the Austrian red cross, diluted with PBS to a total volume of 480 ml, 30 ml transferred to 50ml falcons and 11 ml lymphoprep added. Cell suspensions were centrifuged for 20 min at 2200 rpm and the PBMC containing layer transferred, three times washed with PBS and counted.

### 2.2 Isolation of CD4+ and CD8+ T-Cells

10^7 PBMCs were magnetically labeled with CD4 and CD8 microbeads according to manufacturer's instructions and separated using autoMACS device. For dose response experiments with purified CD4+CD8+ T-cells 80000 cells were seeded per 96-well flat bottom cell culture dish.

### 2.3 Serial dilution of compounds of the invention

10 mM compounds of the invention stocks were further diluted using DMSO and equal amounts transferred to T-cell containing 96-wells. Final concentrations tested were: 30 µM, 10 µM, 3 µM, 1 µM, 0.3 µM, 0.1 µM, 0.03 µM, 0.01 µM, 0.003 µM and 0.001 µM.

### 2.4 Stimulation setup for EC₅₀ testing

For the stimulation assays 80.000 purified CD4+ and CD8+ T-cells were seeded per 96-well and incubated with 1 µg/ml anti-CD3, 1 µg/ml anti-CD28 and compounds of the invention ranging from 0.001 µM to 30 µM. DMSO only wells served as control to determine minimal T-cell activation upon stimulation and CD3/28 coupled Dynabeads were used to determine the maximal activation of T-cells. Cells were cultured for 16 hours in a humidified incubator at 37 °C and 5 % CO₂.

### 2.5 Determination of EC₅₀ values for compounds of the invention by surface FACS

T-cells were stained for surface antigens (anti-CD4 and anti-CD8) and cell activation markers (anti-CD25 and anti-CD69). Additionally, a fixable viability dye was used to stain viable cells. Staining was performed for 15 minutes and cells analyzed using a Fortessa flow cytometer and FlowJo Software. EC₅₀s were calculated using GraphPad Prism^{®} and a variable slope model (agonist vs. response - variable slope).
The assay results are summarized in Table B-#.

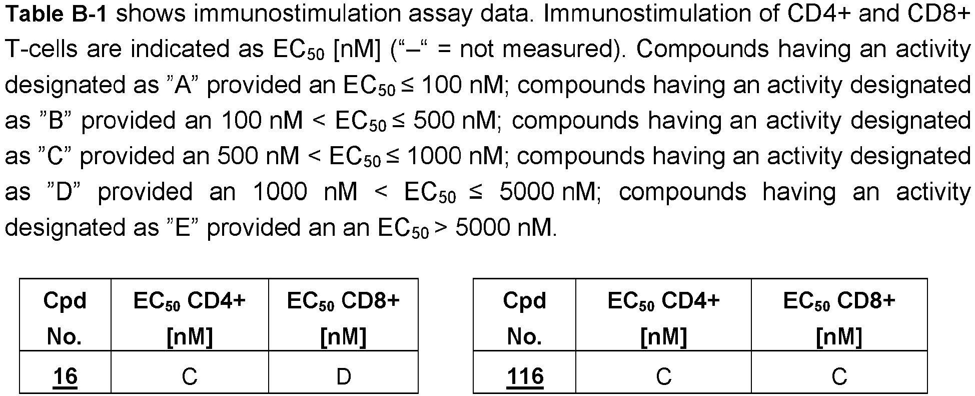

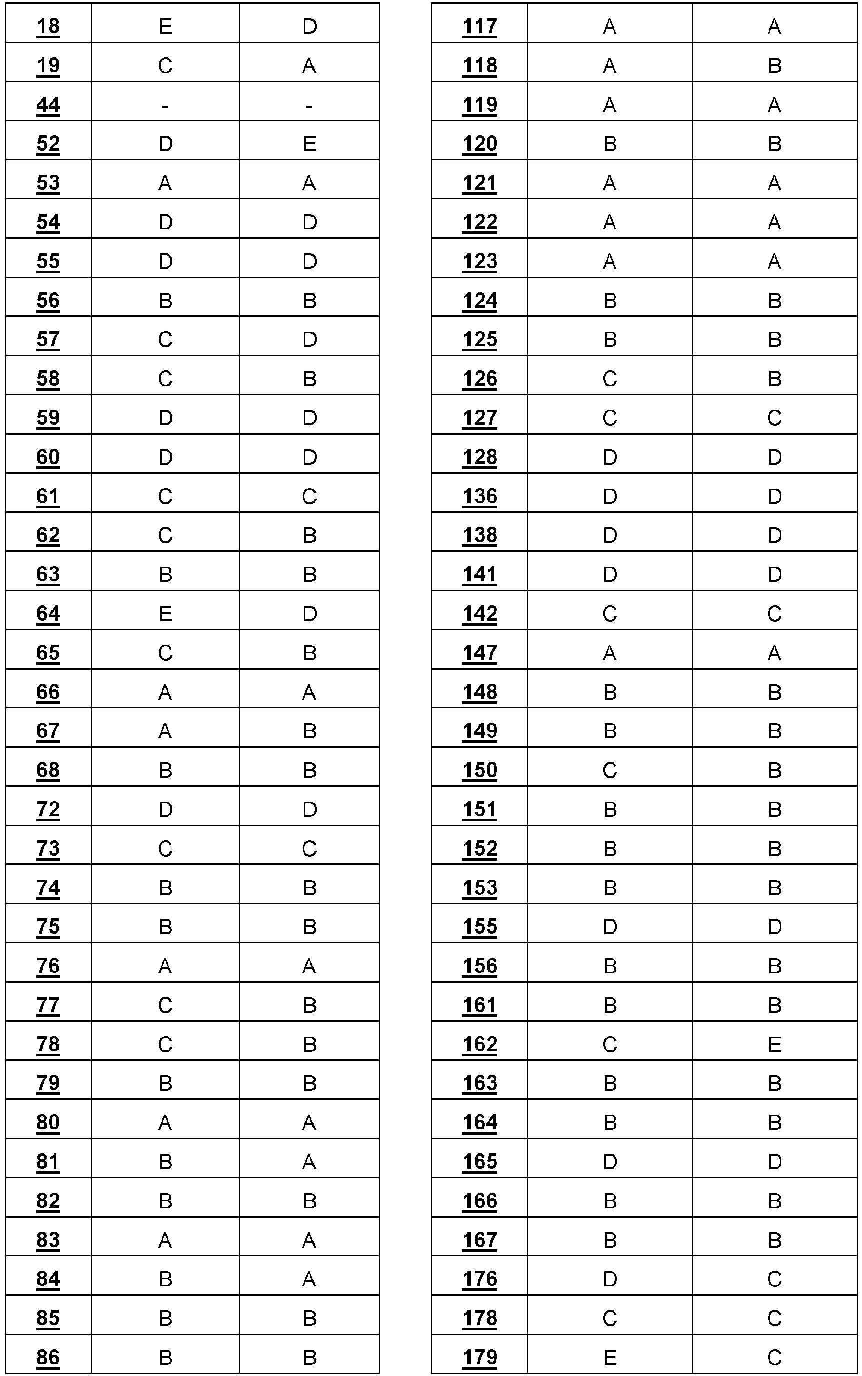

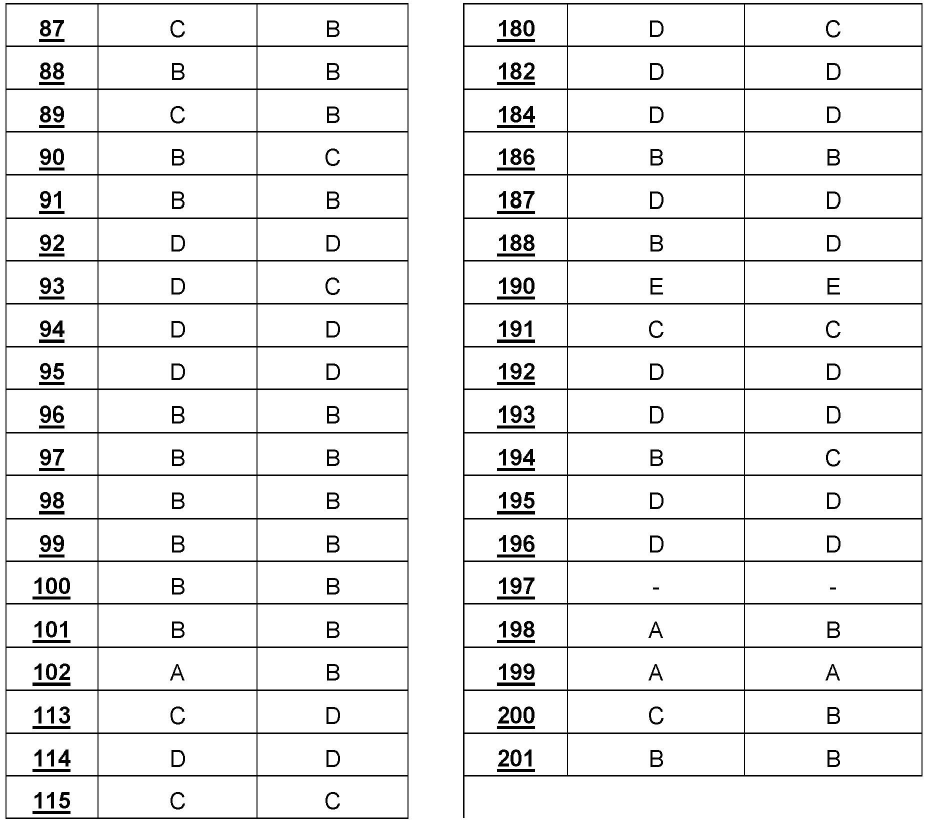

### Example B-2: Efficacy on B16-SIY melanoma

### 1 Materials and Equipment

### 1.1 Materials for Bioassay

| Product | Distributor | Cat.# |
|---|---|---|
| DMEM, low glucose, pyruvate, HEPES | Gibco^{™} | 12320032 |
| FBS heat inactivated | Gibco^{™} | 10500064 |
| Penicillin-Streptomycin (10.000 U/ml) | Gibco^{™} | 15140122 |
| L-Glutamin (200 mM) | Gibco^{™} | 25030024 |
| Trypsin-EDTA | Gibco^{™} | 25300054 |
| PBS | Gibco^{™} | 14190-144 |
| DMSO | Sigma/Merck | D2650 |
| PEG400 | Sigma/Merck | 8.07485 |
| HPbCD (2-Hydroxypropyl-β-cyclodextrin) | Sigma/Merck | 332607 |
| HPMC (Hydroxypropyl-methylcellulose) | Sigma/Merck | 09963 |
| ACK Lysis Buffer | Gibco^{™} | A1049201 |
| Antibodies for Flow Cytometry | Biolegend | |
| Viability dye | Invitrogen^{™} | 65-0865-14 |
| LEGENDplex^{™} Mouse Proinflammatory Chemokine Panel (13-plex) | Biolegend | 740451 |
| LEGENDplex^{™} Mouse Th17 Panel (7-Plex) with Filter Plate V03 | Biolegend | 741047 |

### 2 Procedure / Description

### 2.1 Animals and Ethics

Eight-weeks-old C57BL/6 mice were purchased from Charles River. All animal experiments were in accordance with institutional guidelines of the Research Institute of Molecular Pathology (Austria) and approved according to the European Community rules of animal care with the permission of the Austrian Ministry of Science.

### 2.2 B16-SIY cell culture

B16-SIY melanoma cells were cultured in DMEM containing 10 % FCS, 1 % PenStrep and 1 % glutamine and grown in a humidified incubator at 37 °C and 5 % CO₂. For tumor inoculations sub-confluent B16-SIYcells were trypsinized, washed, counted and resuspended in PBS to a final concentration of 1×10⁷ B16-SIY cells/ml.

### 2.3 Tumor inoculation and compound treatment

### Efficacy Experiment:

30 C57BL/6 mice were inoculated intradermally with 1×10⁶ B16-SIY cells and tumor bearing mice randomly assigned to 3 different groups 4 days post transplantation: Compound **142** 30 mg/kg 3 days post B16-SIY inoculation and subsequent daily administration of 10 mg/kg (10 mice), Compound **142** 9 mg/kg 3 days post B16-SIY inoculation and subsequent daily treatment of 3 mg/kg (10 mice), or vehicle control (10 mice). Compound **142** was diluted in 10 % DMSO, 20 % PEG400, 21 % HPbCD, 0.35 % HPMC and 48.65 % H₂O and mice treated p.o. with 2 different treatment schedules: d3: 30 mg/kg + QD: 10 mg/kg or d3: 9 mg/kg + QD: 3 mg/kg (**Figure 1**). 10 % DMSO, 20 % PEG400 and 21 % HPbCD, 0,35 % HPMC and 48,65 % H2O served as vehicle control. Tumors were measured daily and mice sacrificed 21 days post B16-SIY inoculation (**Figure 1**).

### 3 Results

### 3.1 Efficacy Experiment 3: Compound 142

C57BL/6 mice were inoculated intradermally with B16-SIY cells and treated p.o. with 30 mg/kg compound **142** 3 days post B16-SIY injection followed by daily administration of 10 mg/kg (d3: 30 mg/kg + QD: 10 mg/kg) or received 9 mg/kg compound **142** 3 days post B16-SIY inoculation followed by 3 mg/kg daily (d3: 9 mg/kg + QD: 3 mg/kg). Vehicle treated mice were used as controls. Tumor volumes in mice upon treatment with both compound **142** regimes were significantly reduced compared to vehicle control over the course of 21 days (**Figure 2**).

## Claims

1. A compound of the formula (**I**) 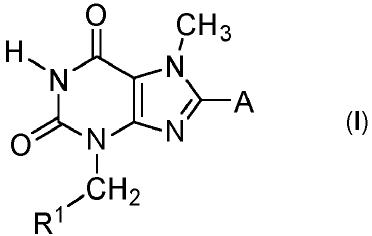 wherein
A represents 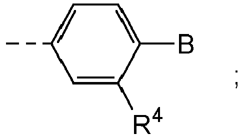
**B** is -O-**R³**, -O-CH₂-**R³**, -O-CH₂-CH₂-**R³**, -O-CH₂-CH₂-CH₂-**R³**;
**R¹** represents 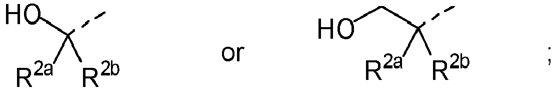
**R^{2a}** and **R^{2b}** represents independently of each other -H, or C₁-₃ alkyl, and the C₁-₃ alkyl is optionally substituted with 1 to 6 fluoro atoms or -OH;
**R³** represents 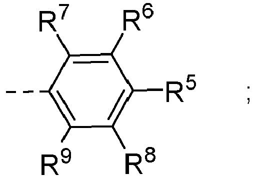
**R⁴** represents -H, halogen, C₁-₄ alkyl, -O-C₁-₄ alkyl, C₃-₄ cycloalkyl, -O-C₃-₄ cycloalkyl, and the C₁-₄ alkyl, -O-C₁-₄ alkyl, -O-C₃-₄ cycloalkyl, and C₃-₄ cycloalkyl may optionally be substituted with 1 to 6 fluoro atoms;
**R⁵**, **R⁶**, **R⁷**, **R⁸**, **R⁹**, **R¹⁰**, **R¹¹**, **R¹²** and **R¹³** represent independently of each other -H, -cyclo-C₃H₅, -cyclo-C₄H₇, -cyclo-C₅H₉, -cyclo-C₆H₁₁, -cyclo-C₇H₁₃, -cycloC₃H₅O, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -CI, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂, -OCF₃, -CH₂-OCF₃, -C₂H₄-OCF₃, -C₃H₆-OCF₃, -CH₂-OCHF₂, -C₂H₄-OCHF₂, -C₃H₆-OCHF₂, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CON(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, -C≡C-C≡C-C₂H₅, 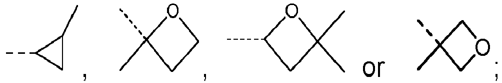 or
**R⁵** and **R⁶** or **R⁶** and **R⁷** may form together with the two carbon atoms of the phenyl ring to which they are attached a 4 to 8-membered ring system, which is optionally substituted with one or more substituents selected from **R¹⁰**, **R¹¹**, **R¹²,** and **R¹³**;
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

2. The compound according to claim 1, wherein
**R³** represents

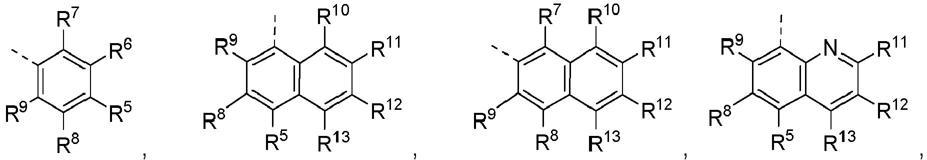

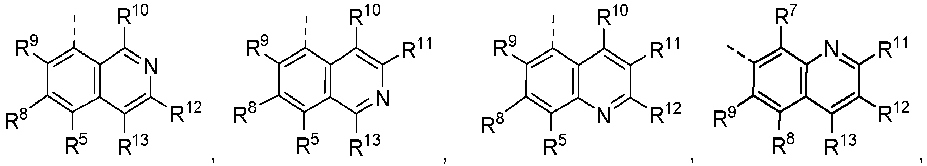

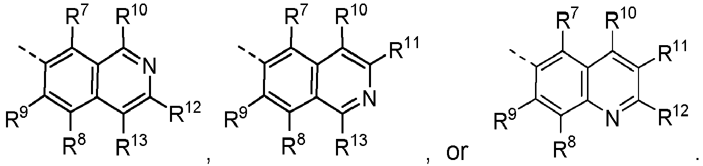

3. The compound according to claim 1 or 2, wherein
**R^{2a}** represents -H or -CH₃; and
**R^{2b}** represents -H, -CH₃, -CH₂F, -CHF₂, -CF₃, -CH₂CH₃, -CH₂OH, or -CH₂OCH₃;
**B** represents -O-**R³** or -O-CH₂-**R³**;
**R³** represents 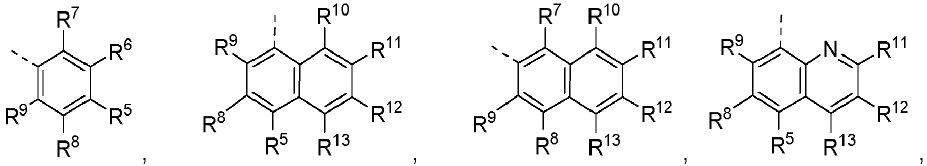 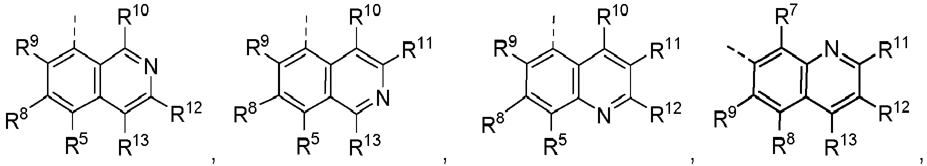 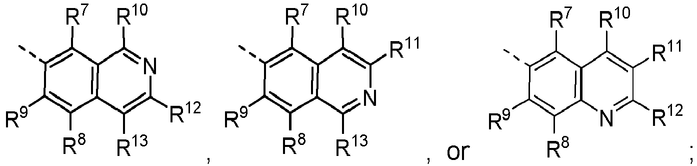
**R⁴** represents -H, -F, -Br, -Cl, -I, -CH₃, -CHF₂, -CF₃, -OCH₃, -OCHF₂, or -OCF₃;
and **R⁵**, **R⁶**, **R⁷**, **R⁸**, **R⁹**, **R¹⁰**, **R¹¹**, **R¹²** and **R¹³** have the same meanings as defined in claim 1.

4. The compound according to any one of the claims 1 - 3, wherein **R¹** represents

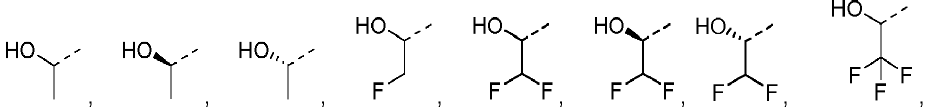

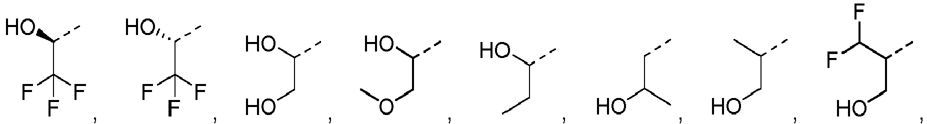

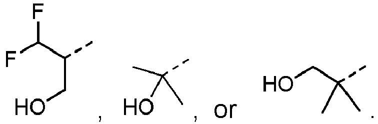

5. The compound according to any one of the claims 1 - 4, wherein the compound has any one of the following formulae (**II-1**) to (**II-5**), (**III-1**) to (**III-6**), (**IV**-1) to (**IV**-6), (**V**-1) to (**V**-3): 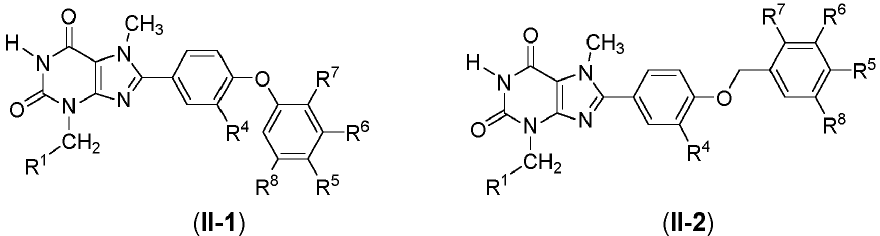 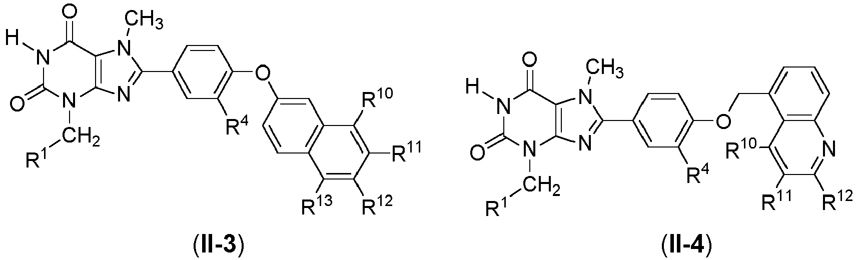 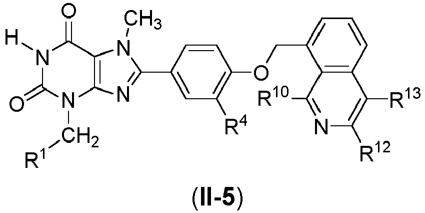 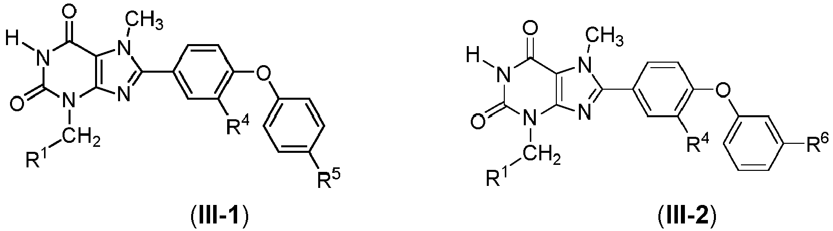 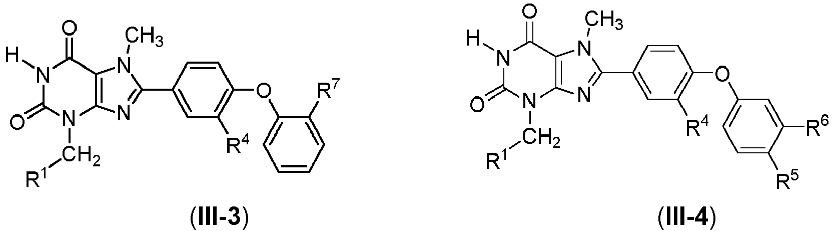 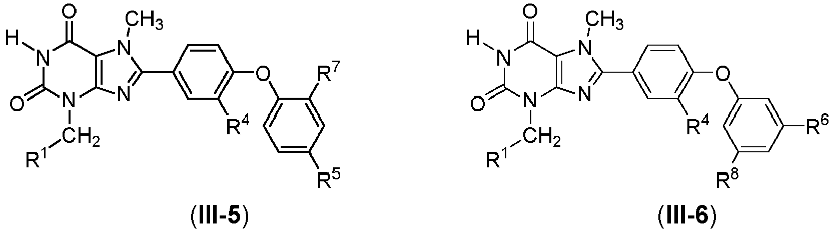 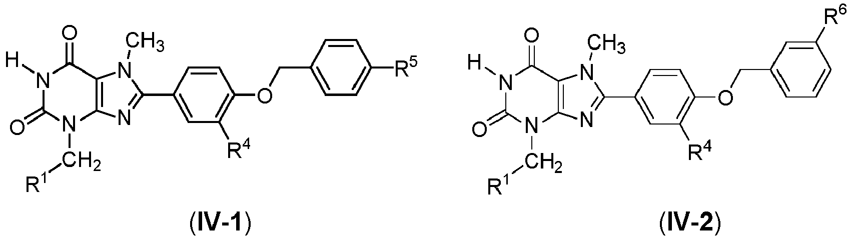 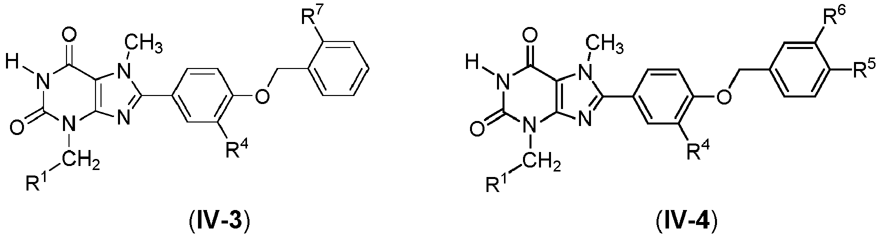 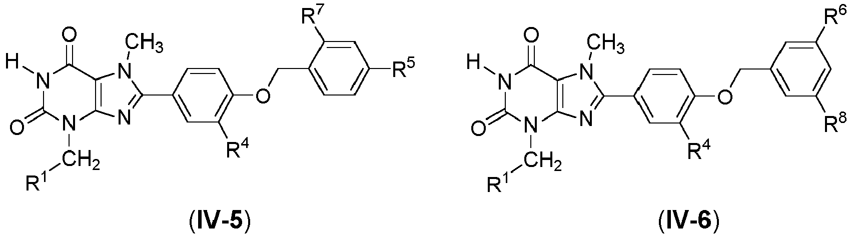 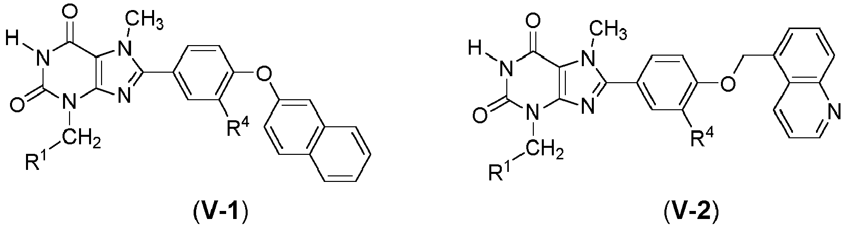 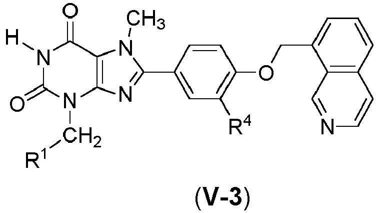 wherein **R¹**, **R⁴**, **R⁵**, **R⁶**, **R⁷**, **R⁸**, **R¹⁰**, **R¹¹**, **R¹²**, and **R¹³** have the same meanings as defined in any one of the claims 1 - 5.

6. The compound according to any one of the claims 1 - 5, wherein 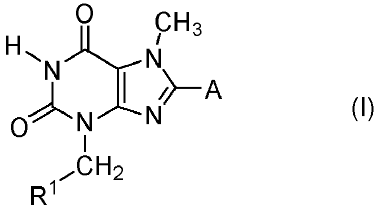 wherein
**A** represents 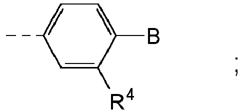
**B** represents -O-**R³** or -O-CH₂-**R³**;
**R¹** represents 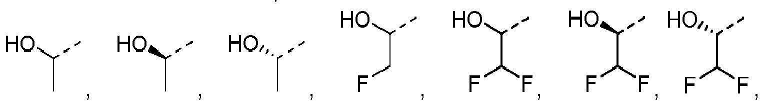 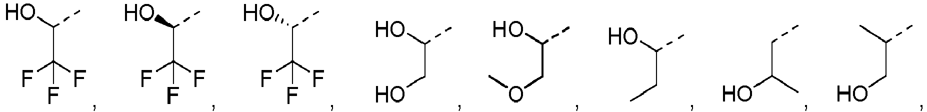 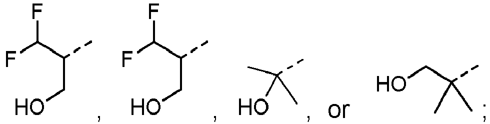
**R³** represents 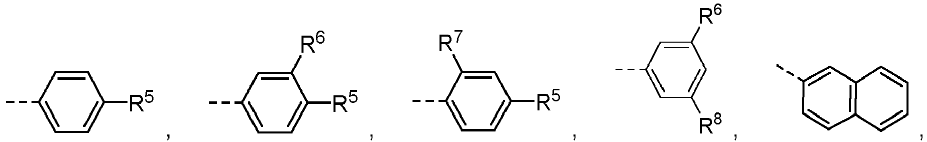 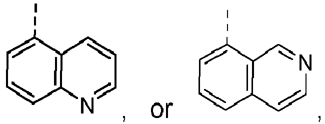
**R⁴** represents -H, -F, -Cl, -CH₃, -CHF₂, -CF₃, -OCH₃, -OCHF₂, or -OCF₃;
and
**R⁵**, **R⁶**, **R⁷**, and **R⁸** represent independently of each other -H, -F, -Cl, -CN, -CH₃, -CHF₂, -CF₃, -OCHF₂, -OCF₃, or -SO₂CH₃.

7. The compound according to any one of claims 1 - 6, wherein **R³** represents

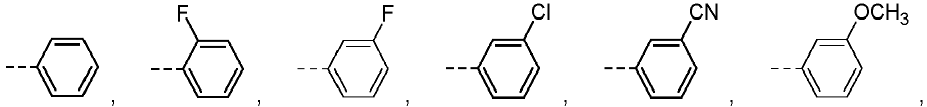

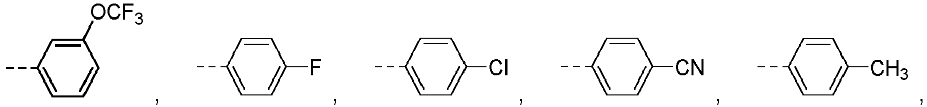

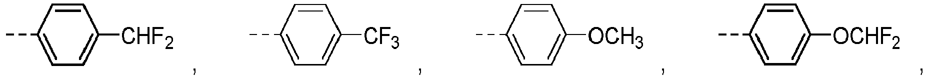

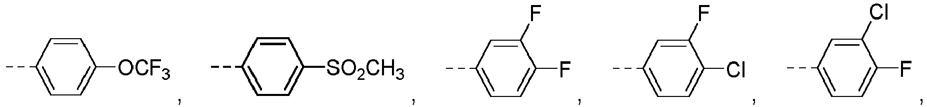

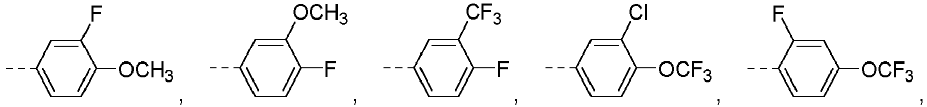

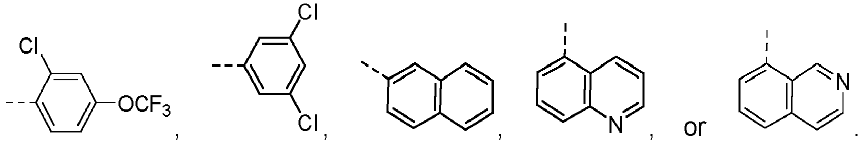

8. A compound selected from the group consisting of:
| **Cpd No.** | **IUPAC-Name** |
|---|---|
| **16** | 8-(4-(benzyloxy)-3-methoxyphenyl)-3-(2-hydroxypropyl)-7-methyl-3,7-dihydro-1H-purine-2,6-dione |
| **18** | 8-(4-(benzyloxy)-3-(trifluoromethoxy)phenyl)-3-(3,3-difluoro-2-(hydroxymethyl)propyl)-7-methyl-3,7-dihydro-1H-purine-2,6-dione |
| **19** | 3-(3,3-difluoro-2-hydroxypropyl)-8-(3-methoxy-4-((4-methylbenzyl)oxy)phenyl)-7-methyl-3,7-dihydro-1H-purine-2,6-dione |
| **44** | 8-(4-(benzyloxy)-3-fluorophenyl)-3-(2-hydroxypropyl)-7-methyl-3,7-dihydro-1H-purine-2,6-dione |
| **52** | 8-(4-(benzyloxy)-3-methoxyphenyl)-3-(2-hydroxy-2-methylpropyl)-7-methyl-3,7-dihydro-1H-purine-2,6-dione |
| **53** | (S)-8-(4-(benzyloxy)-3-methoxyphenyl)-3-(3,3-difluoro-2-hydroxypropyl)-7-methyl-3,7-dihydro-1H-purine-2,6-dione |
| **54** | 8-(4-(benzyloxy)-3-methoxyphenyl)-3-(2-hydroxy-3-methoxypropyl)-7-methyl-3,7-dihydro-1H-purine-2,6-dione |
| **55** | 8-(4-(benzyloxy)-3-methoxyphenyl)-3-(2,3-dihydroxypropyl)-7-methyl-3,7-dihydro-1H-purine-2,6-dione |
| **56** | 8-(4-(benzyloxy)-3-methoxyphenyl)-3-(3-hydroxy-2-methylpropyl)-7-methyl-3,7-dihydro-1H-purine-2,6-dione |
| **57** | 8-(4-(benzyloxy)-3-methoxyphenyl)-7-methyl-3-(3,3,3-trifluoro-2-hydroxypropyl)-3,7-dihydro-1H-purine-2,6-dione |
| **58** | 8-(4-(benzyloxy)-3-methoxyphenyl)-3-(3-fluoro-2-hydroxypropyl)-7-methyl-3,7-dihydro-1H-purine-2,6-dione |
| **59** | 8-(4-(benzyloxy)-3-methoxyphenyl)-3-(2-hydroxybutyl)-7-methyl-3,7-dihydro-1H-purine-2,6-dione |
| **60** | (R)-8-(4-(benzyloxy)-3-methoxyphenyl)-3-(2-hydroxypropyl)-7-methyl-3,7-dihydro-1H-purine-2,6-dione |
| **61** | (S)-8-(4-(benzyloxy)-3-methoxyphenyl)-3-(2-hydroxypropyl)-7-methyl-3,7-dihydro-1H-purine-2,6-dione |
| **62** | 3-(3,3-difluoro-2-hydroxypropyl)-8-(4-((4-(difluoromethyl)benzyl)oxy)-3-fluorophenyl)-7-methyl-3,7-dihydro-1H-purine-2,6-dione |
| **63** | 8-(4-((4-(difluoromethyl)benzyl)oxy)-3-fluorophenyl)-3-(3-fluoro-2-hydroxypropyl)-7-methyl-3,7-dihydro-1H-purine-2,6-dione |
| **64** | 8-(4-((4-(difluoromethyl)benzyl)oxy)-3-fluorophenyl)-7-methyl-3-(3,3,3-trifluoro-2-hydroxypropyl)-3,7-dihydro-1H-purine-2,6-dione |
| **65** | 8-(4-((4-(difluoromethyl)benzyl)oxy)-3-fluorophenyl)-3-(2-hydroxypropyl)-7-methyl-3,7-dihydro-1H-purine-2,6-dione |
| **66** | 3-(3,3-difluoro-2-(hydroxymethyl)propyl)-8-(4-((4-(difluoromethyl)benzyl)oxy)-3-methoxyphenyl)-7-methyl-3,7-dihydro-1H-purine-2,6-dione |
| **67** | 3-(3,3-difluoro-2-(hydroxymethyl)propyl)-8-(3-fluoro-4-((4-methylbenzyl)oxy)phenyl)-7-methyl-3,7-dihydro-1H-purine-2,6-dione |
| **68** | 8-(4-(benzyloxy)-3-methoxyphenyl)-3-(3,3-difluoro-2-hydroxypropyl)-7-methyl-3,7-dihydro-1H-purine-2,6-dione |
| **72** | 8-(4-((4-(difluoromethyl)benzyl)oxy)-3-methoxyphenyl)-3-(2,3-dihydroxypropyl)-7-methyl-3,7-dihydro-1H-purine-2,6-dione |
| **73** | 8-(4-((4-(difluoromethyl)benzyl)oxy)-3-methoxyphenyl)-3-(3-fluoro-2-hydroxypropyl)-7-methyl-3,7-dihydro-1H-purine-2,6-dione |
| **74** | 8-(4-((4-(difluoromethyl)benzyl)oxy)-3-methoxyphenyl)-3-(3-hydroxy-2-methylpropyl)-7-methyl-3,7-dihydro-1H-purine-2,6-dione |
| **75** | 8-(4-((4-(difluoromethyl)benzyl)oxy)-3-methoxyphenyl)-7-methyl-3-(3,3,3-trifluoro-2-hydroxypropyl)-3,7-dihydro-1H-purine-2,6-dione |
| **76** | 8-(3-(difluoromethoxy)-4-((4-methylbenzyl)oxy)phenyl)-3-(2-hydroxypropyl)-7-methyl-3,7-dihydro-1H-purine-2,6-dione |
| **77** | 3-(2-hydroxypropyl)-7-methyl-8-(4-((4-methylbenzyl)oxy)phenyl)-3,7-dihydro-1H-purine-2,6-dione |
| **78** | 8-(4-(benzyloxy)phenyl)-3-(2-hydroxypropyl)-7-methyl-3,7-dihydro-1H-purine-2,6-dione |
| **79** | 3-(3,3-difluoro-2-hydroxypropyl)-8-(4-((4-(difluoromethyl)benzyl)oxy)-3-methoxyphenyl)-7-methyl-3,7-dihydro-1H-purine-2,6-dione |
| **80** | 3-(3,3-difluoro-2-hydroxypropyl)-8-(3-fluoro-4-((4-methylbenzyl)oxy)phenyl)-7-methyl-3,7-dihydro-1H-purine-2,6-dione |
| **81** | 8-(4-(4-chlorophenoxy)-3-(difluoromethoxy)phenyl)-3-(2-hydroxypropyl)-7-methyl-3,7-dihydro-1H-purine-2,6-dione |
| **82** | 3-(2-hydroxypropyl)-8-(3-methoxy-4-((4-(trifluoromethoxy)benzyl)oxy)phenyl)-7-methyl-3,7-dihydro-1H-purine-2,6-dione |
| **83** | 3-(3,3-difluoro-2-hydroxypropyl)-8-(4-((4-(difluoromethoxy)benzyl)oxy)-3-methoxyphenyl)-7-methyl-3,7-dihydro-1H-purine-2,6-dione |
| **84** | 3-(3,3-difluoro-2-hydroxypropyl)-8-(4-((4-fluorobenzyl)oxy)-3-methoxyphenyl)-7-methyl-3,7-dihydro-1H-purine-2,6-dione |
| **85** | 8-(4-((4-(difluoromethoxy)benzyl)oxy)-3-methoxyphenyl)-3-(2-hydroxypropyl)-7-methyl-3,7-dihydro-1H-purine-2,6-dione |
| **86** | 8-(4-((4-(difluoromethyl)benzyl)oxy)-3-methoxyphenyl)-3-(2-hydroxypropyl)-7-methyl-3,7-dihydro-1H-purine-2,6-dione |
| **87** | 8-(4-((4-fluorobenzyl)oxy)-3-methoxyphenyl)-3-(2-hydroxypropyl)-7-methyl-3,7-dihydro-1H-purine-2,6-dione |
| **88** | 8-(4-((4-chlorobenzyl)oxy)-3-methoxyphenyl)-3-(2-hydroxypropyl)-7-methyl-3,7-dihydro-1H-purine-2,6-dione |
| **89** | 3-(2-hydroxypropyl)-8-(3-methoxy-4-((4-(trifluoromethyl)benzyl)oxy)phenyl)-7-methyl-3,7-dihydro-1H-purine-2,6-dione |
| **90** | 4-((4-(3-(2-hydroxypropyl)-7-methyl-2,6-dioxo-2,3,6,7-tetrahydro-1H-purin-8-yl)-2-methoxyphenoxy)methyl)benzonitrile |
| **91** | 8-(3-chloro-4-((4-fluorobenzyl)oxy)phenyl)-3-(2-hydroxypropyl)-7-methyl-3,7-dihydro-1H-purine-2,6-dione |
| **92** | 8-(4-((3,4-difluorobenzyl)oxy)-3-fluorophenyl)-3-(2-hydroxypropyl)-7-methyl-3,7-dihydro-1H-purine-2,6-dione |
| **93** | 8-(4-((4-chloro-3-fluorobenzyl)oxy)-3-fluorophenyl)-3-(2-hydroxypropyl)-7-methyl-3,7-dihydro-1H-purine-2,6-dione |
| **94** | 3-(2,3-dihydroxypropyl)-8-(3-methoxy-4-((4-methylbenzyl)oxy)phenyl)-7-methyl-3,7-dihydro-1H-purine-2,6-dione |
| **95** | 3-(2,3-dihydroxypropyl)-8-(3-fluoro-4-((4-methylbenzyl)oxy)phenyl)-7-methyl-3,7-dihydro-1H-purine-2,6-dione |
| **96** | 3-(3-fluoro-2-hydroxypropyl)-8-(3-fluoro-4-((4-methylbenzyl)oxy)phenyl)-7-methyl-3,7-dihydro-1H-purine-2,6-dione |
| **97** | 8-(3-fluoro-4-((4-methylbenzyl)oxy)phenyl)-3-(3-hydroxy-2-methylpropyl)-7-methyl-3,7-dihydro-1H-purine-2,6-dione |
| **98** | 8-(4-(benzyloxy)-3-(trifluoromethyl)phenyl)-3-(2-hydroxypropyl)-7-methyl-3,7-dihydro-1H-purine-2,6-dione |
| **99** | 8-(4-((3-chloro-4-fluorobenzyl)oxy)-3-methoxyphenyl)-3-(2-hydroxypropyl)-7-methyl-3,7-dihydro-1H-purine-2,6-dione |
| **100** | 3-(3-hydroxy-2-methylpropyl)-7-methyl-8-(4-(4-(trifluoromethoxy)phenoxy)phenyl)-3,7-dihydro-1H-purine-2,6-dione |
| **101** | 3-(2-hydroxypropyl)-8-(4-(isoquinolin-8-ylmethoxy)-3-methoxyphenyl)-7-methyl-3,7-dihydro-1H-purine-2,6-dione |
| **102** | 8-(3-fluoro-4-((4-methylbenzyl)oxy)phenyl)-7-methyl-3-(3,3,3-trifluoro-2-hydroxypropyl)-3,7-dihydro-1H-purine-2,6-dione |
| **113** | 3-(2-hydroxypropyl)-8-(3-methoxy-4-(quinolin-5-ylmethoxy)phenyl)-7-methyl-3,7-dihydro-1H-purine-2,6-dione |
| **114** | 8-(3-fluoro-4-((4-methoxybenzyl)oxy)phenyl)-3-(2-hydroxypropyl)-7-methyl-3,7-dihydro-1H-purine-2,6-dione |
| **115** | 8-(3-fluoro-4-((4-methylbenzyl)oxy)phenyl)-3-(2-hydroxypropyl)-7-methyl-3,7-dihydro-1H-purine-2,6-dione |
| **116** | 8-(4-(benzyloxy)-3-(trifluoromethoxy)phenyl)-3-(2,3-dihydroxypropyl)-7-methyl-3,7-dihydro-1H-purine-2,6-dione |
| **117** | 8-(4-(benzyloxy)-3-(trifluoromethoxy)phenyl)-3-(3-fluoro-2-hydroxypropyl)-7-methyl-3,7-dihydro-1H-purine-2,6-dione |
| **118** | 8-(4-(benzyloxy)-3-(trifluoromethoxy)phenyl)-3-(3-hydroxy-2-methylpropyl)-7-methyl-3,7-dihydro-1H-purine-2,6-dione |
| **119** | 8-(4-(benzyloxy)-3-(trifluoromethoxy)phenyl)-7-methyl-3-(3,3,3-trifluoro-2-hydroxypropyl)-3,7-dihydro-1H-purine-2,6-dione |
| **120** | 8-(4-(benzyloxy)-3-(trifluoromethoxy)phenyl)-3-(2-hydroxypropyl)-7-methyl-3,7-dihydro-1H-purine-2,6-dione |
| **121** | 8-(3-methoxy-4-((4-methylbenzyl)oxy)phenyl)-7-methyl-3-(3,3,3-trifluoro-2-hydroxypropyl)-3,7-dihydro-1H-purine-2,6-dione |
| **122** | 3-(2-hydroxypropyl)-7-methyl-8-(4-((4-methylbenzyl)oxy)-3-(trifluoromethoxy)phenyl)-3,7-dihydro-1H-purine-2,6-dione |
| **123** | 3-(2-hydroxypropyl)-7-methyl-8-(4-((4-methylbenzyl)oxy)-3-(trifluoromethyl)phenyl)-3,7-dihydro-1H-purine-2,6-dione |
| **124** | 8-(4-(benzyloxy)-3-methylphenyl)-3-(2-hydroxypropyl)-7-methyl-3,7-dihydro-1H-purine-2,6-dione |
| **125** | 3-(2-hydroxypropyl)-8-(3-methoxy-4-((4-methylbenzyl)oxy)phenyl)-7-methyl-3,7-dihydro-1H-purine-2,6-dione |
| **126** | 8-(4-((3-fluoro-4-methoxybenzyl)oxy)-3-methoxyphenyl)-3-(2-hydroxypropyl)-7-methyl-3,7-dihydro-1H-purine-2,6-dione |
| **127** | 8-(4-((4-fluoro-3-methoxybenzyl)oxy)-3-methoxyphenyl)-3-(2-hydroxypropyl)-7-methyl-3,7-dihydro-1H-purine-2,6-dione |
| **128** | 3-(2-hydroxypropyl)-8-(3-methoxy-4-((4-methoxybenzyl)oxy)phenyl)-7-methyl-3,7-dihydro-1H-purine-2,6-dione |
| **136** | 3-(2-hydroxypropyl)-7-methyl-8-(4-(4-(trifluoromethyl)phenoxy)phenyl)-3,7-dihydro-1H-purine-2,6-dione |
| **138** | 8-(4-(4-chlorophenoxy)phenyl)-3-(2-hydroxypropyl)-7-methyl-3,7-dihydro-1H-purine-2,6-dione |
| **141** | 8-(3-chloro-4-phenoxyphenyl)-3-(2-hydroxypropyl)-7-methyl-3,7-dihydro-1H-purine-2,6-dione |
| **142** | 3-(2-hydroxypropyl)-7-methyl-8-(4-(4-(trifluoromethoxy)phenoxy)phenyl)-3,7-dihydro-1H-purine-2,6-dione |
| **147** | 8-(3-chloro-4-(4-chlorophenoxy)phenyl)-3-(3-fluoro-2-hydroxypropyl)-7-methyl-3,7-dihydro-1H-purine-2,6-dione |
| **148** | 8-(3-chloro-4-(4-(difluoromethoxy)phenoxy)phenyl)-3-(3-fluoro-2-hydroxypropyl)-7-methyl-3,7-dihydro-1H-purine-2,6-dione |
| **149** | 3-(3-fluoro-2-hydroxypropyl)-8-(3-fluoro-4-(4-(trifluoromethoxy)phenoxy)phenyl)-7-methyl-3,7-dihydro-1H-purine-2,6-dione |
| **150** | 8-(3-chloro-4-(4-chlorophenoxy)phenyl)-3-(3-hydroxy-2-methylpropyl)-7-methyl-3,7-dihydro-1H-purine-2,6-dione |
| **151** | 8-(3-chloro-4-(4-(difluoromethoxy)phenoxy)phenyl)-3-(3-hydroxy-2-methylpropyl)-7-methyl-3,7-dihydro-1H-purine-2,6-dione |
| **152** | 8-(3-fluoro-4-(4-(trifluoromethoxy)phenoxy)phenyl)-3-(3-hydroxy-2-methylpropyl)-7-methyl-3,7-dihydro-1H-purine-2,6-dione |
| **153** | 8-(3-chloro-4-(4-chlorophenoxy)phenyl)-7 -methyl-3-(3, 3, 3-trifluoro-2-hydroxypropyl)-3,7-dihydro-1H-purine-2,6-dione |
| **155** | 3-(2-hydroxypropyl)-7-methyl-8-(4-(4-(trifluoromethoxy)phenoxy)-3-(trifluoromethyl)phenyl)-3,7-dihydro-1H-purine-2,6-dione |
| **156** | 3-(2-hydroxypropyl)-7-methyl-8-(3-(trifluoromethoxy)-4-(4-(trifluoromethoxy)phenoxy)phenyl)-3,7-dihydro-1H-purine-2,6-dione |
| **161** | 8-(4-(2-fluoro-4-(trifluoromethoxy)phenoxy)phenyl)-7-methyl-3-(3,3,3-trifluoro-2-hydroxypropyl)-3,7-dihydro-1H-purine-2,6-dione |
| **162** | 8-(3-chloro-4-(4-(difluoromethoxy)phenoxy)phenyl)-7-methyl-3-(3,3,3-trifluoro-2-hydroxypropyl)-3,7-dihydro-1H-purine-2,6-dione |
| **163** | 8-(3-chloro-4-(2-fluoro-4-(trifluoromethoxy)phenoxy)phenyl)-7-methyl-3-(3,3,3-trifluoro-2-hydroxypropyl)-3,7-dihydro-1H-purine-2,6-dione |
| **164** | 8-(4-(4-chlorophenoxy)phenyl)-7-methyl-3-(3,3,3-trifluoro-2-hydroxypropyl)-3,7-dihydro-1H-purine-2,6-dione |
| **165** | 8-(4-(4-(difluoromethoxy)phenoxy)phenyl)-7-methyl-3-(3,3,3-trifluoro-2-hydroxypropyl)-3,7-dihydro-1H-purine-2,6-dione |
| **166** | 8-(4-(3-chloro-4-(trifluoromethoxy)phenoxy)-3-fluorophenyl)-7-methyl-3-(3,3,3-trifluoro-2-hydroxypropyl)-3,7-dihydro-1H-purine-2,6-dione |
| **167** | 8-(3-fluoro-4-(4-(trifluoromethoxy)phenoxy)phenyl)-7-methyl-3-(3,3,3-trifluoro-2-hydroxypropyl)-3,7-dihydro-1H-purine-2,6-dione |
| **176** | 3-(2-hydroxypropyl)-7-methyl-8-(4-(4-(methylsulfonyl)phenoxy)phenyl)-3,7-dihydro-1H-purine-2,6-dione |
| **178** | 8-(3-chloro-4-(4-(difluoromethoxy)phenoxy)phenyl)-3-(2-hydroxypropyl)-7-methyl-3,7-dihydro-1H-purine-2,6-dione |
| **179** | 8-(3-chloro-4-(4-(trifluoromethoxy)phenoxy)phenyl)-3-(2-hydroxypropyl)-7-methyl-3,7-dihydro-1H-purine-2,6-dione |
| **180** | 8-(3-chloro-4-(4-chlorophenoxy)phenyl)-3-(2-hydroxypropyl)-7-methyl-3,7-dihydro-1H-purine-2,6-dione |
| **182** | 3-(2-hydroxypropyl)-8-(4-(3-methoxyphenoxy)phenyl)-7-methyl-3,7-dihydro-1H-purine-2,6-dione |
| **184** | 8-(4-(3-chlorophenoxy)phenyl)-3-(2-hydroxypropyl)-7-methyl-3,7-dihydro-1H-purine-2,6-dione |
| **186** | 3-(2-hydroxypropyl)-7-methyl-8-(4-(naphthalen-2-yloxy)phenyl)-3,7-dihydro-1H-purine-2,6-dione |
| **187** | 3-(4-(3-(2-hydroxypropyl)-7-methyl-2,6-dioxo-2,3,6,7-tetrahydro-1H-purin-8-yl)phenoxy)benzonitrile |
| **188** | 8-(3-fluoro-4-(4-(trifluoromethoxy)phenoxy)phenyl)-3-(2-hydroxypropyl)-7-methyl-3,7-dihydro-1H-purine-2,6-dione |
| **190** | 3-(2-hydroxypropyl)-7-methyl-8-(4-(2-(trifluoromethoxy)phenoxy)phenyl)-3,7-dihydro-1H-purine-2,6-dione |
| **191** | 3-(2-hydroxypropyl)-7-methyl-8-(4-(3-(trifluoromethoxy)phenoxy)phenyl)-3,7-dihydro-1H-purine-2,6-dione |
| **192** | 8-(4-(2-chloro-4-(trifluoromethoxy)phenoxy)phenyl)-3-(2-hydroxypropyl)-7-methyl-3,7-dihydro-1H-purine-2,6-dione |
| **193** | 8-(4-(2-fluoro-4-(trifluoromethoxy)phenoxy)phenyl)-3-(2-hydroxypropyl)-7-methyl-3,7-dihydro-1H-purine-2,6-dione |
| **194** | 8-(4-(3-chloro-4-(trifluoromethoxy)phenoxy)phenyl)-3-(2-hydroxypropyl)-7-methyl-3,7-dihydro-1H-purine-2,6-dione |
| **195** | 8-(4-(4-fluoro-3-(trifluoromethyl)phenoxy)phenyl)-3-(2-hydroxypropyl)-7-methyl-3, 7 -dihydro-1H-purine-2,6-dione |
| **196** | 3-(2-hydroxypropyl)-8-(3-methoxy-4-(4-(trifluoromethoxy)phenoxy)phenyl)-7-methyl-3,7-dihydro-1H-purine-2,6-dione |
| **197** | 8-(4-(4-chlorophenoxy)-3-fluorophenyl)-3-(2-hydroxypropyl)-7-methyl-3,7-dihydro-1H-purine-2,6-dione |
| **198** | 8-(4-(benzyloxy)-3-(trifluoromethoxy)phenyl)-3-(3,3-difluoro-2-(hydroxymethyl)propyl)-7-methyl-3,7-dihydro-1H-purine-2,6-dione |
| **199** | 8-(4-(benzyloxy)-3-(trifluoromethoxy)phenyl)-3-(3,3-difluoro-2-hydroxypropyl)-7-methyl-3,7-dihydro-1H-purine-2,6-dione |
| **200** | 8-(3-fluoro-4-phenethoxyphenyl)-3-(2-hydroxypropyl)-7-methyl-3,7-dihydro-1H-purine-2,6-dione |
| **201** | 8-(3-fluoro-4-(3-phenylpropoxy)phenyl)-3-(2-hydroxypropyl)-7-methyl-3,7-dihydro-1H-purine-2,6-dione |
or an enantiomer, a diastereomer, a tautomer, a mixture of enantiomers, a mixture of diastereomers, a mixture of tautomers, a hydrate, a solvate, a pharmaceutically acceptable salt thereof.

9. A pharmaceutical composition comprising at least one compound according to any one of the claims 1 - 8 as an active ingredient, together with at least one pharmaceutically acceptable carrier, excipient and/or diluent.

10. The pharmaceutical composition according to claim 9 further comprising at least one stimulating agent for activating immune cells.

11. A compound according to any one of the claims 1 - 8, a pharmaceutically acceptable salt thereof, or a pharmaceutical composition according to claim 9 or 10 for use as a medicament.

12. A compound according to any one of the claims 1 - 8 for use, a pharmaceutically acceptable salt thereof for use, or the pharmaceutical composition according to claim 9 or 10 for use in the prophylaxis or treatment of a neoplastic and/or infectious disease.

13. The compound for use, the pharmaceutically acceptable salt thereof for use, or the pharmaceutical composition for use according to claim 12, wherein the compound, the pharmaceutically acceptable salt thereof or the pharmaceutical composition is administered in combination with one or more further stimulating agents activating immune cells.

14. The compound for use, the pharmaceutically acceptable salt thereof for use, or the pharmaceutical composition according to claim 12 or 13, wherein the neoplastic disease is a cancer.

15. An *in vitro* or ex *vivo* method for the production of activated immune cells comprising the steps of:
(i) providing immune cells;
(ii) contacting the cells of step (i) with:
(a) at least one compound or pharmaceutically acceptable salt thereof as defined in any one of the claims 1 - 9, and optionally
(b) one or more further stimulating agents activating said immune cells; and
(iii) cultivating the cells of step (ii) under conditions suitable for maintaining the viability of said cells.
